# EUROPEAN PATENT SPECIFICATION

(11) **EP 1 496 911 B1**
(45) Date of publication and mention of the grant of the patent: **19.12.2007**
(21) Application number: 03728372.8
(22) Date of filing: 10.04.2003
(51) Int. Cl.: A61K 31/52, A61P 29/00

(54) **USE OF A COMBINATION COMPRISING A2A ADENOSINE RECEPTOR AGONISTS AND ANTI-PATHOGENIC AGENTS FOR THE TREATMENT OF INFLAMMATORY DISEASES**
VERWENDUNG VON A2A ADENOSIN REZEPTOR AGONISTEN UND ANTI-PATHOGENE MITTEL ENTHALTENDEN KOMBINATIONEN ZUR BEHANDLUNG VON ENTZÜNDUNGSKRANKHEITEN
UTILISATION D'UNE COMBINAISON COMPRENANT UN AGONISTE DU RECEPTEUR D'ADENOSINE A2A ET UN AGENT ANTI-PATHOGENE DANS LE TRAITEMENT DE MALADIES INFLAMMATOIRES

(30) Priority: 10.04.2002 US 371434 P; 07.06.2002 US 387184 P
(43) Date of publication of application: 19.01.2005
(73) Proprietor: UNIVERSITY OF VIRGINIA PATENT FOUNDATION, Charlottesville, Virginia 22903 (US)
(72) Inventor: LINDEN, Joel, M., Charlottesville, VA 22901 (US); SULLIVAN, Gail, W., Charlottesville, VA 22903 (US); MACDONALD, Timothy, L., Charlottesville, VA 22903 (US); SCHELD, W. Michael, Earlysville, VA 22936 (US); OBRIG, Tom G., Charlottesville, VA 22903 (US); RIEGER, Jayson M., Charlottesville, VA 22911 (US)
(74) Representative: Lucas, Brian Ronald
(86) International application number: PCT/US2003/011146
(87) International publication number: WO 2003/086408

(56) References cited:
- WO-A-00/72799
- WO-A-01/94368
- WO-A-99/34804
- US-A- 5 877 180
- US-B1- 6 350 735
- SULLIVAN G W ET AL: "NEUTROPHIL A2A ADENOSINE RECEPTOR INHIBITS INFLAMMATION IN A RAT MODEL OF MENINGITIS: SYNERGY WITH THE TYPE IV PHOSPHODIESTERASE INHIBITOR, ROLIPRAM" JOURNAL OF INFECTIOUS DISEASES, CHICAGO, IL, US, vol. 180, no. 5, November 1999 (1999-11), pages 1550-1560, XP000978330 ISSN: 0022-1899
- BUSTER B(A) ET AL: "The effect of adenosine receptor agonists on neutrophil pleocytosis and blood-brain barrier pathophysiology in experimental bacterial meningitis" PROGRAM AND ABSTRACTS OF THE INTERSCIENCE CONFERENCE ON ANTIMICROBIAL AGENTS AND CHEMOTHERAPY, XX, XX, vol. 37, 1997, page 39 XP002104913
- SULLIVAN G W ET AL: "ROLE OF A2A ADENOSINE RECEPTORS IN INFLAMMATION" DRUG DEVELOPMENT RESEARCH, NEW YORK, NY, US, vol. 45, no. 3/4, November 1998 (1998-11), pages 103-112, XP000978332 ISSN: 0272-4391
- SULLIVAN GW ET AL: "The specific type IV phosphodiesterase inhibitor rolipram combined with adenosine reduces tumor necrosis factor-alpha-primed neutrophil oxidative activity" INTERNATIONAL JOURNAL OF IMMUNOPHARMACOLOGY, ELMSFORD,NY, US, vol. 17, no. 10, 1995, pages 793-803, XP002104914 ISSN: 0192-0561
- JEAN F. ET AL: 'alpha1-Antitrypsin Portland, a bioengineered serpin highly selective for furin: application as an antipathogenic agent' PROCEEDINGS OF THE NATIONAL ACADEMY OF SCIENCES U.S.A. vol. 95, no. 13, 23 June 1998, pages 7293 - 7298
- FEI H. ET AL: 'The inhibitory activities of recombinant eglin C mutants on kexin and furin, using site-directed mutagenesis and molecular modeling' ACTA BIOCHIMICA ET BIOPHYSICA SINICA vol. 33, no. 6, 2001, pages 591 - 599
- ANONYMOUS: 'Soybean promoter can be used to increase disease- and pest-resistance without affecting seeds (T99068)', [Online] Retrieved from the Internet: <URL:http://www.otm.uiuc.edu/techs/techdeta il.asp?id=137> [retrieved on 2006-02-21]
- ANONYMOUS: 'Jian Li (Senior Research Scientist, Facility for Anti-Infective Drug Development and Innovation (FADDI))', [Online] Retrieved from the Internet: <URL:http://www.vcp.monash.edu.au/departmen ts/pharmpract/staff/jli.html> [retrieved on 2006-02-21]
- ANONYMOUS: 'LACTO-AC Information', [Online] Retrieved from the Internet: <URL:http://www.tjpc.com/lactoac.htm> [retrieved on 2006-02-21]
- CLAYTON D.H.; VERNON J.G.: 'Common glackle anting with lime fruit and its effect on ectoparasites' THE AUK vol. 110, no. 4, October 1993, pages 951 - 952
- MERIEL JONES: 'Hot off the Press' MICROBIOLOGY TODAY vol. 29, February 2002, page 48

## Description

### Government Funding

The invention described herein was made with government support under Grant Number RO1 HL37942 awarded by the National Institute of Health. The United States Government has certain rights in the invention

### Cross-Reference to Related Applications

This application claims priority from U.S. provisional patent application Serial No. 60/371,434, filed April 10, 2002, and U.S. provisional patent application Serial No. 60/387,184, filed June 7, 2002.

### FIELD OF THE INVENTION

The present invention provides the use of an A_{2A} adenosine receptor agonist and an anti-pathogenic agent for the manufacture of a medicament for treating inflammation caused by bacterial, fungal or viral infections and the inflammation caused by the treatment of these infections, *e.g*., by the death of the bacterial or viral cells.

### BACKGROUND OF THE INVENTION

Bacterial, fungal and viral pathogens can cause infections which can lead to severe illness and even death. For example, the spore-forming Gram-positive rod *Bacillus anthracis* causes anthrax, a worldwide disease primarily affecting herbivores. Human infections occur sporadically from contact with infected animals or contaminated animal products. The disease is a constant threat in endemic regions because spores can persist for years in the soil. Recent events in the United States underscore the potential of anthrax as a bioterrorism agent.

The pathogenisis of lethal infections is complex, requiring germination of the spore inoculum, systemic invasion, multiplication, and toxin production leading to death. Often the symptoms of infections can include development of fatal inflammatory (septic) shock. Thus, adjunctive therapies to minimize the detrimental effects of inflammatory shock are under active investigation.

Inflammatory shock can be caused by the pathogens directly or by the death of the pathogens after treating the patient with a drug that kills the pathogen. Often, the sudden development of fatal inflammatory (septic) shock and the progression of the disease, despite the availability of a bacteriological or antiviral cure, account for the high mortality from these pathogens.

The inflammatory shock can be caused by the bacteria, fungal or viral pathogens directly or from the treatment thereof, i.e., the death of the pathogens due to treatment with antibacterial, antifungal or antiviral agents. Agonists of A_{2A} adenosine receptors inhibit inflammation caused by dying pathogens. Accordingly, there is a need for selective, potent, and specific A_{2A}AR agonists for use in adjunctive therapy for treating inflammatory bacterial, fungal and viral infections.

In accordance with the present invention, selective, potent, and specific A_{2A}AR agonists have utility as a potential adjunct in therapy for treatment in combination with other agents that kill bacterial, fungal and viral infections such as, for example, anthrax, tularemia, escherichia coli and plague.

There is currently a need for pharmaceutical agents that are useful to reduce an inflammatory response due to the invasion of bacteria, funguses, or viruses or to reduce the inflammatory response due to toxins released by the bacteria, funguses, or viruses while alive or after they are killed using antibacterial agents, anti fungal agents or antiviral agents.

### SUMMARY OF THE INVENTION

The present invention provides use of an A_{2A} adenosine receptor agonist in the preparation of a medicament for treating inflammation caused by pathogenic organisms in which an anti-pathogenic agent and said A_{2A} adenosine receptor agonist are administered to a patient simultaneously or sequentially.

The present invention provides use in preparation of a medicament for treating biological diseases that includes the administration of an effective amount of a suitable antibiotic agent, antifungal agent or antiviral agent in conjunction with an A_{2A} adenosine receptor agonist. If no anti-pathogenic agent is known the A_{2A} agonist can be used alone to reduce inflammation, as may occur during infection with antibiotic resistant bacteria, or certain viruses such as those that cause SARS or Ebola. Optionally, the method includes administration of a type IV PDE inhibitor. The A_{2A} adenosine receptor agonist can provide adjunctive therapy for treatment conditions such as, the inflammation, caused by sepsis, for example, *human uremic syndrome* when administered with antibiotics in the treatment of bio-terrorism weapons, such as anthrax, tularemia, Escherichia coli, plague. The present invention also provides adjunctive therapy for treatment of lethal bacterial, fungal and viral infections such as anthrax, tularemia, escherichia and plague comprising administration of an antibacterial agent, an antifungal agent or an antiviral agent in conjunction with selective, A_{2A} adenosine receptor agonists.

The present invention provides use in preparation of a medicament for treating biological diseases that provoke inflammation either alone or in combination with a disease killing medicine. These include bacteria in combination with antibiotics, including but not limited to bacteria that cause anthrax, tularemia, plague, lyme disease and anthrax. Also included are viruses including those that cause RSV, severe acute respiratory syndrome (SARS), influenza and Ebola with or without anti-viral therapy. Also included are yeast and fungal infections with or without anti-yeast or anti-fungal agents.

The antibacterial agent, antifungal agent or antiviral agent can be co-administered (*e.g.*, simultaneously) with the A_{2A} adenosine receptor agonist or they can be can be administered either simultaneously or as a mixture or they can be administered subsequently. The subsequent administration of the A_{2A} adenosine receptor agonists can be prior to the agent, within minutes or up to about 48 hours after the administration of the agent Preferably the administration of the A_{2A} adenosine receptor agonists will be within about 24 hours and more preferably within about 12 hours.

The invention will also be useful for treating patients with sepsis, severe sepsis, and potentially, the systemic inflammatory response syndrome, in addition to septic shock. The A_{2A}AR agonists exert multiple anti-inflammatory effects early in the inflammatory cascade, and thus a short course of an A_{2A}AR agonists could produce profound benefit in serious, life-threatening infectious and inflammatory disorders of humans, including inhalational anthrax, tularemia, escherichia and plague.

The anti-inflammatory effect of A_{2A}AR agonists has been documented *in vivo,* in experimental models of meningitis, peritonitis and arthritis. The potentially fatal syndrome of bacterial sepsis is an increasingly common problem in acute care units. Sepsis and septic shock, now the eleventh leading cause of death in the United States, are increasing in frequency. Current estimates indicate that about 900,000 new cases of sepsis (approximately 60% Gram negative) occur in the United States annually with an estimated crude mortality rate of 35%. Furthermore, the mortality rate, as assessed in recent clinical trials, is approximately 25%, while approximately 10 % of patients die from their underlying disease. Shock develops in approximately 200,000 cases annually with an attributable mortality rate of 46 % (92,000 deaths). Sepsis accounts for an estimated $ 5-10 billion annually in health care expenditures. It is now widely appreciated that among hospitalized patients in non-coronary intensive care units, sepsis is the most common cause of death. Sepsis syndrome is a public health problem of major importance. A_{2A}AR agonists are anticipated to have use as a new and unique adjunctive therapeutic approach to reduce morbidity and mortality. It is believed that this treatment will improve the outcome in systemic anthrax, tularemia, escherichia and plague.

The agonists of A_{2A} adenosine receptors of the invention can inhibit neutrophil, macrophage and T cell activation and thereby reduce inflammation caused by bacterial and viral infections. The compounds, in conjunction with antibiotics or antiviral agents can prevent or reduce mortality caused by sepsis or hemolytic uremic syndrome or other inflammatory conditions. The effects of adenosine A_{2A} agonists are enhanced by type IV phosphodiesterase inhibitors such as rolipram.

The invention also provides a compound of formula I for use in medical therapy (*e.g.*, for use as an adjunct in the treatment of potentially lethal bacterial infections, such as, anthrax, tularemia, Escherichia, plague, or other bacterial or viral infections, and treatment of systemic intoxification caused by bacterial and/or viral infections, as well as the use of a compound of formula I for the manufacture of a medicament for reducing inflammation caused by the bacteria or virus or the treatment thereof in a mammal, such as a human. The compounds of the invention are also useful for treatment of treating systemic intoxification wherein the bacterial or viral agents cause inflammation either directly or as a result of treatment, *e.g.*, with an antibiotic or antiviral agent.

Sepsis is a severe illness caused by overwhelming infection of the bloodstream by toxin-producing bacteria or viruses. The infection, which can manifest as inflammation, can be caused by the bacteria or virus pathogens directly or from the treatment thereof, *i.e.*, the death of the pathogens due to treatment with antibacterial or antiviral agents. Sepsis can be also be viewed as the body's response to an infection. The infection can be caused by microorganisms or "germs" (usually bacteria) invade the body, can be limited to a particular body region (*e.g.*, a tooth abscess) or can be widespread in the bloodstream (often referred to as "septicemia" or "blood poisoning")

The systemic intoxification or inflammatory shock is often referred to as Septic shock; Bacteremic shock; Endotoxic shock; Septicemic shock; or Warm shock.

Septic shock is a serious, abnormal condition that occurs when an overwhelming infection leads to low blood pressure and low blood flow. Vital organs, such as the brain, heart, kidneys, and liver may not function properly or may fail. Septic shock occurs most often in the very old and the very young. It also occurs in people with underlying illnesses. Any bacterial organism can cause septic shock. Fungi and viruses may also cause this condition. Toxins released by the bacteria, fungi or viruses may cause direct tissue damage, and may lead to low blood pressure and/or poor organ function. These toxins can also produce a vigorous inflammatory response from the body, which contributes to septic shock.

In another aspect, the present invention also provides the use of the claimed combination for the manufacture of a medicament for the treatment of severe acute respiratory syndrome (SARS), comprising administering to a mammal in need of said therapy, an effective anti-inflammatory amount of an agonists of A_{2A} adenosine receptor and an anti-pathogenic agent, optionally with a PDE-IV inhibitor, such as, rolipram.

### DESCRIPTION OF THE FIGURES

Figure 1 illustrates the dose dependent response from the A_{2A}AR agonist ATL146e (ATL) and protection of mice from E. Coli 026:B6 LPS-induced endotoxemia. The mice were treated with IP injection of the indicated doses of ATL one hour prior to LPS (12.5 µg/kg) and at 6 hour intervals for a total of 8 doses/48 hours.
Figure 2 illustrates the doses dependent response of ATL146e (DWH) on survival of mice treated with LPS. The treatment schedule is the same as in Figure 1.
Figure 3 illustrates that the A_{2A}AR agonist ATL146e (ATL) protects mice from LPS-induced endotoxemia after a delay in the onset of therapy. Animals were treated with LPS and ATL146e (50 µg/kg) as in Figure 1 except that the first treatment with ATL was delayed for the indicated period of time.
Figure 4 illustrates that the A_{2A}AR antagonist ZM241385 (ZM) inhibits protection by ATL146e (ATL) in mice treated with LPS.
Figure 5 illustrates that the A_{2A}AR agonist ATL146e provides less protection to A_{2A}AR KO mice, relative to wild type mice, from *E. coli* 026:B6 LPS-induced endotoxemia.
Figure 6 illustrates that ATL146e (ATL) increases survival of mice injected with live E. Coli. and treated with an antibiotic (ceftriaxone) compared to mice treated with antibiotic alone. All mice were injected with 20 million E. Coli IP at time 0. Where indicated mice were treated once at time 0 with ceftriaxone or with 50 µg/kg ATL146e 8 times at 6 hour intervals..
Figure 7 illustrates the reduction of the Renal IL-6 in mice exposed to LPS/Stx2 for 6 hours using ATL-146e and ATL-203.
Figure 8 illustrates the reduction of chemokine Renal RANTES in kidney samples in mice exposed to LPS/Stx2 for 6 hours using ATL-146e and ATL-203.
Figure 9 illustrates the reduction of infiltration of neutrophils in kidneys of C57BL/6 mice using ATL-203.

### DETAILED DESCRIPTION OF THE INVENTION

The following definitions are used, unless otherwise described. Halo is fluoro, chloro, bromo, or iodo. Alkyl, alkoxy, aralkyl, alkylaryl, etc. denote both straight and branched alkyl groups; but reference to an individual radical such as "propyl" embraces only the straight chain radical, a branched chain isomer such as "isopropyl" being specifically referred to. Aryl includes a phenyl radical or an ortho-fused bicyclic carbocyclic radical having about nine to ten ring atoms in which at least one ring is aromatic. Heteroaryl encompasses a radical attached via a ring carbon of a monocyclic aromatic ring containing five or six ring atoms consisting of carbon and one to four heteroatoms each selected from the group consisting of non-peroxide oxygen, sulfur, and N(X) wherein X is absent or is H, O, (C₁-C₄)alkyl, phenyl or benzyl, as well as a radical of an ortho-fused bicyclic heterocycle of about eight to ten ring atoms derived therefrom, particularly a benz-derivative or one derived by fusing a propylene, trimethylene, or tetramethylene diradical thereto.

It will be appreciated by those skilled in the art that the compounds of formulas (I), (II), (III), and (IV) have more than one chiral center and may be isolated in optically active and racemic forms. Preferably, the riboside moiety of the compounds is derived from D-ribose, i.e., the 3',4'-hydroxyl groups are alpha to the sugar ring and the 2' and 5' groups is beta (3R, 4S, 2R, 5S). When the two groups on the cyclohexyl group are in the 1- and 4-position, they are preferably *trans.* Some compounds may exhibit polymorphism. It is to be understood that the present invention encompasses any racemic, optically-active, polymorphic, or stereoisomeric form, or mixtures thereof, of a compound of the invention, which possess the useful properties described herein, it being well known in the art how to prepare optically active forms (for example, by resolution of the racemic form by recrystallization techniques, or enzymatic techniques, by synthesis from optically-active starting materials, by chiral synthesis, or by chromatographic separation using a chiral stationary phase) and how to determine adenosine agonist activity using the tests described herein, or using other similar tests which are well known in the art.

Specific and preferred values listed below for radicals, substituents, and ranges, are for illustration only; they do not exclude other defined values or other values within defined ranges for the radicals and substituents.

Specifically, (C₁-C₈)alkyl can be methyl, ethyl, propyl, isopropyl, butyl, iso-butyl, sec-butyl, pentyl, 3-pentyl, hexyl, heptyl or octyl. As used herein, the term "cycloalkyl" encompasses bicycloalkyl (norbornyl, 2.2.2-bicyclooctyl, etc.) and tricycloalkyl (adamantyl, etc.), optionally comprising 1-2 N, O or S. Cycloalkyl also encompasses (cycloalkyl)alkyl. Thus, (C₃-C₆)cycloalkyl can be cyclopropyl, cyclobutyl, cyclopentyl, cyclohexyl and the like. (C₁-C₈)alkoxy can be methoxy, ethoxy, propoxy, isopropoxy, butoxy, iso-butoxy, sec-butoxy, pentoxy, 3-pentoxy, or hexyloxy; (C₂-C₆)alkenyl can be vinyl, allyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, or 5-hexenyl; (C₂-C₆)alkynyl can be ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, or 5-hexynyl; (C₁-C₆)alkanoyl can be acetyl, propanoyl or butanoyl; halo(C₁-C₆)alkyl can be iodomethyl, bromomethyl, chloromethyl, fluoromethyl, trifluoromethyl, 2-chloroethyl, 2-fluoroethyl, 2,2,2-trifluoroethyl, or pentafluoroethyl; hydroxy(C₁-C₆)alkyl can be hydroxymethyl, 1-hydroxyethyl, 2-hydroxyethyl, 1-hydroxypropyl, 2-hydroxypropyl, 3-hydroxypropyl, 1-hydroxybutyl, 4-hydroxybutyl, 1-hydroxypentyl, 5-hydroxypentyl, 1-hydroxyhexyl, or 6-hydroxyhexyl; (C₁-C₆)alkoxycarbonyl (CO₂R²) can be methoxycarbonyl, ethoxycarbonyl, propoxycarbonyl, isopropoxycarbonyl, butoxycarbonyl, pentoxycarbonyl, or hexyloxycarbonyl; (C₁-C₆)alkylthio can be methylthio, ethylthio, propylthio, isopropylthio, butylthio, isobutylthio, pentylthio, or hexylthio, (C₂-C₆)alkanoyloxy can be acetoxy, propanoyloxy, butanoyloxy, isobutanoyloxy, pentanoyloxy, or hexanoyloxy; aryl can be phenyl, indenyl, or naphthyl; and heteroaryl can be furyl, imidazolyl, triazolyl, triazinyl, oxazoyl, isoxazoyl, thiazolyl, isothiazoyl, pyraxolyl, pyrrolyl, pyrazinyl, tetrazolyl, puridyl (or its N-oxide), thientyl, pyrimidinyl (or its N-oxide), indolyl, isoquinolyl (or its N-oxide) or quinolyl (or its N-oxide).

Aryl denotes a phenyl radical or an ortho-fused bicyclic carbocyclic radical having about nine to ten ring atoms in which at least one ring is aromatic. Heteroaryl denotes a radical of a monocyclic aromatic ring containing five or six ring atoms consisting of carbon and 1, 2, 3, or 4 heteroatoms each selected from the group consisting of non-peroxide oxygen, sulfur, and N(Y) wherein Y is absent or is H, O, (C₁-C₈)alkyl, phenyl or benzyl, as well as a radical of an ortho-fused bicyclic heterocycle of about eight to ten ring atoms derived therefrom, particularly a benz-derivative or one derived by fusing a propylene, trimethylene, or tetramethylene diradical thereto.

The term "heterocycle" generally represents a non aromatic heterocyclic group, having from 3 to about 10 ring atoms, which can be saturated or partially unsaturated, containing at least one heteroatom (*e.g.*, 1, 2, or 3) selected from the group consisting of oxygen, nitrogen, and sulfur. Specific, "heterocycle" groups include monocyclic, bicyclic, or tricyclic groups containing one or more heteroatoms selected from the group consisting of oxygen, nitrogen, and sulfur. A "heterocycle" group also can include one or more oxo groups (=O) attached to a ring atom. Non-limiting examples of heterocycle groups include 1,3-dioxolane, 1,4-dioxane, 1,4-dithiane, 2*H*-pyran, 2-pyrazoline, 4*H*-pyran, chromanyl, imidazolidinyl, imidazolinyl, indolinyl, isochromanyl, isoindolinyl, morpholine, piperazinyl, piperidine, piperidyl, pyrazolidine, pyrazolidinyl, pyrazolinyl, pyrrolidine, pyrroline, quinuelidine, thiomorpholine, and the like.

The term "alkylene" refers to a divalent straight or branched hydrocarbon chain (*e.g.* ethylene -CH₂CH₂-).

The term "aryl(C₁-C₈)alkylene" for example includes benzyl, phenethyl, 3-phenylpropyl, naphthylmethyl.

The terms "systemic intoxification" or "inflammatory shock" refer to the build-up of toxins or an intense inflammatory response in the body due to the invasion and/or treatment of bacteria, fungi or viruses.

As used herein "anti-pathogenic agent" refers to compounds that have anti-bacterial, anti-fungal or antiviral activity.

As used herein the term "in conjunction with" refers to co-administration of an antibacterial agent, an antifungal agent or an antiviral agent with the A_{2A} adenosine receptor agonist. The agents and the A_{2A} adenosine receptor agonists can be administered either simultaneously or as a mixture or they can be administered subsequently. The subsequent administration of the A_{2A} adenosine receptor agonists can be prior to the agent, within minutes or up to about 48 hours after the administration of the agent. Preferably the administration of the A_{2A} adenosine receptor agonists will be within 24 hours and more preferably within 12 hours.

The carbon atom content of various hydrocarbon-containing moieties is indicated by a prefix designating the minimum and maximum number of carbon atoms in the moiety, *i.e.*, the prefix Cᵢ-Cⱼ indicates a moiety of the integer "i" to the integer "j" carbon atoms, inclusive. Thus, for example, (C₁-C₈)alkyl refers to alkyl of one to eight carbon atoms, inclusive.

The compounds of the present invention are generally named according to the IUPAC or CAS nomenclature system. Abbreviations which are well known to one of ordinary skill in the art may be used (*e.g.*, "Ph" for phenyl, "Me" for methyl, "Et" for ethyl, "h" for hour or hours and "rt" for room temperature).

In one embodiment, agonists of A_{2A} adenosine receptors that are useful in the practice of the present invention include compounds having the formula (I): 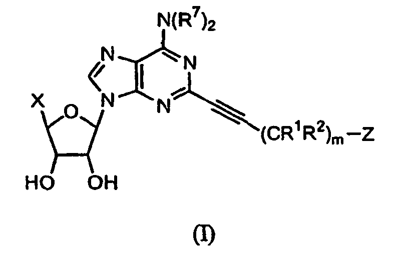 wherein
Z is CR³R⁴R⁵ or NR⁴R⁵; each R¹ is independently hydrogen, halo, -OR^{a}, -SR^{a}, (C₁-C₈)alkyl, cyano, nitro, trifluoromethyl, trifluoromethoxy, (C₃-C₈)cycloalkyl, heterocycle, hetrocycle(C₁-C₈)alkylene-, aryl, aryl(C₁-C₈)alkylene-, heteroaryl, heteroaryl(C₁-C₈)alkylene-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, R^{a}S(=O)₂-, -N=NR^{b}, or -OPO₂R^{a};
each R² is independently hydrogen, halo, (C₁-C₈)alkyl, (C₃-C₈)cycloalkyl, heterocycle, heterocycle(C₁-C₈)alkylene-, aryl, aryl(C₁-C₈)alkylene-, heteroaryl, or heteroaryl(C₁-C₈)alkylene-; or
R¹ and R² and the atom to which they are attached is C=O, C=S or C=NR^{d},
R⁴ and R⁵ together with the atoms to which they are attached form a saturated or partially unsaturated, mono-, bicyclic- or aromatic ring having 3, 4, 5, 6, 7, 8, 9 or 10 ring atoms optionally comprising 1, 2, 3, or 4 heteroatoms selected from non-peroxide oxy (-O-), thio (-S-), sulfinyl (-SO-), sulfonyl (-S(O)₂-) or amine (-NR^{b}-) in the ring;
   wherein any ring comprising R⁴ and R⁵ is substituted with from 1 to 14 R⁶ groups; wherein each R⁶ is independently halo, -OR^{a}, -SR^{a}, (C₁-C₈)alkyl, cyano, nitro, trifluoromethyl, trifluoromethoxy, (C₁-C₈)cycloalkyl, (C₆-C₁₂)bicycloalkyl, heterocycle or hetrocycle (C₁-C₈)alkylene-, aryl, aryl (C₁-C₈)alkylene-, heteroaryl, heteroaryl(C₁-C₈)alkylene-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, -NNR^{b},-OPO₂R^{a}, or two R⁶ groups and the atom to which they are attached is C=O, C=S or, two R⁶ groups together with the atom or atoms to which they are attached can form a carbocyclic or heterocyclic ring;
R³ is hydrogen, halo, -OR^{a}, -SR^{a}, (C₁-C₈)alkyl, cyano, nitro, trifluoromethyl, trifluoromethoxy, (C₃-C₈)cycloalkyl, heterocycle, hetrocycle(C₁-C₈)alkylene-, aryl, aryl(C₁-C₈)alkylene-, heteroaryl, heteroaryl(C₁-C₈)alkylene-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, R^{a}S(=O)₂-, -NNR^{b}, -OPO₂R^{a}; or if the ring formed from CR⁴R⁵ is aryl or hetreroaryl or partially unsaturated then R³ can be absent;
each R⁷ is independently hydrogen, (C₁-C₈)alkyl, (C₃-C₈)cycloalkyl, aryl or aryl(C₁-C₈)alkylene, heteroaryl, heteroaryl(C₁₋C₈)alkylene-;
X is -CH₂OR^{a}, -CO₂R^{a}, -OC(O)R^{a}, -CH₂OC(O)R^{a}, -C(O)NR^{b}R^{b}, -CH₂SR^{a}, -C(S)OR^{a}, -OC(S)R^{a}, -CH₂OC(S)R^{a} or -C(S)NR^{b}R^{c} or -CH₂N(R^{b})(R^{c});
   wherein any of the alkyl, cycloalkyl, heterocycle, aryl, or heteroaryl, groups of R¹, R², R³, R⁶ and R⁷is optionally substituted on carbon with one or more (e.g. 1, 2, 3, or 4) substituents selected from the group consisting of halo, -OR^{a}, -SR^{a}, (C₁-C₈)alkyl, cyano, nitro, trifluoromethyl, trifluoromethoxy, (C₃-C₈)cycloalkyl, (C₆-C₁₂)bicycloalkyl, heterocycle or hetrocycle(C₁-C₈)-alkylene-, aryl, aryloxy, aryl(C₁-C₈)alkylene-, heteroaryl, heteroaryl(C₁-C₈)-alkylene-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}C(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)ₚ-, R^{b}R^{c}NS(O)ₚ-, N=NR^{b}, and -OPO₂R^{a};
   wherein any (C₁-C₈)alkyl, (C₃-C₈)cycloalkyl, (C₆-C₁₂)bicycloalkyl, (C₁-C₈)alkoxy, (C₁-C₈)alkanoyl, (C₁-C₈)alkylene, or heterocycle, is optionally partially unsaturated;
each R^{a}, R^{b} and R^{c} is independently hydrogen, (C₁-C₈)alkyl, or (C₁-C₈)alkyl substituted with 1-3 (C₁-C₈)alkoxy, (C₃-C₈)cycloallcyl, (C₁-C₈)alkylthio, amino acid, aryl, aryl(C₁-C₈)alkylene, heteroaryl, or heteroaryl(C₁-C₈)alkylene; or R^{b} and R^{c}, together with the nitrogen to which they are attached, form a pyrrolidino, piperidino, morpholino, or thiomorpholino ring; and R^{d} is hydrogen or (C₁-C₆)alkyl; m is 0 to 8 and p is 0 to 2; or a pharmaceutically acceptable salt thereof.

In another embodiment, the invention includes the use of compounds of formula (1) provided that when CR⁴R⁵ is a carbocyclic ring then at least one of R¹, R², or R³ is a group other than hydrogen or at least one R⁶ group is a group other than -CH₂OH, -CO₂R^{a}, R^{a}C(=O)O-, R^{a}C(=O)OCH₂- or R^{b}R^{c}NC(=O)-; and provided that m is at least 1 when Z is NR⁴R⁵.

Specific and preferred values listed below for radicals, substituents, and ranges, are for illustration only; they do not exclude other defined values or other values within defined ranges for the radicals and substituents.

A specific value for R¹ is hydrogen, -OH, -CH₂OH, -OMe, -OAc, -NH₂, -NHMe, -NMe₂ or -NHAc.

Another specific value for R¹ is hydrogen, -OH, -OMe, -OAc, -NH₂, -NHMe, -NMe₂ or -NHAc.

Another specific value for R¹ is hydrogen, -OH, -OMe, or -NH₂.

Another specific value for R¹ is hydrogen, -OH, or -NH₂.

A more specific value for R¹ is hydrogen or -OH.

A specific value for R¹, R² and the carbon atom to which they are attached is carbonyl (C=O).

A specific value for R² is hydrogen or (C₁-C₈)alkyl, cyclopropyl, cyclohexyl or benzyl.

Another specific value for R² is hydrogen, methyl, ethyl or propyl.

Another specific value for R² is hydrogen or methyl.

A more specific value for R² is hydrogen

A specific value for R³ is hydrogen, OH, OMe, OAc, NH₂, NHMe, NMe₂ or NHAc.

Another specific value for R³ is hydrogen, OH, OMe, or NH₂.

Another specific value for R³ is hydrogen, OH, or NH₂.

A more specific value for R³ is hydrogen or OH.

A specific value for the ring comprising R⁴, R⁵ and the atom to which they are connected is cyclopentane, cyclohexane, piperidine, dihydro-pyridine, tetrahydro-pyridine, pyridine, piperazine, decaline, tetrahydro-pyrazine, dihydro-pyrazine, pyrazine, dihydro-pyrimidine, tetrahydro-pyrimidine, hexahydro-pyrimidine, pyrazine, imidazole, dihydro-imidazole, imidazolidine, pyrazole, dihydro-pyrazole, and pyrazolidine.

A more specific value for the ring comprising R⁴ and R⁵ and the atom to which they are connected is, cyclohexane, piperidine or piperazine.

A specific value for R⁶ is (C₁-C₈)alkyl, or substituted (C₁-C₈)alkyl, -OR^{a}, -CO₂R^{a}, R^{a}C(=O)-, R^{a}C(=O)O-, R^{b}R^{c}N-, R^{b}R^{c}NC(=O)-, or aryl.

Another specific value for R⁶ is (C₁-C₈)alkyl, -OR^{a}, -CO₂R^{a}, R^{a}C(=O)-, R^{a}C(=O)O-, R^{b}R^{c}N-, R^{b}R^{c}NC(=O)-, or aryl.

Another specific value for R⁶ is methyl, ethyl, butyl, OH, OR^{a}, -CO₂R^{a}, R^{a}C(=O)-, OC(=O)CH₂CH₃, -CONR^{b}R^{c}, -NR^{b}R^{c} or phenyl.

Another specific value for R⁶ is OH, OMe, methyl, ethyl, t-butyl, -CO₂R^{a}, -C(=O)NR^{b}R^{c}, -OAc, -NH₂, NHMe, -NMe₂, -NHEt or -N(Et)₂.

Another specific value for R⁶ is-(CH₂)₁₋₂OR^{a}, -(CH₂)₁₋₂C(=O)OR^{a}, -(CH₂)₁₋₂OC(=O)R^{a}, -(CH₂)₁₋₂C(=O)R^{a}, -(CH₂)₁₋₂OCO₂R^{a}, -(CH₂)₁₋₂NHR^{a}, -(CH₂)₁₋₂NR^{b}R^{c}, -(CH₂)₁₋₂OC(=O)NHR^{a}, or -(CH₂)₁₋₂OC(=O)NR^{b}R^{c}.

Another specific value for R⁶ is -CH₂OH, -CH₂OAc, -CH₂OCH₃, -CH₂C(=O)OCH₃, -CH₂OC(=O)CH₃, -CH₂C(=O)CH₃, -CH₂OCO₂CH₃, -CH₂NH(CH₃), or -(CH₂)₁₋₂N(CH₃)₂.

Another specific value for R⁶ is methyl, ethyl, t-butyl, phenyl, -CO₂R^{a}, -CONR^{b}R^{c}, or R^{a}C(=O)-.

Another specific value for R⁶ is -CH₂OH, -CH₂OAc, -C(=O)OCH₃, -C(=O)CH₃, OCO₂CH₃ -OCO₂CH₃, -CH₂NH(CH₃), or -(CH₂)₁₋₂N(CH₃)₂.

A more specific value for R⁶ is methyl, ethyl, -CO₂R^{a} -CONR^{b}R^{c}, or R⁸C(=O)-.

A specific number of R⁶ groups substituted on the R⁴R⁵ ring is from1 to about 4.

Specific values for R^{a} and R^{b} are independently hydrogen, (C₁-C₄)alkyl, aryl or aryl(C₁-C₈)alkylene.

More specific values for R^{a} and R^{b} are independently hydrogen, methyl, ethyl, phenyl or benzyl.

A more specific value for R^{a} is (C₁-C₈)alkyl.

Another specific value for R^{a} is methyl, ethyl, propyl or butyl.

A more specific value for R^{a} is methyl, ethyl, i-propyl, i-butyl or *t*e*rt*-butyl.

Another specific value for R^{b} and R^{c} is a ring

A specific value for R⁷ is hydrogen, alkyl, aryl or aryl(C₁-C₈)alkylene.

Another specific value for R⁷ is hydrogen, methyl or ethyl, phenyl or benzyl.

A more specific value for R⁷ is H, or methyl.

A specific value for -N(R⁷)₂ is amino, methylamino, dimethylamino, ethylamino, pentylamino, diphenylethylamino, pyridylmethylamino, diethylamino or benzylamino.

A specific value for -N(R⁷)₂ is amino, methylamino, dimethylamino, ethylamino, diethylamino diphenylethylamino, pentylamino or benzylamino.

A specific value for N(R⁷)₂ is amino, or methylamino.

A specific value for X is -CH₂OR^{a}, -CO₂R^{a}, -OC(O)R^{a}, -CH₂OC(O)R^{a}, -C(O)NR^{b}R^{c}.

Another specific value for X is -CH₂OR^{a} or -C(O)NR^{b}R^{c}.

A more specific value for X is -CH₂OH or -C(O)NHCH₂CH₃.

A specific value for m is 0,1, or 2.

A more specific value for m is 0, or 1.

Specific examples of rings comprising R⁴, R⁵ and the atom to which they are connected include: 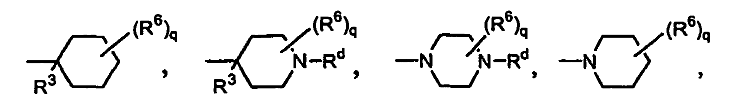 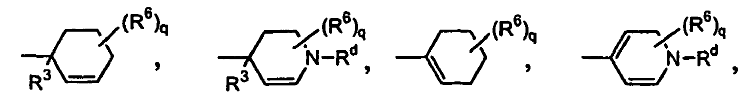 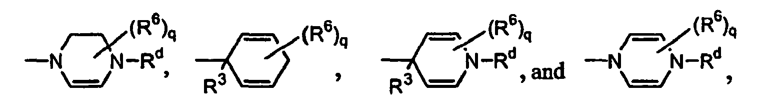 where q is from 0 to 14 and R^{d} is hydrogen, provided that when q is zero then R^{d} is not hydrogen.

More specific examples of rings comprising R⁴, R⁵ and the atom to which they are connected include:

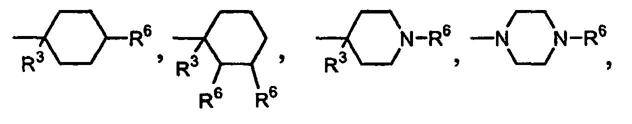

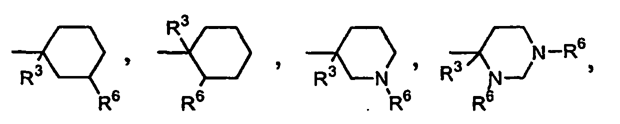

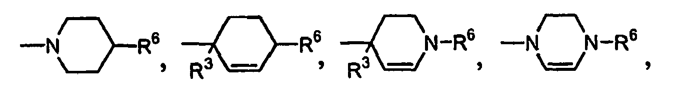

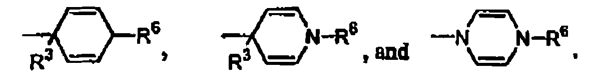

Specific values for the ring comprising R⁴, R⁵ and the atom to which they are connected are 2-methyl cyclohexane, 2,2-dimethylcyclohexane, 2-phenylcyclohexane, 2-ethylcyclohexane, 2,2-diethylcyclohexane, 2-tert-butyl cyclohexane, 3-methyl cyclohexane, 3,3-dimethylcyclohexane, 4-methyl cyclohexane, 4-ethylcyclohexane, 4-phenyl cyclohexane. 4-tert-butyl cyclohexane, 4-caboxymethyl cyclohexane, 4-carboxyethyl cyclohexane, 3,3,5,5-tetramethyl cyclohexane, 2,4-dimethyl cyclopentane. 4-cyclohexanecarboxyic acid, 4-cyclohexanecarboxyic acid esters, or 4-methyloxyalkanoyl-cyclohexane.

More specific values for the ring comprising R⁴, R⁵ and the atom to which they are connected are 4-piperidine, 4-piperidine-1-carboxylic acid, 4-piperidine-1-carboxyic acid methyl ester, 4-piperidine-1-carboxylic acid ethyl ester, 4-piperidine-1-camoxylic acid propyl ester, 4-piperidine-1-carboxylic acid tert-butyl ester, 1-piperidine, 1-piperidine-4-carboxylic acid methyl ester, 1-piperidine-4-carboxylic acid ethyl ester, 1-piperidine-4-carboxylic acid propyl ester, 1-piperidine-4-caboxylic acid tert-butyl ester, 1-piperidine-4-carboxylic acid methyl ester, 3-piperidine, 3-piperidine-1-carboxylic acid, 3-piperidine-1-carboxylic acid methyl ester, 3-piperidine-1-carboxylic acid tert-butyl ester, 1,4-piperazine, 4-piperazine-1-carboxylic acid, 4-piperazine-1-carboxylic acid methyl ester, 4-piperazine-1-carboxylic acid ethyl ester, 4-piperazine-1-carboxylic acid propyl ester, 4-piperazine-1-carboxylic acid tert-butylester, 1,3-piperazine, 3-piperazine-1-carboxylic acid, 3-piperazine-1-carboxylic acid methyl ester, 3-piperazine-1-carboxylic acid ethyl ester, 3-piperazine-1-carboxylic acid propyl ester, 3-piperidine-1-carboxylic acid tert-butylester, 1-piperadine-3-carboxylic acid methyl ester, 1-piperidine-3-carboxylic acid ethyl ester, 1-piperidine-3-carboxylic acid propyl ester or 1-piperidine-3-caboxylic acid tert-butyl ester.

Another group of specific values for the ring comprising R⁴ and R⁵ are 2-methyl cyclohexane, 2,2-dimethylcyclohexane, 2-phenyl cyclohexane, 2-ethylcyclohexane, 2,2-diethylcyclohexane, 2-tert-butyl cyclohexane, 3-methyl cyclohexane, 3,3-dimethylcyclohexane, 4-methyl cyclohexane, 4-ethylcyclohexane, 4-phenyl cyclohexane, 4-tert-butyl cyclohexane, 4-carboxymethyl cyclohexane, 4-carboxyethyl cyclohexane, 3,3,5,5-tetramethyl cyclohexane, 2,4-dimethyl cyclopentane, 4-piperidine-1-carboxylic acid methyl ester, 4-piperidine-1-carboxylic acid tert-butyl ester 4-piperidine, 4-piperazine-1-carboxylic acid methyl ester, 4-piperidine-1-carboxylic acid tert-butylester, 1-piperidine-4-carboxylic acid methyl ester, 1-piperidine-4-caboxylic acid tert-butyl ester, tert-butylester, 1-piperidine-4-carboxylic acid methyl ester, or 1-piperidine-4-caboxylic acid tert-butyl ester, 3-piperidine-l-carboxylic acid methyl ester, 3-piperidine-l-carboxylic acid tert-butyl ester, 3-piperidine, 3-piperazine-1-carboxylic acid methyl ester, 3-piperidine-1-carboxylic acid *tert-*butylester, 1-piperidine-3-carboxylic acid methyl ester, 1-piperidine-3-caboxylic acid *tert*-butyl ester

Specific compounds of formula (I) are those wherein each R⁷ is H, X is ethylaminocarbonyl and

R¹ is hydroxy, R² is hydrogen, and Z is 4-carboxycyclohexyl, wherein R^{a} is hydrogen, **4**; Z is 4-methoxycarbonylcyclohexylmethyl, R^{a} is methyl, 5; R¹ and R² together are oxo, Z is a 4-carbonylcyclohexyl group, wherein R^{a} is methyl, methoxy, ethyl, ethoxy, propyl, isopropoxy, -isobutyl, *tert*-butyl, amine, methylamine or dimethylamine, **6**.

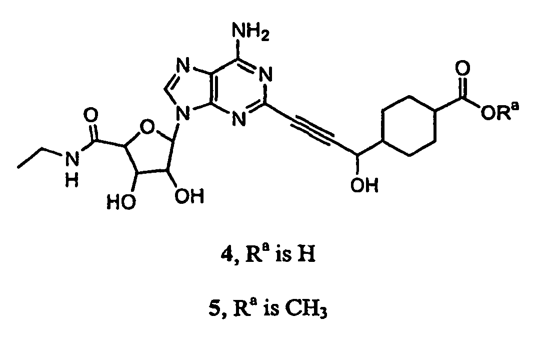

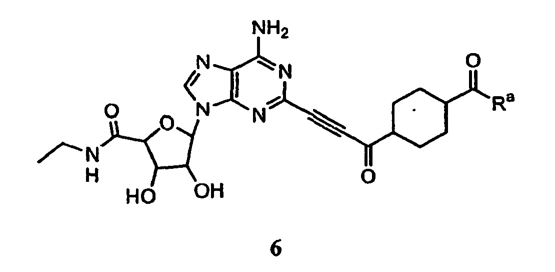

Another group of specific compounds of formula (I) are those
wherein each R⁷ is H, X is ethylaminocarbonyl, R¹ is hydroxy, R² is hydrogen, and Z is a substituted 4-(methyleneoxycarbonyl)cyclohexyl group, wherein R^{a} is methyl, ethyl, propyl, *tert-butyl,* methoxy, ethoxy, methylamine or dimethylamine, **7**; or R¹ and R² together are oxo, and Z is a substituted
- (methyleneoxycarbonyl)cyclohexyl group, wherein R^{a} is methyl, ethyl, propyl, tert-butyl, methoxy, ethoxy, methylamine or dimethylamine, 8.

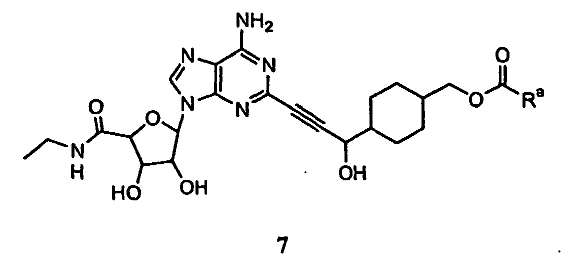

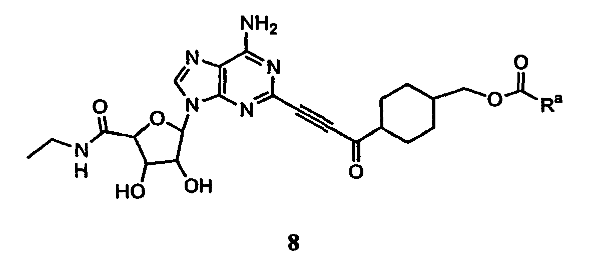

Another group of specific compounds of formula (I) are those wherein each R⁷ is H, X is ethylaminocarbonyl, and R¹ and R² are each hydrogen, and Z is a 1-piperidyl-4-carboxylic acid or ester group, wherein R^{a} is hydrogen, methyl, ethyl, propyl, isopropyl, or t-butyl, **9**; R¹ and R² together are oxo, and Z is a 1-piperidyl-4-carboxylic acid or ester group, wherein R^{a} is hydrogen, methyl, ethyl, propyl, isopropyl, or t-butyl, **10**; R¹ and R² are each hydrogen and Z is a 4-(methyleneoxycarbonyl)piperidin-4-yl group wherein R^{a} is methyl, ethyl, propyl or t-butyl, amine, methylamine, dimethylamine, **11**; or R¹ and R² together are oxo, and Z is a 4-(methyleneoxycarbonyl)piperidin-4-yl wherein R^{a} is methyl, ethyl, propyl or t-butyl, amine, methylamine, dimethylamine, **12**; R¹ and R² are each hydrogen and Z is a 4-(methyleneoxy-carbonyl)piperidin-4-yl-oxy wherein R^{a} is hydrogen, methyl, ethyl, propyl isopropyl, isobutyl, or t-butyl, **13** or R¹ and R² together are oxo, Z is a 4-(methyleneoxycarbonyl)piperidin-4-yl-oxy wherein R^{a} is hydrogen, methyl, ethyl, propyl, isopropyl, isobutyl, or t-butyl, **14**.

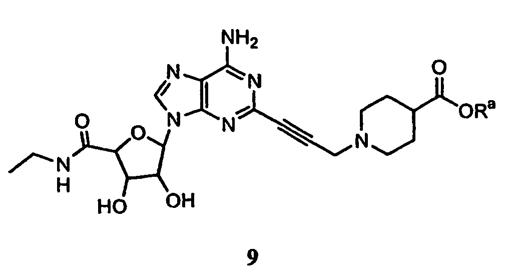

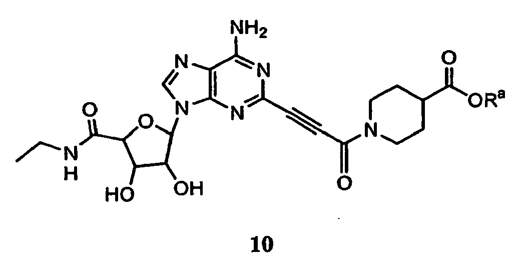

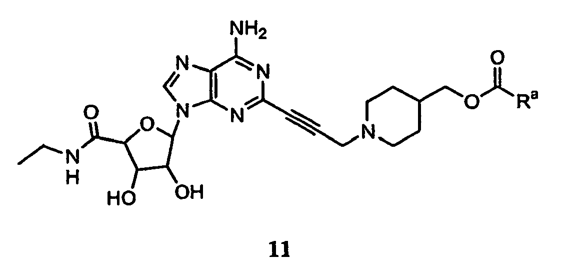

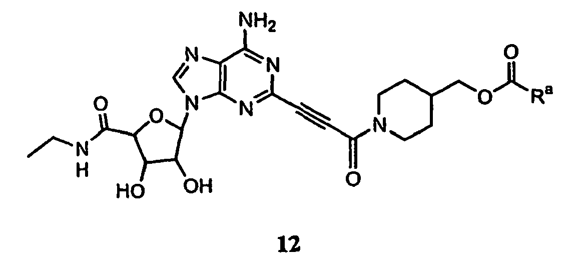

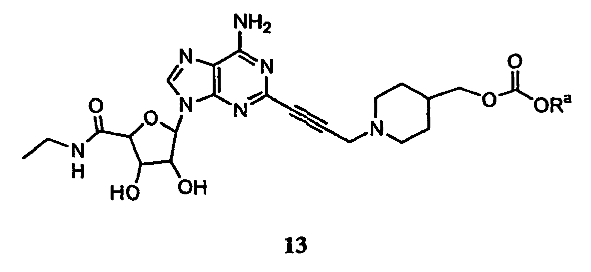

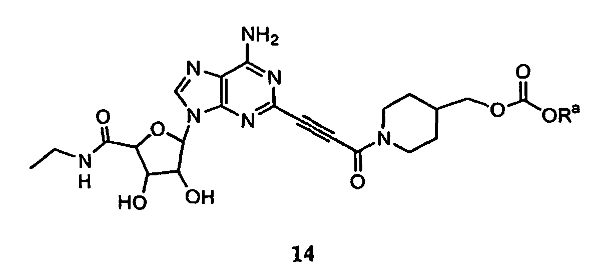

Another group of specific compounds of formula (I) are those wherein each R⁷ is H, X is ethylaminocarbonyl, R¹ and R² are each hydrogen, and Z is a 4-piperidyl-1-carboxylic acid or ester group, wherein R^{a} is methyl, ethyl, propyl, isopropyl, isobutyl, or t-butyl, **15**, R¹ is hydroxy, R² is hydrogen, and Z is a 4-piperidyl-1-carboxylic acid or ester group, wherein R^{a} is methyl, ethyl, propyl, isopropyl, isobutyl, or t-butyl, **16**; or R¹ and R² together are oxo, and Z is a 4-piperidyl-1-carboxylic acid or ester group, wherein R^{a} is methyl, ethyl, propyl, isopropyl, isobutyl, or t-butyl, **17**.

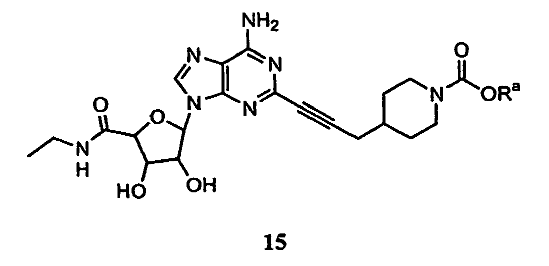

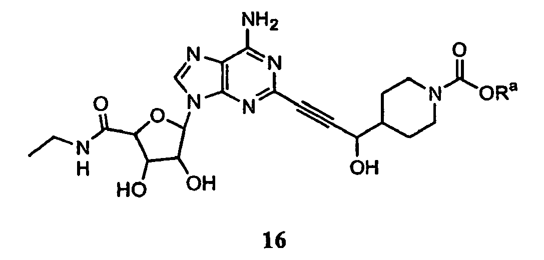

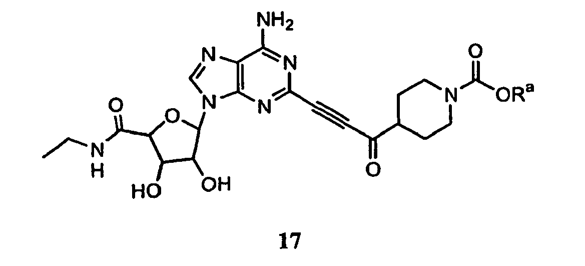

Another group of specific compounds of formula (I) are those wherein each R⁷ is H, X is ethylaminocarbonyl, R¹ and R² are each hydrogen, Z is a 4-piperazine-1-carboxylic acid or ester group wherein R^{a} is methyl, ethyl, isopropyl, isobutyl, or t-butyl, **18**; or R¹ and R² together are oxo, Z is a 4-piperazine-1-carboxylic acid or ester group wherein R^{a} is methyl, ethyl, isopropyl, isobutyl, or t-butyl, **19**.

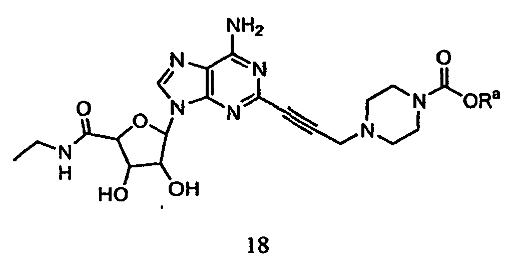

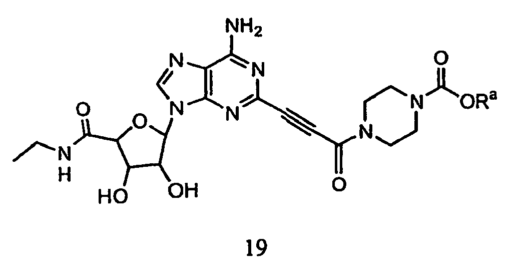

Additional compounds useful to practice the invention are depicted in tables 1, 2, 3, 4, 5, 6 and 7 below:

**Table 1**

| | | | | |
|---|---|---|---|---|
| 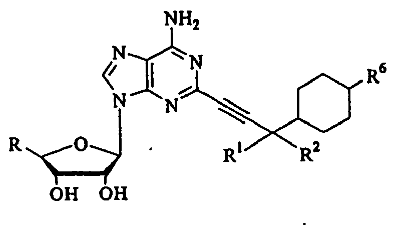 | | | | |

| **Compound** | **R** | **R¹** | **R²** | **R⁶** |
|---|---|---|---|---|
| ATL2037 | NECA | H | H | CH₂OH |
| MP9056 | NECA | OH | H | CH₂OH |
| ATL146a | NECA | H | H | CO₂H |
| MP9057 | NECA | OH | H | CO₂H |
| ATL146e | NECA | H | H | CO₂Me |
| MP9058 | NECA | OH | H | CO₂Me |
| JR2145 | CH₂OH | H | H | CO₂Me |
| MP9059 | CH₂OH | OH | H | CO₂Me |
| ATL193 | NECA | H | H | CH₂OAc |
| MP9060 | NECA | OH | H | CH₂Oac |
| JR2147 | CH₂OH | H | H | CH₂Oac |
| MP9061 | CH₂OH | OH | H | CH₂Oac |
| JR3023 | NECA | H | H | CH₂N(CH₃)₂ |
| MP9062 | NECA | OH | H | CH₂N(CH₃)₂ |
| JR3021 | NECA | H | H ₂NHBoc | COOCH₂CH |
| MP9063 | NECA | OH | H ₂NHBoc | COOCH₂CH |
| JR3033 | NECA | H | H | COOCH₂CH₂NH₂ |
| MP9064 | NECA | OH | H | COOCH₂CH₂NH₂ |
| JR3037 | NECA | H | H | CONHCH₂CH₃ |
| MP9065 | NECA | OH | H | CONHCH₂CH₃ |
| JR3055 | NECA | H | H | CONH₂ |
| MP9072 | NECA | OH | H | CONH₂ |
| JR3065 | NECA | H | H | CONHMe |
| MP9066 | NECA | OH | H | CONHMe |
| JR3067B | NECA | H | H | Me, cis CO₂Me |
| MP9067 | NECA | OH | H | Me, cis CO₂Me |
| JR3067A | NECA | H | H | Me, trans CO₂Me |
| MP9068 | NECA | OH | H | Me, trans CO₂Me |
| JR3087 | NECA | H | H | CH₂CH₃ |
| MP9069 | NECA | OH | H | CH₂CH₃ |
| JR3159A | NECA | OH | H | H |
| JR3159B | NECA | OH | H | H |
| JR3119 | NECA | H | H | COCH₃ |
| MP9070 | NECA | OH | H | COCH₃ |
| JR3121 | NECA | H | H | CHCH₃(OH) |
| MP9071 | NECA | OH | H | CHCH₃(OH) |
| JR3139 | NECA | OH | C₆H₁₁ | H |

| | | | | |
|---|---|---|---|---|
| NECA = CH₃CH₂N(H)C(O)- | | | | |

**Table 2**

| | | | |
|---|---|---|---|
| 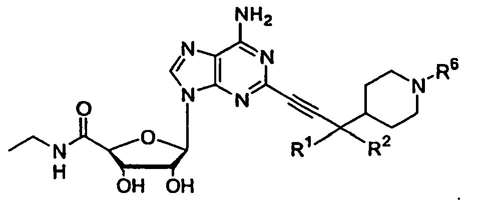 | | | |

| **Compound** | **R¹** | **R²** | **R⁶** |
|---|---|---|---|
| JR3261 | H | H | H |
| JR3259 | H | H | CO₂tBu |
| JR3269 | H | H | CO₂Et |
| JR4011 | H | H | CO₂iBu |
| JR4009 | H | H | CO₂iPr |
| JR4007 | H | H | COMe |
| JR4051 | H | H | COC(CH₃)₃ |
| JR4047 | H | H | COCH₂(CH₃)₃ |
| MP9047 | H | H | COCH₃ |
| MP9048 | H | H | C(O)N(CH₃)₂ |
| MP9049 | H | H | C(O)N(CH₃)Et |
| MP9050 | H | H | C(0)N(CH₃)iPr |
| MP9051 | H | H | C(O)N(CH₃)iBu |
| MP9052 | H | H | C(O)NH(CH₃) |
| MP9053 | H | H | C(O)NH(Et) |
| MP9054 | H | H | C(O)NH(iPr) |
| MP9055 | H | H | C(O)NH(iBu) |
| TX3261 | OH | H | H |
| TX3259 | OH | H | CO₂tBu |
| TX3269 | OH | H | CO₂Et |
| TX4011 | OH | H | CO₂iBu |
| TX4009 | OH | H | CO₂iPr |
| TX4007 | OH | H | COMe |
| TX4051 | OH | H | COC(CH₃)₃ |
| TX4047 | OH | H | COCH₂(CH₃)₃ |
| TX9047 | OH | H | COCH₃ |
| TX9048 | OH | H | C(O)N(CH₃)₂ |
| TX9049 | OH | H | C(O)N(CH₃)Et |
| TX9050 | OH | H | C(O)N(CH₃)iPr |
| TX9051 | OH | H | C(O)N(CH₃)iBu |
| TX9052 | OH | H | C(O)NH(CH₃) |
| TX9053 | OH | H | C(O)NH(Et) |
| TX9054 | OH | H | C(O)NH(iPr) |
| TX9055 | OH | H | C(O)NH(iBu) |

**Table 3**

| | | | |
|---|---|---|---|
| 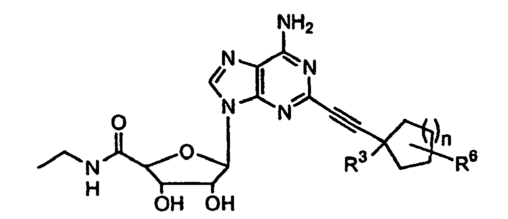 | | | |

| **Compound** | **n** | **R³** | **R⁶** |
|---|---|---|---|
| JR3135 | 1 | OH | H |
| JR3089 | 2 | OH | H |
| JR3205 | 2 | NH₂ | H |
| JR3177A | 2 | OH | 2-CH₃ |
| JR3177B | 2 | OH | 2-CH₃ |
| JR3181A | 2 | OH | 2-CH₃ |
| JR3181B | 2 | OH | 2-CH₃ |
| JR3227 | 2 | OH | 2-C(CH₃)₃ |
| JR9876 | 2 | OH | 2-C₆H₅ |
| JR3179 | 2 | OH | 3-CH₃ |
| JR3221 | 2 | OH (R) | 3-CH₃ (R) |
| ATL 203 | 2 | OH (S) | 3-CH₃ (R) |
| MP9041 | 2 | OH (R) | 3-CH₃ (S) |
| MP9042 | 2 | OH (S) | 3-CH₃ (S) |
| JR3201B | 2 | OH | 3-(CH₃)₂ |
| MP9043 | 2 | OH (R) | 3-CH₂CH₃ (R) |
| MP9044 | 2 | OH (S) | 3-CH₂CH₃ (R) |
| MP9045 | 2 | OH (R) | 3-CH₂CH₃ (S) |
| MP9046 | 2 | OH (S) | 3-CH₂CH₃ (S) |
| JR3163 | 2 | OH | 3-(CH₃)₂, 5-(CH₃)₂ |
| JR9875 | 2 | OH | 4-CH₃ |
| JR3149 | 2 | OH | 4-C₂H₅ |
| JR3203 | 2 | OH | 4-C(CH₃)₃ |
| JR3161 | 2 | OH | 4-C₆H₅ |

**Table 4**

| | | | |
|---|---|---|---|
| 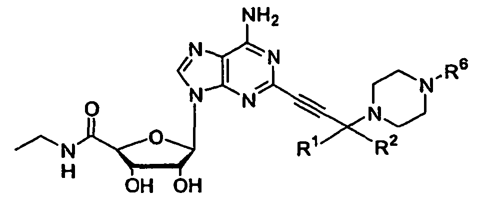 | | | |

| **Compound** | **R¹** | **R²** | **R⁶** |
|---|---|---|---|
| JR3213 | H | H | CO₂Et |
| JR3281 | H | H | CO₂tBu |
| JR3289 | H | H | H |
| JR4025 | H | H | cyclohexyl |
| JR4053 | H | H | COMe |
| JR4049 | H | H | CO₂iBu |
| JR3283 | H | H | 2-Pyrimidinyl |
| MP9029 | H | H | COMe |
| MP9030 | H | H | COC(CH₃)₃ |
| MP9031 | H | H | COCH₂(CH₃)₃ |
| MP9032 | H | H | COCH₃ |
| MP9033 | H | H | C(O)N(CH₃)₂ |
| MP9034 | H | H | C(O)N(CH₃)Et |
| MP9035 | H | H | C(O)N(CH₃)iPr |
| MP9036 | H | H | C(O)N(CH₃)iBu |
| MP9037 | H | H | C(O)NH(CH₃) |
| MP9038 | H | H | C(O)NH(Et) |
| MP9039 | H | H | C(O)NH(iPr) |
| MP9040 | H | H | C(O)NH(iBu) |

**Table 5**

| | | | | |
|---|---|---|---|---|
| 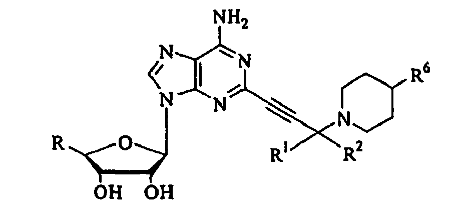 | | | | |

| **Compound** | **R** | **R¹** | **R²** | **R⁶** |
|---|---|---|---|---|
| MP9021 | NECA | H | H | CH₂OH |
| MP9022 | NECA | H | H | CO₂H |
| JR3251 | NECA | H | H | CO₂Me |
| JR3279 | NECA | H | H | CO₂Et |
| MP9027 | CH₂OH | H | H | CO₂Me |
| MP9028 | NECA | H | H | CO₂MeCH₂OAc |
| MP9015 | CH₂OH | H | H | CH₂OAc |
| MP9016 | NECA | H | H | CH₂N(CH₃)₂ |
| MP9017 | NECA | H | H | COOCH₂CH₂NHBoc |
| MP9018 | NECA | H | H | COOCH₂CH₂NH₂ |
| MP9019 | NECA | H | H | CONHCH₂CH₃ |
| MP9020 | NECA | H | H | CONH₂ |
| MP9023 | NECA | H | H | CONHMe |
| MP9024 | NECA | H | H | CH₂CH₃ |
| MP9025 | NECA | H | H | COCH₃ |
| MP9026 | NECA | H | H | CHCH₃(OH) |

| | | | | |
|---|---|---|---|---|
| NECA = CH₃CH₂N(H)C(O)- | | | | |

**Table 6**

| | | | | |
|---|---|---|---|---|
| 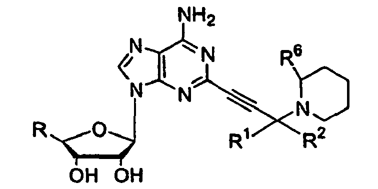 | | | | |

| **Compound** | **R** | **R¹** | **R²** | **R⁶** |
|---|---|---|---|---|
| MP9001 | NECA | H | H | CH₂OH |
| MP9002 | NECA | H | H | CO₂H |
| JR3253 | NECA | H | H | CO₂Me |
| MP9003 | CH₂OH | H | H | CO₂Me |
| MP9004 | NECA | H | H | CH₂OAc |
| MP9005 | CH₂OH | H | H | CH₂OAc |
| MP9006 | NECA | H | H | CH₂N(CH₃)₂ |
| MP9007 | NECA | H | H | COOCH₂CH₂NHBoc |
| MP9008 | NECA | H | H | COOCH₂CH₂NH₂ |
| MP9009 | NECA | H | H | CONHCH₂CH₃ |
| MP9010 | NECA | H | H | CONH₂ |
| MP9011 | NECA | H | H | CONHMe |
| MP9012 | NECA | H | H | CH₂CH₃ |
| MP9013 | NECA | H | H | COCH₃ |
| MP9014 | NECA | H | H | CHCH₃(OH) |

| | | | | |
|---|---|---|---|---|
| NECA = CH₃CH₂N(H)C(O)- | | | | |

**Table 7**

| | | | | |
|---|---|---|---|---|
| 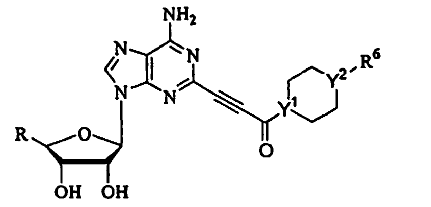 | | | | |

| ***Compound*** | **R** | **Y** | **Y'** | **R6** |
|---|---|---|---|---|
| RJ1111 | NECA | CH | CH | CO₂Me |
| RJ1112 | NECA | CH | N | CO₂Me |
| RJ1113 | NECA | N | CH | CO₂Me |
| RJ1114 | NECA | N | N | CO₂Me |
| RJ1115 | NECA | CH | CH | CH₂OH |
| RJ1116 | NECA | CH | N | CH₂OH |
| RJ1117 | NECA | N | CH | CH₂OH |
| RJ1118 | NECA | N | N | CH₂OH |
| RJ1119 | NECA | CH | CH | CO₂H |
| RJ1120 | NECA | CH | N | CO₂H |
| RJ1121 | NECA | N | CH | CO₂H |
| RJ1122 | NECA | N | N | CO₂H |
| RJ1123 | NECA | CH | CH | CH₂OAc |
| RJ1124 | NECA | CH | N | CH₂OAc |
| RJ1125 | NECA | N | CH | CH₂OAc |
| RJ1126 | NECA | N | N | CH₂OAc |
| RJ1127 | NECA | CH | CH | CONH₂ |
| RJ1128 | NECA | CH | N | CONH₂ |
| RJ1129 | NECA | N | CH | CONH₂ |
| RJ1130 | NECA | N | N | CONH₂ |
| RJ1131 | NECA | CH | CH | CONHMe |
| RJ1132 | NECA | CH | N | CONHMe |
| RJ1133 | NECA | N | CH | CONHMe |
| RJ1134 | NECA | N | N | CONHMe |
| RJ1135 | NECA | CH | CH | CO₂tBu |
| RJ1136 | NECA | CH | N | CO₂tBu |
| RJ1137 | NECA | N | CH | CO₂tBu |
| RJ1138 | NECA | N | N | CO₂tBu |
| RJ1139 | NECA | CH | CH | CO₂Et |
| RJ1140 | NECA | CH | N | CO₂Et |
| RJ1141 | NECA | N | CH | CO₂Et |
| RJ1142 | NECA | N | N | CO₂Et |
| RJ1143 | NECA | CH | CH | CO₂iBu |
| RJ1144 | NECA | CH | N | CO₂iBu |
| RJ1145 | NECA | N | CH | CO₂iBu |
| RJ1146 | NECA | N | N | CO₂iBu |
| RJ1147 | NECA | CH | CH | CO₂iPr |
| RJ1148 | NECA | CH | N | CO₂iPr |
| RJ1149 | NECA | N | CH | CO₂iPr |
| RJ1150 | NECA | N | N | CO₂iPr |
| RJ1151 | NECA | CH | CH | COMe |
| RJ1152 | NECA | CH | N | COMe |
| RJ1153 | NECA | N | CH | COMe |
| RJ1154 | NECA | N | N | COMe |
| RJ1155 | NECA | CH | CH | COC(CH₃)₃ |
| RJ1156 | NECA | CH | N | COC(CH₃)₃ |
| RJ1157 | NECA | N | CH | COC(CH₃)₃ |
| RJ1158 | NECA | N | N | COC(CH₃)₃ |
| RJ1159 | NECA | CH | CH | COCH₂(CH₃)₃ |
| RJ1160 | NECA | CH | N | COCH₂(CH₃)₃ |
| RJ1161 | NECA | N | CH | COCH₂(CH₃)₃ |
| RJ1162 | NECA | N | N | COCH₂(CH₃)₃ |
| RJ1163 | NECA | CH | CH | C(O)N(CH₃)₂ |
| RJ1164 | NECA | CH | N | C(O)N(CH₃)₂ |
| RJ1165 | NECA | N | CH | C(O)N(CH₃)2 |
| RJ1166 | NECA | N | N | C(O)N(CH₃)₂ |
| RJ1167 | NECA | CH | CH | C(O)N(CH₃)Et |
| RJ1168 | NECA | CH | N | C(O)N(CH₃)Et |
| RJ1169 | NECA | N | CH | C(O)N(CH₃)Et |
| RJ1170 | NECA | N | N | C(O)N(CH₃)Et |
| RJ1171 | NECA | CH | CH | C(O)N(CH₃)iPr |
| RJ1172 | NECA | CH | N | C(O)N(CH₃)iPr |
| RJ1173 | NECA | N | CH | C(O)N(CH₃)iPr |
| RJ1174 | NECA | N | N | C(O)N(CH₃)iPr |
| RJ1175 | NECA | CH | CH | C(O)N(CH₃)iBu |
| RJ1176 | NECA | CH | N | C(O)N(CH₃)iBu |
| RJ1177 | NECA | N | CH | C(O)N(CH₃)iBu |
| RJ1178 | NECA | N | N | C(O)N(CH₃)iBu |
| RJ1179 | NECA | CH | CH | C(O)NH(Et) |
| RJ1180 | NECA | CH | N | C(O)NH(Et) |
| RJ1181 | NECA | N | CH | C(O)NH(Et) |
| RJ1182 | NECA | N | N | C(O)NH(Et) |
| R11183 | NECA | CH | CH | C(O)NH(iPr) |
| RJ1184 | NECA | CH | N | C(O)NH(iPr) |
| RJ1185 | NECA | N | CH | C(O)NH(iPr) |
| RJ1186 | NECA | N | N | C(O)NH(iPr) |
| RJ1187 | NECA | CH | CH | C(O)NH(iBu) |
| RJ1188 | NECA | CH | N | C(O)NH(iBu) |
| RJ1189 | NECA | N | CH | C(O)NH(iBu) |
| RJ1190 | NECA | N | N | C(O)NH(iBu) |
| RJ1191 | NECA | CH | CH | CH₂OCOCH₃ |
| RJ1192 | NECA | N | CH | CH₂OCOCH₃ |
| RJ1193 | NECA | CH | CH | CH₂OCOEt |
| RJ1194 | NECA | N | CH | CH₂OCOEt |
| RJ1195 | NECA | CH | CH | CH₂COiPr |
| RJ1196 | NECA | N | CH | CH₂OCOiPr |
| RJ1197 | NECA | CH | CH | CH₂OCOiBu |
| RJ1198 | NECA | N | CH | CH₂OCOiBu |

| | | | | |
|---|---|---|---|---|
| NECA = CH₃CH₂N(H)C(O)- | | | | |

Examples of anti-bacterial agents suitable for use in the present invention include, acediasulfone, acetosulfone, amikacin, amoxicillin, amphotericin B, ampicillin, apramycin, arbekacin, aspoxicillin, aztreonam, brodimoprim, butirosin, capreomycin, carumonam, cefadroxil, cefatrizine, cefclidin, cefdinir, cefditoren, cefepime, cefetamet, cefmenoxime, cefminox, cefodizime, ceforanide, cefotaxime, cefotiam, cefozopran, cefpirome, cefprozil, cefroxadine, ceftazidime, cefteram, ceftibuten, ceftriaxone, cefuzonam, cephalexin, cephaloglycin, cephalosporin C, cephradine, ciprofloxacin, clinafloxacin, colistin, cyclacillin, dapsone, diathymosulfone, dibekacinm, enviomycinm, epicillin, fortimicin(s), gentamicin(s), gramicidin S, isepamicin, kanamycin(s), lucensomycin, lymecycline, micronomicin, natamycin, neomycin, netilmicin, paromomycin, pazufloxacin, penicillin N, peplomycin, perimycin A, polymyxin, p-sulfanilylbenzylamine, ribostamycin, ristocetin, sisomicin, sparfloxacin, succisulfone, 2-p-sulfanilylanilinoethanol, 4,4'-sulfinyldianiline, sulfachrysoidine, sulfamidochrysoidine, sulfanilic acid, sulfoxone, teicoplanin, tetroxoprim, thiazolsulfone, tigemonam, tobramycin, tosufloxacin, trimethoprim, trovafloxacin, tuberactinomycin, vancomycin. Preferred antibiotic agents are ciprofloxacin and ceftriaxone.

Examples of anti-fungal (anti-yeast) agents suitable for use in the present invention include, amphotericin B, azaserine, candicidin(s), lucensomycin, mepartricin, natamycin, nystatin, tubercidin.

Examples of antiviral agents suitable for use in the present invention include, abacavir, acyclovir, amantadine, famciclovir, foscavir, ganciclovir, indinavir, lamivudine, lopinavir, ritonavir.

In another embodiment, agonists of A_{2A} adenosine receptors that are useful in the practice of the present invention include compounds having the formula (II): 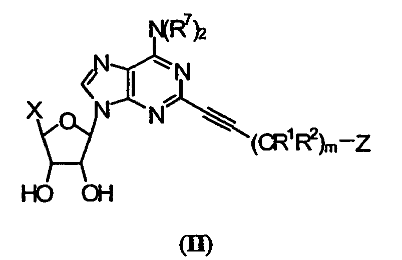 wherein Z is CR³R⁴R⁵; each R¹, R² and R³ is hydrogen; R⁴ and R⁵ together with the carbon atom to which they are attached form a cycloalkyl ring having 3, 4, 5, 6, 7, 8, 9 or 10 ring atoms; and
wherein the ring comprising R⁴ and R⁵ is substituted with -(CH₂)₀₋₆-Y; where Y is -CH₂OR^{a}, -CO₂R^{a}, -OC(O)R^{a}, -CH₂OC(O)R^{a}, -C(O)NR^{b}R^{c},-CH₂SR^{a}, -C(S)OR^{a}, -OC(S)R^{a}, -CH₂OC(S)R^{a} or C(S)NR^{b}R^{c} or -CH₂N(R^{b})(R^{c});
each R⁷ is independently hydrogen, (C₁-C₈)alkyl, (C₃-C₈)cycloalkyl, aryl or aryl(C₁-C₈)alkylene;
X is -CH₂OR^{a}, -CO₂R^{a}, -OC(O)R^{a}, -CH₂OC(O)R^{a}, -C(O)NR^{b}R^{c}, -CH₂SR^{a}, -C(S)OR^{a}, -OC(S)R^{a}, -CH₂OC(S)R^{a} or C(S)NR^{b}R^{c} or -CH₂N(R^{b})(R^{c});
each R^{a}, R^{b} and R^{c} is independently hydrogen, (C₁-C₈)alkyl, or (C₁-C₈)alkyl substituted with 1-3 (C₁-C₈)alkoxy, (C₃-C₈)cycloalkyl, (C₁-C₈)alkylthio, amino acid, aryl, aryl(C₁-C₈)alkylene, heteroaryl, or heteroaryl(C₁-C₈)alkylene; or R^{b} and R^{c}, together with the nitrogen to which they are attached, form a pyrrolidino, piperidino, morpholino, or thiomorpholino ring; and m is 0 to about 6; or a pharmaceutically acceptable salt thereof.

A specific value for -N(R⁷)₂ is amino, monomethylamino or cyclopropylamino.

A specific value for Z is carboxy- or -(C₁-C₄)alkoxycarbonyl-cyclohexyl(C₁-C₄)alkyl.

A specific value for R^{a} is H or (C₁-C₄)alkyl, i.e., methyl or ethyl.

A specific value for R^{b} is H, methyl or phenyl.

A specific value for R^{c} is H, methyl or phenyl.

A specific value for -(CR¹R²)ₘ- is -CH₂- or -CH₂-CH₂-.

A specific value for X is CO₂R^{a}, (C₂-C₅)alkanoylmethyl or amido.

A specific value for Y is CO₂R^{a}, (C₂-C₅)alkanoylmethyl or amido.

A specific value for m is 1.

Specific A_{2A} adenosine receptor agonists suitable for use with the present invention having formula (II) include those described in US Patent No: 6, 232,297. Preferred compounds of formula (II) are those wherein each R⁷ is H, X is ethylaminocarbonyl and Z is 4-carboxycyclohexylmethyl (DWH-146a), Z is 4-methoxycarbonylcyclohexylmethyl (DWH-146e), Z is 4-isopropylcarbonyl-cyclohexylmethyl (AB-1), Z is 4-acetoxymethyl-cyclohexylmethyl (JMR-193) or Z is 4-pyrrolidine-1-carbonylcyclohexylmethyl (AB-3). These comounds are depicted below.

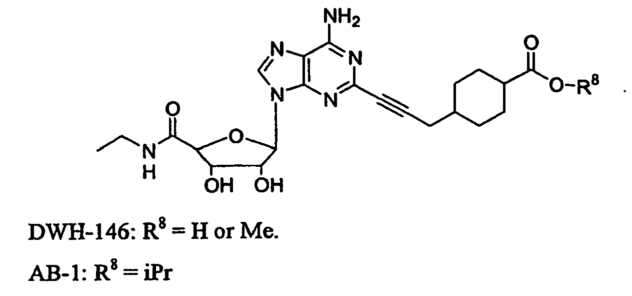

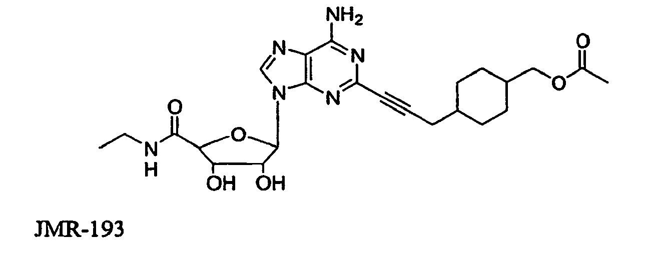

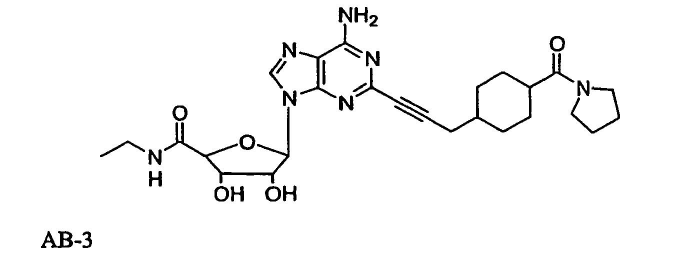

The specific A_{2A} adenosine receptor agonists suitable for use with the present invention having formula (II) include those described in U.S. Patent No. 6, 232,297. These compounds, having formula (II), can be prepared according to the methods described therein.

Another specific group of agonists of A_{2A} adenosine receptors that are useful in the practice of the present invention include compounds having the general formula (III): 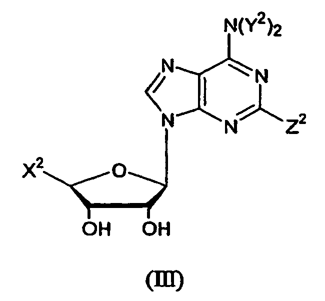 wherein Z² is a group selected from the group consisting of -OR¹², -NR¹³R¹⁴, a -C=-C-Z³, and -NH-N=R¹⁷;
each Y² is individually H, C₁-C₆ alkyl, C₃-C₇ cycloalkyl, phenyl or phenyl C₁-C₃ alkyl;
R¹² is
   a) C₁₋₄ -alkyl;
   b) C₁₋₄-alkyl substituted with one or more C₁₋₄ -alkoxy groups, halogens (fluorine, chlorine or bromine), hydroxy groups, amino groups, mono(C₁₋₄ -alkyl)amino groups, di(C₁₋₄-alkyl)amino groups or C₆₋₁₀-aryl groups wherein the aryl groups may be substituted with one or more halogens (fluorine, chlorine or bromine), C₁₋₄-alkyl groups, hydroxy groups, amino groups, mono(C₁₋₄-alkyl)amino groups or di(C₁₋₄-alkyl)amino groups); or
   c) C₆₋₁₀-aryl; or (d) C₆₋₁₀-aryl substituted with one or more halogens (fluorine, chlorine or bromine), hydroxy groups, amino groups, mono(C₁₋₄-alkyl)amino groups, di(C₁₋₄-alkyl)amino groups or C₁₋₄alkyl groups;
one of R¹³ and R¹⁴ has the same meaning as R¹² and the other is hydrogen; and
R¹⁷ is a group having the formula (i) 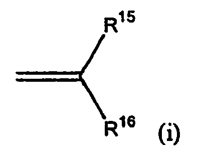 wherein each of R¹⁵ and R¹⁶ independently may be hydrogen, (C₃-C₇)cycloalkyl or any of the meanings of R¹², provided that R¹⁵ and R¹⁶ are not both hydrogen;
X² is CH₂OH, CH₃, CO₂R²⁰ or C(=O)NR²¹R²² wherein R²⁰ has the same meaning as R¹³ and wherein R²¹ and R²² have the same meanings as R¹⁵ and R¹⁶ or R²¹ and R²² are both H;
Z³ has one of the following meanings:
   a) C₆-C₁₀ aryl, optionally substituted with one to three halogen atoms, C₁-C₆ alkyl, C₁-C₆ haloalkyl, C₁-C₆ alkoxy, C₁-C₆ haloalkoxy, C₂-C₆ alkoxycarbonyl, C₂-C₆ alkoxyalkyl, C₁-C₆ alkylthio, thio, CHO, cyanomethyl, nitro, cyano, hydroxy, carboxy, C₂-C₆ acyl, amino C₁-C₃ monoalkylamino, C₂-C₆ dialkylamino, methylenedioxy or aminocarbonyl;
   b) a group of formula -(CH₂)_{q}-Het wherein q is 0 or an integer from 1 to 3 and Het is 5 or 6 membered heterocyclic aromatic or non-aromatic ring, optionally benzocondensed, containing 1 to 3 heteroatoms selected from nonperoxide oxygen, nitrogen or sulphur, linked through a carbon atom or through a nitrogen atom;
   c) C₃-C₇ cycloalkyl optionally containing unsaturation or C₂-C₄ alkenyl;
   d) 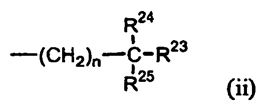 wherein
      R²³ is hydrogen, methyl or phenyl;
      R²⁴ is hydrogen, C₁-C₆ linear or branched alkyl, C₅-C₆ cycloalkyl or C₃-C₇ cycloalkenyl, phenyl-C₁-C₂-alkyl or
      R²³ and R²⁴, taken together, form a 5 or 6-membered carbocyclic ring or R²⁵ is hydrogen and R²³ and R²⁴, taken together, form an oxo group or a corresponding acetalic derivative;
      R²⁵ is OH, NH₂ dialkylamino, halogen, cyano; and n is 0 or 1 to 4; or
   e) C₁-C₁₆ alkyl, optionally comprising 1-2 double bonds, O, S or NY²;
   or a pharmaceutically acceptable salt thereof.

Specific C₆₋₁₀-aryl groups include phenyl and naphthyl.

Preferably, in the compound of formula (1), Z² is a group of the formula (iii)

-O-(CH₂)ₙ-Ar (iii)

wherein n is an integer from 1-4, preferably 2, and Ar is a phenyl group, tolyl group, naphthyl group, xylyl group or mesityl group. Most preferably Ar is a para-tolyl group and n = 2.

Preferably, in the compound of formula (II), Z² is a group of the formula (iv)

-NH-N=CHCy (iv)

wherein Cy is a C₃₋₇-cycloalkyl group, preferably cyclohexyl or a C₁₄ alkyl group, preferably isopropyl.

Preferably, in the compound of formula (II), Z² is a group of the formula (vii)

-C ≡ C-Z³ (v)

wherein Z³ is C₃-C₁₆ alkyl, hydroxy C₂-C₆ alkyl or (phenyl) (hydroxymethyl).

Specific examples of such compounds of formula (I) include WRC-0470, WRC-0474 [SHA 211], WRC-0090 and WRC-0018, shown below: 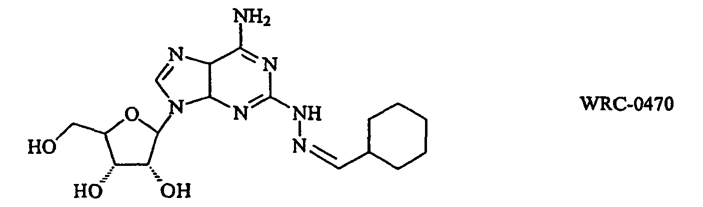 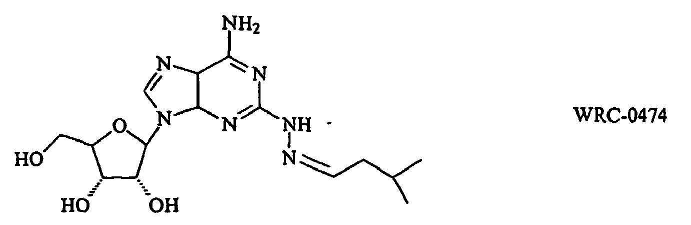 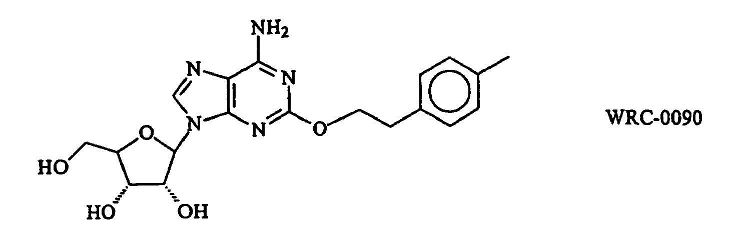 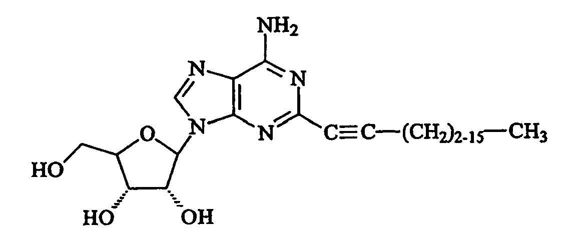 and 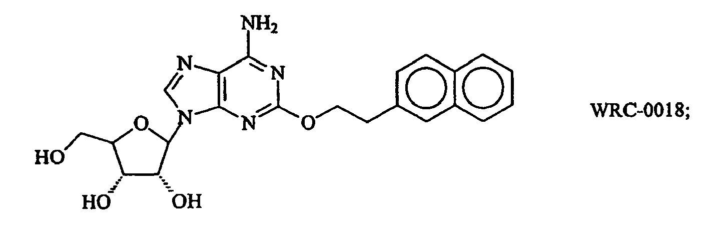 wherein the H on CH₂OH can optionally be replaced by ethylaminocarbonyl. Of these specific examples, WRC-0474[SHA 211] and WRC-0470 are particularly preferred.

Such compounds may be synthesized as described in: Olsson et al. (U.S. Pat. Nos. 5,140,015 and 5,278,150); Cristalli (U.S. Pat. No. 5,593,975); Miyasaka et al. (U.S. Pat. No. 4,956,345); Hutchinson, A. J. et al., J. Pharmacol. Exp. Ther., 251, 47 (1989); Olsson, R. A. et al., J. Med. Chem., 29, 1683 (1986); Bridges, A. J. et al., J. Med. Chem., 31, 1282 (1988); Hutchinson, A. J. et al., J. Med. Chem., 33, 1919 (1990); Ukeeda, M. et al., J. Med. Chem., 34, 1334 (1991); Francis, J. E. et al., J. Med. Chem., 34, 2570 (1991); Yoneyama, F. et al., Eur. J. Pharmacol., 213, 199-204 (1992); Peet, N. P. et al., J. Med. Chem., 35, 3263 (1992); and Cristalli, G. et al., J. Med. Chem., 35, 2363 (1992).

Another embodiment includes compounds having formula (III) where Z² is a group having formula (vi): 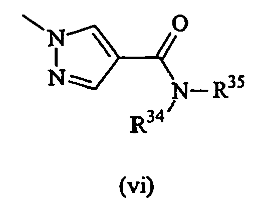 wherein R³⁴ and R³⁵ are independently H, C₁-C₆ alkyl, C₃-C₇ cycloalkyl, phenyl, phenyl C₁-C₃ alkyl or R³⁴ and R³⁵ taken together with the nitrogen atom are a 5-or 6-membered heterocyclic ring containing 1-2 heteroatoms selected from nonperoxide oxygen, nitrogen (N(R¹³)) or sulphur atoms. Preferably one of R³⁴ and R³⁵ is hydrogen and the other is ethyl, methyl or propyl. More preferably one of R³⁴ and R³⁵ is hydrogen and the other is ethyl or methyl.

The 2-(pyrazol-1-yl)adenosine compounds of the invention, wherein Z² is a group having formula (vi), can be prepared by reacting a 2-chloro- or 2-iodo adenosine derivative with an 1H-pyrazole-4-carboxamides compound having formula (vii): 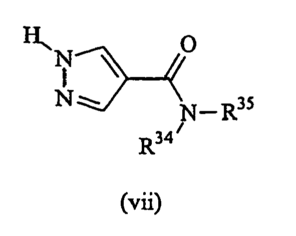 where R³⁴ and R³⁵ are as described above, wherein selective protection/deprotection of the amido group is used as needed. A preferred pyrazole derivative useful in practicing this invention is a compound having the formula :

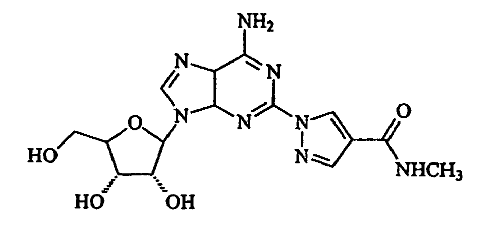

The 1H-pyrazole-4-carboxamides can be prepared starting with 1H-pyrazole-4-carboxylic acid, available fro m Aldrich Chemical Co. In the first step, the acid is converted to an ester, *e.g.*, a methyl or ethyl ester. The ester converted to the amide via aminolysis, *e.g.*, with methylamine to form the methyl amide. The pyrazole-4-carboxamide will react with the 2-halopurines in the presence of a strong base to provide the 2-(pyrazol-1-yl)adenosine compounds having formula (III).

Another specific group of agonists of A_{2A} adenosine receptors that are useful in the practice of the present invention include compounds having the general formula (IV): 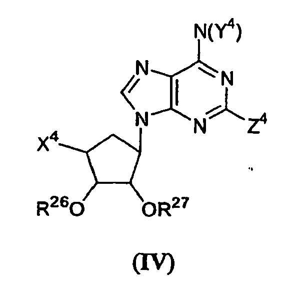 wherein
Z⁴ is -NR²⁸R²⁹;
R²⁸ is hydrogen or (C₁-C₄) alkyl; and R²⁹ is
   a) (C₁-C₄) alkyl;
   b) (C₁-C₄) alkyl substituted with one or more (C₁-C₄) alkoxy, halogen, hydroxy, amino, mono((C₁-C₄) alkyl)amino, di((C₁-C₄) alkyl)amino or (C₆-C₁₀) aryl wherein aryl is optionally substituted with one or more halogen, hydroxy, amino, (C₁-C₄)alkyl, R³⁰OOC-((C₁-C₄)alkyl)-, R³¹R³²NC(=O)-((C₁-C₄)alkyl)-, mono((C₁-C₄)alkyl)amino or di((C₁-C₄)alkyl)amino;
   c) (C₆-C₁₀)aryl; or
   d) (C₆-C₁₀)aryl substituted with one or more halogen, hydroxy, amino, mono((C₁-C₄)alkyl)amino, di((C₁-C₄)alkyl)amino or (C₁-C₄)alkyl;
   wherein each Y⁴ is individually H, (C₁-C₆)alkyl, (C₃-C₇)cycloalkyl, phenyl or phenyl(C₁-C₃)alkyl; and X⁴ is -C(=O)NR³¹R³², -COOR³⁰, or -CH₂OR³⁰;
   wherein each of R³¹ and R³² are independently; hydrogen; C₃₋₇-cycloalkyl; (C₁-C₄)alkyl; (C₁-C₄)alkyl substituted with one or more (C₁-C₄)alkoxy, halogen, hydroxy, -COOR³³, amino, mono((C₁-C₄)alkyl)amino, di((C₁-C₄)alkyl)amino or (C₆-C₁₀)aryl wherein aryl is optionally substituted with one or more halogen, (C₁-C₄)alkyl, hydroxy, amino, mono((C₁-C₄) alkyl)amino or di((C₁-C₄) alkyl)amino; (C₆-C₁₀)aryl; or (C₆-C₁₀)aryl substituted with one or more halogen, hydroxy, amino, mono((C₁-C₄)alkyl)amino, di((C₁-C₄)alkyl)amino or (C₁-C₄)alkyl;
R²⁶ and R²⁷ independently represent hydrogen, lower alkanoyl, lower alkoxy-lower alkanoyl, aroyl, carbamoyl or mono- or di-lower alkylcarbamoyl; and R³⁰ and R³³ are independently hydrogen, (C₁-C₄)alkyl, (C₆-C₁₀)aryl or (C₆-C₁₀)aryl((C₁-C₄)alkyl); or a pharmaceutically acceptable salt thereof.

In one embodiment of formula (IV), at least one of R²⁸ and R²⁹ is (C₁-C₄)alkyl substituted with one or more (C₁-C₄)alkoxy, halogen, hydroxy, amino, mono((C₁-C₄)alkyl)amino, di((C₁-C₄)alkyl)amino or (C₆-C₁₀)aryl wherein aryl is optionally substituted with one or more halogen, hydroxy, amino, (C₁-C₄)alkyl, R³⁰OOC-(C₁-C₄)alkyl, mono((C₁-C₄)alkyl)amino or di((C₁-C₄)alkyl)amino.

In another embodiment, at least one of R³¹ and R³² is C₁₋₄-alkyl substituted with one or more (C₁-C₄)alkoxy, halogen, hydroxy, amino, mono((C₁-C₄)alkyl)amino, di((C₁-C₄)alkyl)amino or C₆₋₁₀-aryl wherein aryl is optionally substituted with one or more halogen, hydroxy, amino, (C₁-C₄)alkyl, R³⁰OOC-(C₁-C₄)alkylene-, mono((C₁-C₄)alkyl)amino or di((C₁-C₄)alkyl)amino.

In another embodiment, at least one of R²⁸ and R²⁹ is C₆₋₁₀-aryl substituted with one or more halogen, hydroxy, amino, mono((C₁-C₄)alkyl)amino, di((C₁-C₄)alkyl)amino or (C₁-C₄)alkyl.

In another embodiment, at least one of R³¹ and R³² is C₆₋₁₀-aryl substituted with one or more halogen, hydroxy, amino, mono((C₁-C₄)alkyl)-amino, di((C₁-C₄)alkyl)amino or (C₁-C₄)alkyl.

In a preferred combination, R³¹ is hydrogen and R³² is (C₁-C₄)alkyl, cyclopropyl or hydroxy-(C₂-C₄)alkyl. A preferred R²⁸ group is (C₁-C₄)alkyl substituted with (C₆-C₁₀)aryl, that is in turn substituted with R³⁰O(O)C-(C₁-C₄)alkyline-.

A preferred compound having formula (IV) is: 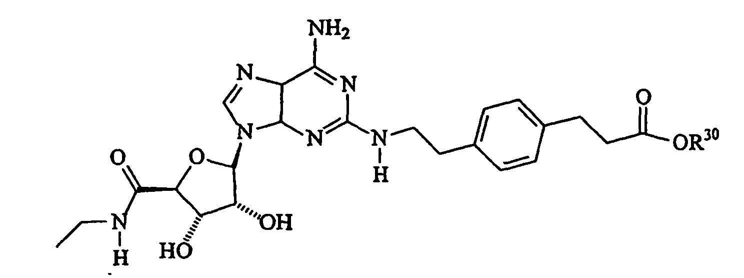 wherein R³⁰ is hydrogen, methyl, ethyl, n-propyl or isopropyl. More preferred is a compound wherein the R³⁰ group is methyl or ethyl. The most preferred R³⁰ group is methyl.

Two compounds that are particularly useful in practicing the present invention have the formula: 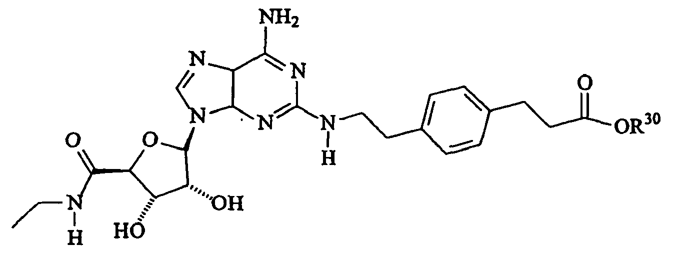 wherein R³⁰ is hydrogen (acid, CGS21680) and where R³⁰ is methyl (ester, JR2171).

The compounds of the invention having formula (IV) may be synthesized as described in: U.S. Patent 4,968,697 or J. Med. Chem., 33, 1919-1924,(1990)

Specifically, the invention also provides the use of a compound of formula (I) or a pharmaceutically acceptable salt thereof to prepare a medicament for treating systemic intoxification in a mammal (e.g. a human),.

Specifically, the invention also provides the use of a compound of formula (I) or a pharmaceutically acceptable salt thereof to prepare a medicament for treating inflammation caused by bacterial, fungal or viral infections and the inflammation caused by the treatment of these infections, e.g., by the death of the bacterial or viral cells in a mammal (e.g. a human).

The present method also includes the administration of a Type IV phosphodiesterase (PDE) inhibitor in combination with compounds having formulae (I), (II), (III), and (IV). The combination of the compounds of the invention with type IV phosphodiesterase inhibitor provides synergistic decreases in the inflammatory response of immune cells. Examples of Type IV phosphodiesterase (PDE) inhibitors include those disclosed in U.S. Patent No. 4,193,926, and WO 92-079778, and Molnar-Kimber, K. L. et al., J. Immunol., 150, 295A (1993).

Suitable Type IV phosphodiesterase (PDE) inhibitors include racemic and optically active 4-(polyalkoxyphenyl)-2-pyrrolidones of general formula (VI): 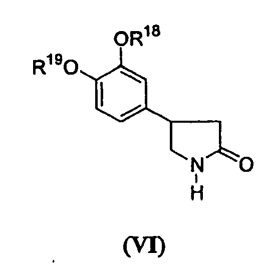 (disclosed and described in U.S. Patent No. 4,193,926) wherein R¹⁸ and R¹⁹ are independently the same or different and are hydrocarbon radicals having up to 18 carbon atoms with at least one being other than methyl, a heterocyclic ring, or alkyl of 1-5 carbon atoms which is substituted by one or more of halogen atoms, hydroxy, carboxy, alkoxy, alkoxycarbonyl or an amino group or amino.

Examples of hydrocarbon R¹⁸ and R¹⁹ groups are saturated and unsaturated, straight-chain and branched alkyl of 1-18, preferably 1-5, carbon atoms, cycloalkyl and cycloalkylalkyl, preferably 3-7 carbon atoms, and aryl and aralkyl, preferably of 6-10 carbon atoms, especially monocyclic.

Rolipram is an example of a suitable Type IV phosphodiesterase or PDE inhibitor included within the above formula. Rolipram has the following formula:

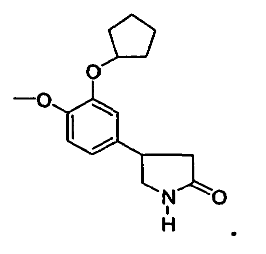

In cases where compounds are sufficiently basic or acidic to form stable nontoxic acid or base salts, administration of the compounds as salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, α-ketoglutarate, and α-glycerophosphate. Suitable inorganic salts may also be formed, including hydrochloride, sulfate, nitrate, bicarbonate, and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

Compounds of the present invention can conveniently be administered in a pharmaceutical composition containing the compound in combination with a suitable excipient. Such pharmaceutical compositions can be prepared by methods and contain excipients which are well known in the art. A generally recognized compendium of such methods and ingredients is Remington's Pharmaceutical Sciences by E.W. Martin (Mark Publ. Co., 15th Ed., 1975). The compounds and compositions of the present invention can be administered parenterally (for example, by intravenous, intraperitoneal or intramuscular injection), topically, orally, or rectally.

For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The compounds or compositions can also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

Pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions can be prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compounds may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, alcohols or glycols or water-alcohol/glycol blends, in which the present compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Useful dosages of the compounds of formula I can be determined by comparing their *in vitro* activity, and *in vivo* activity in animal models. Methods for the extrapolation of effective dosages in mice, and other animals, to humans are known to the art; for example, see U.S. Pat. No. 4,938,949.

The compound is conveniently administered in unit dosage form; for example, containing about 0.05 mg to about 500 mg, conveniently about 0.1 mg to about 250 mg, most conveniently, about 1 mg to about 150 mg of active ingredient per unit dosage form. The desired dose may conveniently be presented in a single dose or as divided doses administered at appropriate intervals, for example, as two, three, four or more sub-doses per day. The sub-dose itself may be further divided, e.g., into a number of discrete loosely spaced administrations.

The compositions can conveniently be administered orally, sublingually, transdermally, or parenterally at dose levels of about 0.01 to about 150 µg/kg, preferably about 0.1 to about 50 µg/kg, and more preferably about 0.1 to about 10 µg/kg of mammal body weight.

For parenteral administration the compounds are presented in aqueous solution in a concentration of from about 0.1 to about 10%, more preferably about 0.1 to about 7%. The solution may contain other ingredients, such as emulsifiers, antioxidants or buffers.

The exact regimen for administration of the compounds and compositions disclosed herein will necessarily be dependent upon the needs of the individual subject being treated, the type of treatment and, of course, the judgment of the attending practitioner.

The preparation of compounds useful in practicing the present invention are disclosed in U.S. Patent Application Serial No. 10/236,379, filed October 1, 2002, and can generally be prepared as illustrated in Schemes 1A and 1B below. Starting materials can be prepared by procedures described in these schemes, procedures described in the General methods below or by procedures that would be well known to one of ordinary skill in organic chemistry. The variables used in Schemes 1A and Scheme 1B are as defined herein or as in the claims.

The preparation of alkynyl cycloalkanols is illustrated in Scheme 1A. A solution of an appropriate cycloalkanone (where j is from 0-5) is prepared in a solvent such as THF. A solution of a suitable ethynylmagnesium halide compound in a solvent is added to the cycloalkanone. After addition, the solution is allowed to stir at about 20 C for about 20 hours. The reaction is monitored via TLC until the starting material is consumed. The reaction is quenched with water, filtered over a plug of sand and silica, washed with a solvent, such as EtOAc, and evaporated to provide the product. Typically, two products are formed, the isomers formed by the axial/equatorial addition of the alkyne (where m is as defined above, and the sum of m1 and m2 is from 0 to about 7) to the ketone. The compounds are purified via flash chromatography using EtOAc/Hexanes to provide the product.

In accordance with one embodiment of the present invention a composition comprising an agonist of A_{2A}AR is administered to a patient to treat septic shock and systemic inflammatory response syndrome. As used herein the term "treating" includes prophylaxis of the specific disorder or condition, or alleviation of the symptoms associated with a specific disorder or condition and/or preventing or eliminating said symptoms. In one embodiment a method for treating septic shock or systemic inflammatory response syndrome is provided wherein an agonist of A_{2A}ARs is administered to a patient to reduce inflammation and improve survival in a patient suffering from septic shock or systemic inflammatory response syndrome. In one embodiment the A_{2A}AR agonist is selected from the group consisting of ATL146e, AB-1, AB-3 and JR-3213.

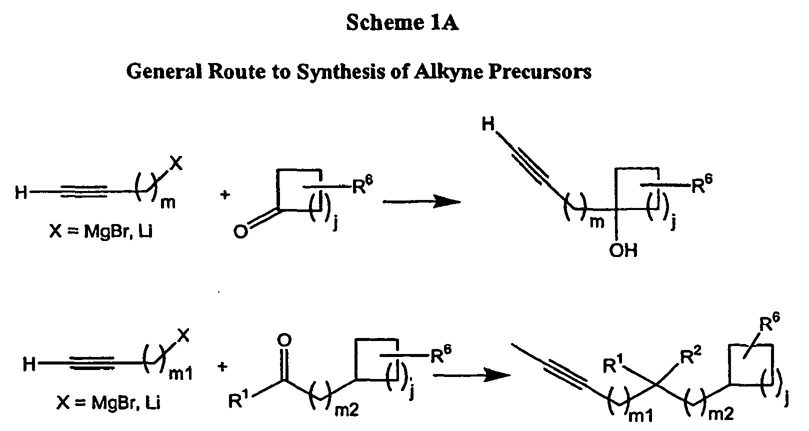

The preparation of 2-alkynyladenosines is illustrated in Scheme 1B. A flame-dried round bottom under nitrogen is charged with 5-(6-Amino-2-iodo-purin-9-yl)-3,4-dihydroxy-tetrahydro-furan-2-carboxylic acid ethylamide (NECA 2-Iodoadenosine) and a solvent such as DMF. The appropriate alkyne is added followed by acetonitrile and TEA. (The solvents are degassed.) The appropriate alkyne is added in acetonitrile, followed by TEA, 5 mole % Pd(PPh₃)₄, and CuI. The solution is allowed to stir for about 24 hours at room temperature, and monitored until complete by HPLC. If the reaction is not complete after this time, additional catalyst, CuI, and TEA are added. After the reaction is complete, the solvents are removed under high-vacuum and the residue taken up in a small amount of DMF. This product is isolated using preparative silica TLC. The product is purified by RP-HPLC.

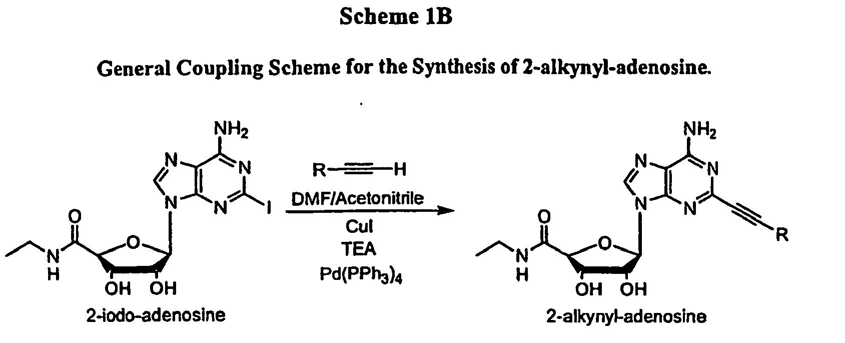

The following abbreviations have been used herein:

| | |
|---|---|
| 2-Aas | 2-alkynyladenosines; |
| ¹²⁵I-ABA | N⁶-(4-amino-3-¹²⁵iodo-benzyl)adenosine |
| APCI | Atmospheric pressure chemical ionization |
| ATL146e | 4- {3-[6-Amino-9-(5-ethylcarbamoyl-3,4-dihydroxytetrahydro-furan-2-yl)-9H-purin-2-yl]-prop-2-ynyl} cyclohexanecarboxylic acid methyl ester; |
| CCPA | 2-chloro-*N*⁶-cyclopentyladenosine; |
| CGS21680 | 2-[4-(2-carboxyethyl)phenethylamino]-5'*-N*-ethyl-carboxamidoadenosine; |
| C1-IB-MECA | *N*⁶-3-iodo-2-chlorobenzyladenosine-5'-*N*-methylur onamide; |
| CPA | *N*⁶-cyclopentyladenosine |
| DMF | dimethylformamide |
| DMSO | dimethylsulfoxide |
| DMSO-d₆ | deuterated dimethylsulfoxide |
| EtOAc | ethyl acetate |
| eq | equivalent |
| GPCR | G protein coupled receptor; hA_{2A}AR, Recombinant human A_{2A} adenosine receptor; |
| IADO | 2-Iodoadenosine |
| ¹²⁵I-APE, | 2-[2-(4-amino-3-[¹²⁵I]iodophenyl)ethylamino]-adenosine; |
| NECA | 5'-*N*-ethylcarboxamidoadenosine; |
| IB-MECA | *N⁶-*3-iodobenzyladenosine-5'-*N*-methyluronamide; |
| 2-Iodoadenosine | 5-(6-amino-2-iodo-purin-9-yl)-3,4-dihydroxytetra-hydro-furan-2carboxylic acid ethylamide |
| HPLC | high-performance liquid chromatography |
| HRMS | high-resolution mass spectrometry |
| ¹²⁵I-ZM241385, | ¹²⁵I-4-(2-[7-amino-2-[2-furyl][1,2,4]triazolo[2,3-*a*]-[1,3,5]triazin-5-yl-amino]ethyl)phenol; |
| INECA | 2-iodo-N-ethylcarboxamidoadenosine |
| LC/MS | liquid chromatography/mass spectrometry |
| m.p. | melting point |
| MHz | megahertz |
| MRS 1220, | N-(9-chloro-2-furan-2-yl-[1,2,4]triazolo[1,5-c]-quinazolin-5-yl)-2-phenylacetamide; |
| MS | mass spectrometry |
| NECA | N-ethylcarboxamidoadenosine |
| NMR | nuclear magnetic resonance |
| RP-HPLC | reverse phase high-performance liquid chromatography |
| TBAF | tetrabutylammonium fluoride |
| TBS | tert-butyldimethylsilyl |
| TBDMSCI | tert-butyldimethylsilylchloride |
| TEA | triethylamine |
| TFA | trifluoroacetic acid |
| THF | tetrahydrofuan |
| TLC | thin layer chromatography |
| p-TSOH | para-toluenesulfonic acid |
| XAC | 8-(4-((2-a-minoethyl)aminocarbonyl-methyloxy)-phenyl)-1-3-dipropylxanthine. |

### Syntheses of compounds useful in practicing the invention.

All melting points were determined with a Thomas Hoover capillary melting point apparatus and are uncorrected. Nuclear magnetic resonance spectra for proton (¹H NMR) were recorded on a 300 MHz GE spectrophotometer. The chemical shift values are expressed in ppm (parts per million) relative to tetramethylsilane. For data reporting, s = singlet, d = doublet, t = triplet, q = quartet, and m = multiplet. Mass spectra were measured on a Finnigan LcQ Classic. High resolution mass spectrometry (HRMS) data was provided by the Nebraska Center for Mass Spectrometry. Analytical HPLC was done on a Waters 2690 Separation Module with a Waters Symmetry C8 (2.1 x 150 mm) column operated at room temperature. Compounds were eluted at 200 µL/min with 70:30 acetonitrile:water, containing 0.5% acetic acid, with UV detection at 214 nm using a Waters 486 Tunable Detector. Preparative HPLC was performed on a Shimadzu Discovery HPLC with a Shim-pack VP-ODS C₁₈ (20 x 100 mm) column operated at room temperature. Compounds were eluted at 30mL/min with a gradient 20-80% of water (containing 0.1% TFA) to methanol over 15 minutes with UV detection at 214 nm using a SPD10A VP Tunable detector. All final compounds presented here were determined to be greater than 98% pure by HPLC. Flash chromatography was performed on Silicyle 60A gel (230-400 mesh) or using reusable chromatography columns and system from RT Scientific, Manchester NH. Analytical thin-layer chromatography was done on Merck Kieselgel 60 F254 aluminum sheets. Preparative thin-layer chromatography was done using 1000 micron Analtech Uniplate with silica gel. All reactions were done under a nitrogen atmosphere in flame-dried glassware unless otherwise stated.

### General method 1: Preparation of alkynyl cyclohexanols

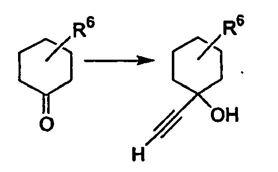

To a solution of about 10 mmol of the appropriate cyclohexanone in about 50 mL of THF is added to about 60 mL (30 mmol) of 0.5 M ethynylmagnesium bromide in THF. The solution is allowed to stir at about 20°C for about 20 hours. After the starting material had been consumed, monitored by TLC, the reaction is quenched with about 5 mL of water, filtered over a plug of sand and silica, washed with EtOAc, and evaporated to yield a yellow oil. Usually the oil contained two spots on TLC with 20% EtOAc/Hexanes, which are visualized with Vanillin. Usually these two products are the different isomers formed by the axial/equatorial addition of the alkyne to the ketone. The compounds are purified via flash chromatography using 10% EtOAc/Hexanes to provide clear oils or white solids in a yield of about 50-80 %.

### General method 2: Preparation of propargyl piperadines/piperazines.

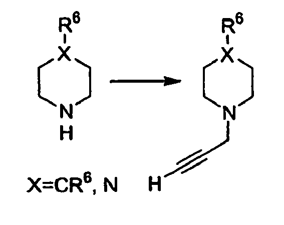

To a solution of of the appropriate piperazine/piperadine(about 10.0 mmol), in about 20 mL acetonitrile, is added about 12.0 mmol of propargyl bromide (80% stabilized in toluene) and about 50.0 mmol of anhydrous potassium carbonate. The reaction mixture is filtered, and evaporated to dryness. The resiude is taken up in about 50 mL of dichloromethane/water and the organic layers removed. The aqueous layer is washed with an additional 3 x 25 mL dichloromethane. The organic layer is dried using anhydrous sodium sulfate, filtered, and concentrated to provide the crude product, which is purified using column chromatography.

### General method 3: Preparation of modified piperadines/piperazines.

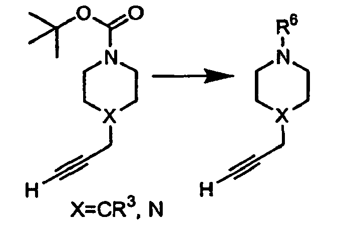

To about 100 mg of the appropriate Boc-protected piperazine/piperadine is added 2-4 mL of neat TFA. The solution is allowed to stir for 6 hours. The TFA is removed under reduced pressure to yield a yellow oil. This oil is taken up in about 10 mL of dichloromethane to which is added 10-fold excess of TEA and 3 equivalents of the appropriate acyl chloride. The yellow solution is allowed to stir at room temperature for about 12 hours, after which time the solvents are removed and the product purified using a 1.1x30 cm 14 g column from Robert Thompson Scientific with a 5%-30% gradient of ethyl acetate/hexanes.

### General method 4: Preparation of 2-AAs (2-alkynyladenosines).

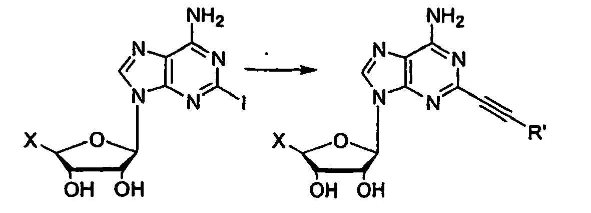

A flame-dried 25 mL round bottom under nitrogen is charged with 5-(6-amino-2-iodo-purin-9-yl)-3,4-dihydroxytetrahydrofuran-2-carboxylic acid ethylamide (2-Iodoadenosine) (about 40 mg) (X = CH₃CH₂NHC(O)-) and dissolved in about 2 mL of DMF. The appropriate alkyne (approx. 0.1mL) is then added followed by about 4mL of acetonitrile and about 0.1mL of TEA. All three solvents had been degassed with nitrogen for at least 24 hours. To this solution is added 5 mole percent Pd(PPh₃)₄ and 6 mole % copper iodide. The yellowish solution is allowed to stir for 24 hours at room temperature, or until complete by HPLC. If the reaction is not complete at this time, additional catalyst, CuI, and TEA are added. After the reaction is complete, the solvents are removed under high-vacuum and the red/black residue taken back up in a small amount of DMF. This solution is added to a preparative silica TLC plate (Analtech 1000 microns, 20cm x 20cm) and eluted first with 120 mL of 40% Hexanes/CH₂Cl₂, and then again after addition of 40 mL of MeOH. The UV active band (usually yellow in color) in the middle of the plate is collected, slowly washed with 4 x 25 mL 20% MeOH/CH₂Cl₂, and concentrated. This product is then purified by RP-HPLC.

### Preparation 1: [(2R,3R,4R,5R)-3,4-diacetyloxy-5-(2-amino-6-oxohyropurin-9-yl)oxolan-2-yl]methyl acetate (6.2).

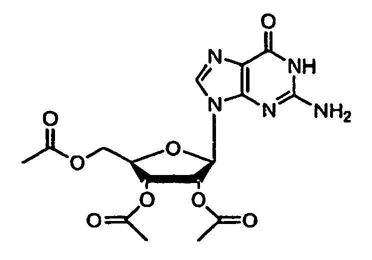

A suspension of 113 g (0.4 mol) of dry guanosine (**6.1**), acetic anhydride (240 mL, 2.5 mol), dry pyridine (120 mL) and dry DMF (320 mL) was heated for 3.75 hours at 75 °C without allowing the temperature to exceed 80 °C. The clear solution was then transferred to a 3L Erlenmyer flask and filled with 2-propanol. Upon cooling the solution to room temperature crystallization was initiated and allowed to proceed at 4 °C overnight. The white solid filtrate was filtered, washed with 2-propanol and recrystallized from 2-propanol to provide 6.2 (96%). ¹H NMR (300 Mhz, CDCl₃) 8.20 (s, 1H, H-8), 6.17 (d, *J*= 5.41 Hz, 1 H, H-1) 5.75 (t, *J=* 5.39 Hz, 1H, H-2), 5.56 (t, *J=* 5.0, H-3), 4.41 (m, 3H, H-4,5), 2.14 (s, 3H, Ac), 2.11 (s, 3H, Ac), 2.10 (s, 3H, Ac). ¹³C NMR (300 MHz, CD₃OD) 171.0, 170.3, 1702, 157.7, 154.8, 152.4, 136.7, 117.7, 85.5, 80.4, 73.0, 71.3, 64.0, 31.3, 21.2, 21.0.

### Preparation 2: [(2R,3R,4R,5R)-3,4-diacetyloxy-5-(2-amino-6-chloropurin-9-yl)oxolan-2-yl]methyl acetate (6.3).

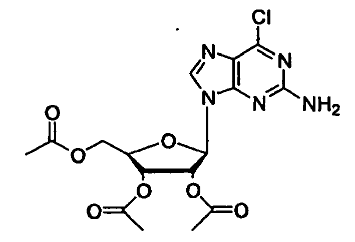

To a 1L flask was added 80 g (0.195 mol) [(2R,3R,4R,5R)-3-4-diacetyloxy-5-(2-amino-6-oxohyropurin-9-yl)oxolan-2-yl]m ethyl acetate (6.2), tetramethylammonium chloride (44 g, 0.4 mol), anhydrous acetonitrile (400 mL) and N,N-dimethlaniline (25 mL). The flask was placed in an ice salt bath and cooled to 2°C. To this solution was added dropwise POCl₃ (107 mL 1.15 mol) at a rate that maintained the temperature below 5°C (45 minutes). The flask was then removed from the ice bath, outfitted with a condenser, placed in an oil bath and allowed to reflux for 10 minutes. The solution changed to a red/brown color. The solvent was removed under reduced pressure to yield an oily residue which was transferred to a beaker containing 1000 g of ice and 400 mL of CHCl₃ and allowed to stir for 1.5 hours to decompose any remaining POCl₃. The organic phase was removed and the aqueous phase extracted with 3 × 50 mL of CHCl₃ and pooled with the organic phase. The pooled organic layeres were back extracted with 50 mL of water followed by stirring with 200 mL of saturated NaHCO₃. The organic layer was further extracted with NaHCO₃ until the aqueous extract was neutral (2X). The organic layer was finally extracted with brine and dried over MgSO₄ for 16 hours. To the solution was added 800 mL of 2-propanol after which the solution was concentrated under reduced pressure. To the oily solid was added 200 mL of 2-propanol and the solution was refrigerated overnight. The crystalline product was filtered, washed, and allowed to dry overnight to give 6.3 (77%). ¹H NMR (300 MHz, CD₃OD) 8.31 (s, 1H, H-8), 7.00 (s, 2H, NH₂) 6.06 (d, *J* = 5.8 Hz, 1H, H-1), 5.83 (t, *J* = 6.16 Hz,1H, H-2), 5.67 (m, 1H, H-3), 4.29 (m, 3H, H-4,5), 2.07 (s, 3H, Ac), 1.99 (s, 3H, Ac), 1.98 (s, 3H, Ac). ¹³C NMR (300 MHz, CD₃OD) 171.0, 170.4, 170.2, 160.8, 154.6, 150.8, 142.2, 124.5, 85.8, 80.6, 72.8, 71.2, 63.9, 21.4, 21.3, 21.1.

### Preparation 3: [(2R,3R,4R,5R)-3,4-diacetyloxy-5-(6-chloro-2-iodopurin-9-yl)oxolan-2-yl]methyl acetate (6.4).

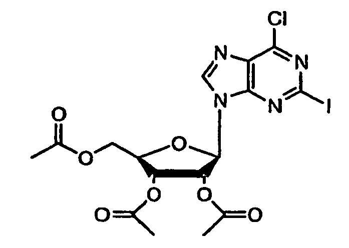

Isoamyl nitrite (5 mL, 37 mmol) was added to a mixture of 5.12 g (12 mmol) [(2R,3R,4R,5R)3-,4-diacetyloxy-5-(2-amino-6-chloropurin-9-yl)oxolan-2-yl]methyl acetate (**6.3**), I₂ (3.04 g, 12 mmol), CH₂I₂ (10 mL, 124 mmol), and CuI (2.4 g, 12.6 mmol) in THF (60 mL). The mixture was heated under reflux for 45 minutes and then allowed to cool to room temperature. To this solution was added 100 ml of saturated Na₂S₂O₃. This step removed the reddish color. The aqueous layer was extracted 3X with chloroform, which was pooled, dried over MgSO₄, and concentrated under reduced pressure. The product was then purified over a silica gel column using CHCl₃-MeOH (98:2) to collect [(2R,3R,4R,SR)-3,4-diacelyloxy-5-(6-chloro-2-iodopurin-9-yl)oxolan-2-yl]meth yl acetate (**6.4**) (80% crystallized from EtOH). ¹H NMR (300 MHz, CDCl₃) 8.20 (s, 1H H-8), 6.17 (d, *J* = 5.41 Hz, 1H, H-1), 5.75 (t, *J*= 5.39 Hz, 1H, H-2 ), 5.56 (t, *J* = 5.40 Hz, 1H, H-3), 4.3 8 (m, 3H, H-4, 5), 2.14 (s, 1H, Ac), 2.11 (s, 1H, Ac), 2.10 (s, 1H, Ac).

### Preparation 4: (4S,2R,3R,5R)-2-(6-amino-2-iodopurin-9-yl)-5-(hydroxymethyl)oxolane-3,4-diol (6.5).

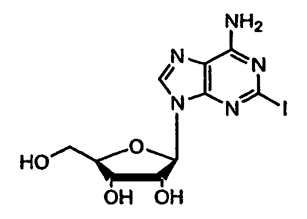

To a flask containing 6.0 g (11.1 mmol) [(2R,3R,4R,5R)-3,4-diacetyloxy-5-(6-chloro-2-iodopurin-9-yl)oxolan-2-yl]meth yl acetate (**6.4**) was added 100 ml of liquid NH₃ at -78°C and the solution was allowed to stir for 6 hours. After which time it was allowed to come to room temperature overnight with concurrent evaporation of the NH₃ to yield a brown oil. The product was crystallized from hot isopropanol to provide 6.5 (80%), m.p. 143-145°C, r.f. = 0.6 in 20% MeOH/CHCl₃. ¹H NMR (300 MHz, DMSO-d₆) 8.24 (s, 1H), 7.68 (s, 2H), 5.75 (d, *J=* 6.16, 1H), 5.42 (d, *J*= 5.40 Hz, 1H), 5.16 (d, *J*= 4.62 Hz, 1H), 4.99 (t, *J=* 5.39 Hz, 1H), 4.67 (d, *J*= 4.81 Hz, 1H), 4.06 (d, *J=* 3.37 Hz, 1H), 3.89 (m, 1H), 3.54 (m, 2H).

### Preparation 5: [(1R,2R,4R,5R)-4-(6-amino-2-iodopurin-9-yl)-7-7-dimethyl-3,6,8-trioxabicyclo[3.3.0]oct-2-yl]methan-1-ol (6.6).

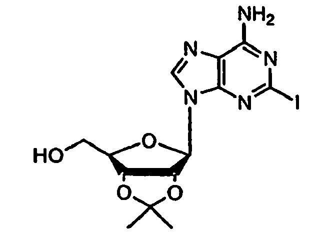

To a solution of 2.0 g (5.08 mmol) (4S,2R,3R,5R)-2-(6-amino-2-iodopurin-9-yl)-5(hydroxymethyl)oxolane-3,4-diol (**6.6**) in 100 mL acetone was added 9.6 g of p-toluenesulfonic acid and 5 ml of dimethoxypropane. The reaction was stirred at room temperature for 1 hour. Solid NaHCO₃, 15 g, was added to the solution. The slurry was stirred for an additional 3 hours. The residue was filtered and washed 2X with EtOAc. The filtrate was then concentrated under reduced pressure. The residue was chromatographed on a silica gel column with MeOH-CHCl₃ (1:99) to give 6.6 (72%) as a solid, m.p. 185-187°C. ¹H NMR (300 MHz, DMSO-d₆) 8.22 (s, 1H, H-8), 7.69 (s, 2H), NH₂), 6.00 (d, *J* = 2.70 Hz, 1H, H-1), 5.21 (m, 1H, H-2), 5.07 (bs, 1H, OH), 4.88 (m, 1H, H-3), 4.13 (m, 1H, H-4), 3.47 (m, 2H, H-5), 1.49 and 1.28 (s, 3H, C(CH₃)₂).

### Preparation 6: (2S,1R,4R,5R)-4-(6-amino-2-iodopurin-9-yl)-7,7-dimethyl-3,6,8-tnoxabicyclo[3.3.0]octane-2-carboxylic acid (6.7).

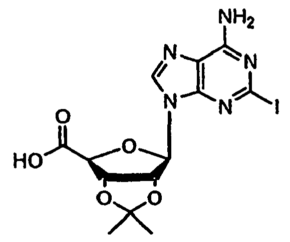

To a stirred solution of 1.6 g (3.7 mmol) of [(1R,2R,4R,5R)-4-(6-amino-2-iodopurin-9-yl)-7-7-dimethyl-3,6,8-trioxabicyclo[ 3.3.0]oct-2-yl]methan-1-ol (**6.6**) in 200 mL of H₂O was added 0.60 g of KOH and, dropwise, a solution of 1.70 g (10.8 mml) of KMnO₄ in 50 mL of H₂O. The mixture was placed in the dark at room temperature for 2-4 days. The reaction mixture was then cooled to 5-10 °C and decolorized by a solution of 4 mL of 30% H₂O₂ in 16 mL of water, while the temperature was maintained below 10 °C using an ice-salt bath. The mixture was filtered through Celite and the filtrate was concentrated under reduced pressure to about 10 mL and then acidified to pH 4 with 2N HCl. The resulting precipitate was filtered off and washed with ether to yield 6.7 (70%) after drying as a white solid, m.p. 187-190 C. ¹H NMR (300 MHz, DMSO-d₆) 8.11 (s, 1H, H-8), 7.62 (s, 2H, NH₂), 7.46 (s, 1H, COOH), 6.22 (s, 1H, H-1), 5.42 (d, *J=* 5.71 Hz, 1H, H-2), 5.34 (d, *J*= 6.16 Hz, 1H, H-3), 4.63 (s, 1H, H-4), 1.46 and 1.30 (s, 3H, C(CH₃)₂).

### Preparation 7: (2S,3S,4R,5R)-5-(6-amino-2-iodopurin-9-yl)-3,4-dihydroxyoxolane-2-carboxylic acid (6.8).

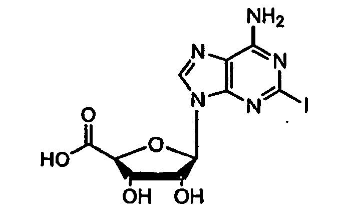

A solution of 1.72 g (3.85 mmol) of (2S,1R,4R,5R)-4-(6-amino-2-iodopurin-9-yl)-7,7-dimethyl-3,6,8-trioxabicyclo[3 .3.0]octane-2-carboxylic acid (**6.7**) in 80 mL of 50% HCOOH was stirred at 80 °C for 1.5 hours. The reaction mixture was evaporated under reduced pressure, dissolved in H₂O, and the solvent was evaporated again. This process was repeated until there was no odor of formic acid in the residue. Recrystallization from water provided 1.33 g (85%) **6.8** as a white solid, m.p. 221-223 °C, dec. ¹H NMR (300 MHz, DMSO-d₆) 8.31 (s, 1H, H-8), 7.68 (s, 2H, NH₂), 5.90 (d, *J=* 6.55 Hz, 1H, H-1), 4.42 (m, 1H, H-2), 4.35 (d, *J*= 2.31 Hz, 1H, H-4), 4.22 (m, 1H, H-3).

### Preparation 8: [(2S,3S,4R,5R)-5-(6-amino-2-iodopurin-9-yl)-3,4-dihydroxyoxolan-2-yl]-N-ethylcarboxamide (6.9).

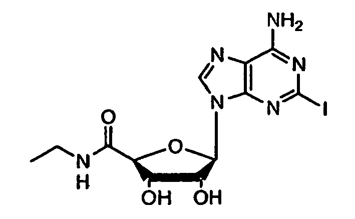

To a cooled (5 °C) and stirred solution of 1.29 g (3.17 mmol) of (2S,3S,4R,5R)-5-(6-amino-2-iodopurin-9-yl)-3,4-dihydroxyoxolane-2-carboxylic acid (**6.8**) in 150 mL of absolute ethanol was added dropwise 1.15 mL of ice-cooled SOCl₂. The mixture was stirred at room temperature overnight and then brought to pH 8 with saturated aqueous NaHCO₃. The mixture was filtered, and then the filtrate was concentrated under reduced pressure to yield a white solid which was dried and then redissolved in 20 mL of dry ethylamine at -20 °C for 3 hours and then at room temperature overnight. The reaction mixture was diluted with absolute ethanol, and the precipitated product was filtered off and washed with dry ether to provide 530 mg (72%) of **6.9** as a pure solid, m.p. 232-234°C. ¹H NMR (300 MHz, DMSO-d₆) 8.34 (s, 1H, H-8), 8.12 (t, 1H, NH), 7.73 (s, 2H, NH₂), 5.85, (d, *J*= 6.93 Hz, 1H, H-1), 4.54 (m, 1H, H-2), 4.25 (d, *J* = 1.92 Hz, 1H, H-4), 4.13 (m, 1H, H-3), 3.28 (m, 2H, CH₂CH₃), 1.00 (t, *J*= 7.2 Hz, 3H, CH₂CH₃).

### Preparation 9:

### [4-(tert-Butyl-dimethyl-silanyloxymethyl)-cyclohexyl]-methanol (83).

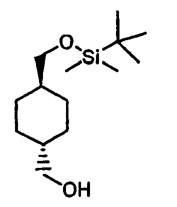

To a 100 mL-flask containing **79** (4.0 g, 27.8 mmol) in DMF (40 mL) was added TBDMSCI (3.56 g, 23.6 mmol) and imidazole (3.79 g, 55.6 mmol). The reaction was allowed to stir at 25 °C for 16hoursafter which time saturated aqueous LiBr (50 mL) was added and the reaction extracted with ether (2 x 50 mL). The ether layers were pooled and extracted again with LiBr (2 x 35 mL). The ether layer became clear. The ether layer was then concentrated *in vacuo* and the product purified by flash chromatography, on a silica gel column, eluting with 1:2 ether/petroleum ether to yield 83 (3.80 g, 62%) as a homogenous oil. ¹H NMR (CDCl₃) δ 3.46 (d, J = 6.2 Hz, 2 H), 3.39 (d, J = 6.2 Hz, 2 H), 1.95-1.72 (m, 4 H), 1.65 (m, 1H), 1.40 (m, 1 H), 1.03 - 0.89 (m, 4 H), 0.88 (s, 9 H), 0.04 (s, 6 H); ¹³C NMR (CDCl₃) δ 69.2, 69.1, 41.2, 41.1, 29.5, 26.5, 18.9, -4.8;. APCI *m*/*z* (rel intensity) 259 (MH⁺, 100).

### Preparation 10: Toluene-4-sulfonic acid 4-(tert-butyl-dimethyl-silanyloxymethyl)-cyclohexylmethyl ester (84).

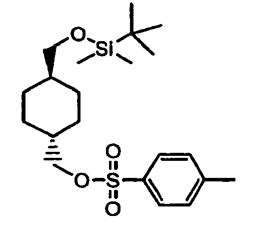

To a 100 mL-flask containing **83** (3.4 g, 13.2 mmol) in CHCl₃ (30 mL) was added tosyl chloride (3.26 g, 17.1 mmol) and pyidine (3.2 mL, 39.6 mmol). The reaction was allowed to stir at 25 °C for 14hoursafter which time the reaction was concentrated *in vacuo* to yield a wet white solid. To this solid was added ether (50 mL) and the solid was filtered and subsequently washed with additional ether (2 x 50 mL). The ether layers were pooled, concentrated *in vacuo* to yield a clear oil which was purified by flash chromatography, on a silica gel column, eluting with 1:4 ether/petroleum ether to yield **84** (4.5 g, 83 %) as a white solid. ¹H NMR (CDCl₃) δ 7.78 (d, J = 7.7, 2 H), 7.33 (d, J = 7.7 Hz, 2 H), 3,81 (d, J = 6.2 Hz, 2H), 3.37 (d, J = 6.2, 2 H), 2.44 (s, 3 H), 1.95-1.72 (m, 4 H), 1.65 (m, 1 H), 1.40 (m, 1 H), 1.03 - 0.89 (m, 4 H), 0.88 (s, 9 H), 0.04 (s, 6 H); ¹³C NMR (CDCl₃) δ 145.1, 133.7, 130.3, 128.4, 75.8, 68.9, 40.7, 38.0, 29.1, 26.5, 22.1, 18.9, -4.9; APCI *m*/*z* (rel intensity) 413 (MH⁺, 100).

### Preparation 11: (4-Prop-2-ynyl-cyclohexyl)-methanol (86).

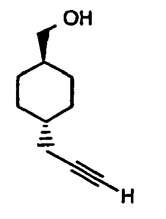

A 3-neck 250 mL-flask equipped with a gas inlet tube and dry-ice condenser was cooled to -78 °C and charged with liquid ammonia (40 mL). To the reaction mixture was added lithium wire (600 mg, 86.4 mmol) generating a deep blue solution. The mixture was allowed to stir for 1hour. Acetylene, passed through a charcoal drying tube, was added to the ammonia until all the lithium had reacted and the solution turned colorless, at which time the flow of acetylene was stopped, the acetylene-inlet tube and condenser removed and the flask outfitted with a thermometer. DMSO (20 mL) was added and the ammonia evaporated with a warm water bath until the mixture reached a temperature of 30 °C. The solution was stirred at this temperature for 2 hours until the solution stopped bubbling. The mixture was cooled to 5 °C and compound **84** (11.25 g, 27.3 mmol), in DMSO (10 mL), was added. The temperature was maintained at 5 °C. The mixture was allowed to stir at 5 °C for 0.5 hours. Then the solution was gradually warmed to room temperature and stirred for an additional 18 hours. The brown/black reaction mixture was poured slowly over ice (300 g) and extracted with ether (4 x 100 mL), dried with anhydrous sodium sulfate, and concentrated *in vacuo* to yield a yellow oil. The oil was subsequently dissolved in THF (200 mL) and changed to a brownish color upon addition of TBAF hydrate (11.20 g, 35.5mmol). The solution was allowed to stir for 24hoursunder N₂ atmosphere. After stirring, the reaction was quenched with water (200 mL) and extracted with ether (3 x 100 mL). The ether extracts were combined and concentrated *in vacuo.* The crude product was purified by chromatography, on a silica gel column, eluting with 1:1 ether/petroleum ether to yield 86 (3.91 g, 93%) as a yellow oil. ¹H NMR (CDCl₃) δ 3.45 (d, J = 6.2, 2 H), 2.10 (d, J = 6.2, 2H), 1.9 (s, 1 H), 1.94 - 1.69 (m, 4 H), 1.52 -1.34 (m, 2 H), 1.16-0.83 (m, 4 H); ¹³C NMR (CDCl₃) δ 83.8, 69.5, 69.0, 40.8, 37.7, 32.3, 29.7, 26.5.

### Preparation 12: (4-prop-2-ynylcyclohexyl)methyl acetate (87).

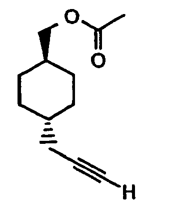

To a solution of 960 mg (6.31 mmol) of **86** in 6 mL DMF was added 0.62 mL (7.57 mmol) pyridine and 0.78 mL (8.27mmol) acetic anhydride. The reaction was allowed to stir overnight at room temperature. After 16 hours, starting material still remained. The reaction mixture was heated at 75 °C for 3 hours. The solvent was removed under reduced pressure to yield a yellow oil which was purified by flash chromatography, on silica gel, eluting with 1:3 ether/petroleum ether to yield 1.12 g (91%) of **87** as an oil. ¹H NMR (CDCl₃) δ3.87 (d, J = 6.2 Hz, 2 H), 2.06 (d, J = 4.3 Hz, 2 H), 2.03 (s, 3 H), 1.98-1.93 (m, 1 H), 1.92 -1.83 (m, 2 H), 1.83 -1.74 (m, 2 H), 1.63 -1.36 (m, 2 H), 1.12 - 0.90 (m, 4 H); ¹³C NMR (CDCl₃) δ 171.7, 83.7, 69.9, 69.6, 37.4, 3 7.3, 32. 1, 29.7, 26.5, 21.4; APCI *m*/*z* (rel intensity) 195 (M⁺, 30), 153 (M⁺, 70), 135 (M⁺, 100).

### Preparation 13: 4-prop-2-ynyl-cyclohexanecarboxylic acid (88).

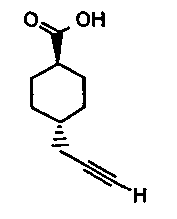

A solution of chromium trioxide (600 mg, 6.0 mmol) in 1.5 M H₂SO₄ (2.6 mL, 150 mmol) was cooled to 5 °C and added to a solution of 86 (280 mg, 1.84 mmol) in acetone (15 mL). The mixture was allowed to warm to room temperature and allowed to stir overnight. Isopropanol (4 mL) was added to the green/black solution, which turned light blue after 1hr. After adding water (15 mL), the solution was extracted with CHCl₃ (6 x 25 mL). The organic layers were pooled and concentrated *in vacuo* to yield a white solid. The solid was dissolved in ether (50 mL) and extracted with 1 M NaOH (2 x 30 mL). The basic extracts were pooled, acidified w/ 10% HCl, and re-extracted with ether (3 x 30mL). The ether layers were combined, dried with sodium sulfate and concentrated *in vacuo* to yield a white solid. The product was recrystallized from acetone/water to yield **88** (222 mg, 73%) as white needles: mp 84-85 °C; ¹H NMR (CDCl₃) δ 2.30 -2.23 (m, 1H), 2.17 - 2.11 (m, 2 H), 2.07-2.03 (m, 2 H), 1.97 - 1.91 (m, 3H), 1.51-1.39 (m, 3 H), 1.13-1.01 (m, 2 H); ¹³CNMR (CDC1₃) δ 182.5, 83.8, 69.6, 40.7, 37.7, 32.3, 29.6, 26.5; APCI *m*/*z* (rel intensity) 165 (M⁻, 100).

### Preparation 14: Methyl 4-prop-2-ynylcyclohexanecarboxylate (89).

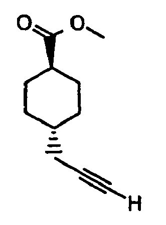

To a solution of 88 (240 mg, 1.45mmol) in 7:3 CH₂Cl₂:MeOH (10 mL) was added TMS Diazomethane (2.0 M in hexanes) (0.9 mL, 1.8 mmol) in 0.2 ml aliquots until the color remained yellow. The reaction was allowed to stir for an additional 0.25 hours at room temperature. After stirring, glacial acetic acid was added dropwise until the solution became colorless. The reaction was concentrated *in vacuo* to an oil which was purified by flash chromatography on silica gel using ether petroleum ether (1:9) to yield 89 (210 mg, 80%) as a clear oil. ¹H NMR (CDCl₃) δ 3.60 (s, 3H), 2.25 - 2.13 (m, 1 H), 2.08 - 1.94 (m, 3 H), 1.95 - 1.90 (m, 2 H), 1.49-1.31 (m, 3H), 1.10-0.93 (m,2H); ¹³C NMR (CDCl₃) δ 176.7, 83.3, 69.8, 51.9, 43.4, 36.7, 31.9, 29.2, 26.3; APCI *m*/*z* (rel intensity) 181 (MH⁺, 100).

### Preparation 15: Trans[4-(1-Propargyl)cyclohexylmethyl] methyl carbonate (90).

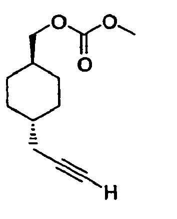

Yield: 345 mg, 81%. ¹H NMR (CDCl₃) δ 0.98-1.07, 1.40-1.52, 1.57-1.70, 1.78-1.93 (4 x m, 10H, cyclohexyl), 1.96 (t, 1H, acetylene), 2.10 (dd, 2H, -C₆H₁₀C*H*₂CCH), 3.78 (s, 3H, -OC*H*₃), 3.96 (d, -C₆H₁₀C*H*₂O-).

### Preparation 16: Trans[4-(1-Propargyl)cyclohexylmethyl] iso-butyl carbonate (91).

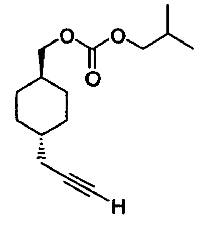

Yield: 433 mg, 83%. ¹H NMR (CDCl₃) δ 0.95 (d, 4H, -OCH₂CH(C*H*₃)₂), 0.98-1.09, 1.40-1.51, 1.57-1.70, 1.78-1.93 (4 x m, 10H, cyclohexyl), 1.94-2.04 (m, 1H, -OCH₂C*H*(CH₃)₂), 1.96 (t, 1H, acetylene), 2.10 (dd, 2H, -C₆H₁₀C*H*₂CCH), 3.91, 3.95 (2 x d, 4H, -OC*H*₂CH(CH₃)₂, -C₆H₁₀C*H*₂O-).

### Preparation 17: Trans[4-(1-Propargyl)cyclohexylmethyl] benzyl carbonate (92).

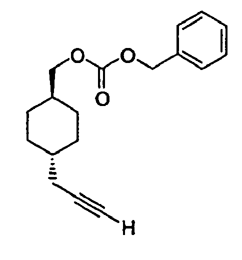

Yield: 340 mg, 69%. ¹H NMR (CDCl₃) δ 0.97-1.08, 1.40-1.49, 1.55-1.69, 1.77-1.93 (4 x m, 10H, cyclohexyl), 1.96 (t, 1H, acetylene), 2.10 (dd, 2H, -C₆H₁₀C*H*₂CCH), 3.98 (d, -C₆H₁₀C*H*₂O-), 5.15 (s, 2H, -OC*H*₂Ph), 7.33-7.40 (m, 5H, Ar).

### Preparation 18: 4-(Toluene-4-sulfonyloxymethyl)-piperidine-1-carboxylic acid tert-butyl ester (JR3215).

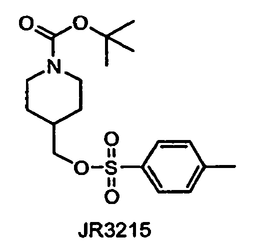

A solution of N-Boc-4-piperidinemethanol, 5.0 g (23.2 mmol) in chloroform, 50 mL, was prepared. Toluene sulfonyl chloride, 5.75 g (30.2 mmol), in 5.6 mL of pyridine (69.6 mmol) was added. The solution was stirred under nitrogen allowed to stir for 24 hours. Standard workup and chromatographic purification provided the title compound. Yield 6.0g

### Preparation 19: (R)-1-Ethynyl-(R)-3-methyl-cyclohezanol (JR3217A), (S)-1-Ethynyl-(R)-3-methyl-cyclohexanol (JR3217B).

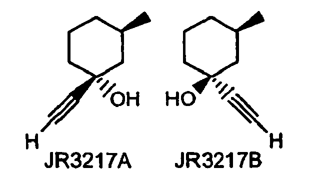

To a solution of 1.0 g (8.9 mmol) (R)-(+)-3-methyl-cyclohexanone in 50 mL of THF was added 54 mL (26.7 mmol) of 0.5 M ethynylmagnesium bromide in THF. The solution was allowed to stir at 20°C for 20 hours. Analysis by TLC indicated that the starting material had been consumed. The reaction was quenched with 5 mL of water, filtered over a plug of sand and silica, washed with EtOAc, and evaporated to yield 1.15 g of a yellow oil containing two spots (r.f.'s 0.33 (minor, JR3217A) and 0.25 (major, JR3217B), 20% EtOAc/Hexanes) which were visualized with Vanillin. The compound was purified via flash chromatography using 10% EtOAc/Hexanes (225 mL silica) to provide JR3217A and JR3217B.

### Preparation 20: 1-Prop-2-ynyl-piperidine-2-carboxylic acid methyl ester (JR3249).

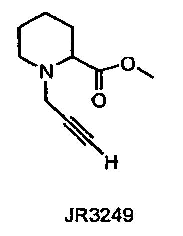

The title compound was prepared starting with 4.0g (22.3 mmol) of methylpipecolinate hydrochloride according to general method 2.

### Preparation 21: 1-Prop-2-ynyl-piperidine-4-carboxylic acid methyl ester (JR3245).

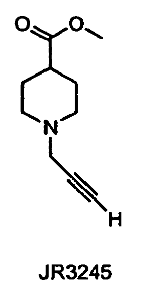

To a solution of methyl isonipecotate 3.5g (24.4 mmol, 3.30 mL) in 100 mL dichloromethane was added TEA (1.5 eq, 36.6 mmol, 5.1 mL), propargyl bromide (3.0eq, 73.2 mmol, 6.5 ml), at room temperature for 36 hrs. The reaction was quenched with 35 mL water to yield to provide a clear solution. The solution was extracted with dichloromethane 2x25 mL, dried with Na2SO4, and the solvent evaporated to provide a yellow oil. r.f. (40% EtOAc/Hexanes) 0.26 stains faint white with Vanillin, starting material r.f. 0.05 stains yellow with Vanillin. The product appeared pure after extraction.

### Preparation 22: 1-Prop-2-ynyl-pipendine-4-carboxylic acid ethyl ester (JR3271).

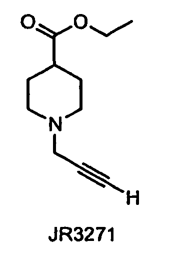

The title compound was prepared starting with 2.0g (12.7 mmol) of ethyl isonipecotate according to general method 2.

### Preparation 23: 4-Prop-2-ynyl-piperazine-1-carboxylic acid tert-butyl ester (JR3275).

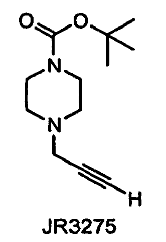

To a solution of 10.0 g (54.8 mmol) of *tert*-butyl-1-piperazine carboxylate in 60 mL acetonitile was added 5.20 mL (60.4 mmol) propargyl bromide and 37.9 g (274 mmol) anhydrous potassium carbonate. Additional propargy bromide, 1.5mL, was added after stirring for 36 hours at room temperature. The residue was evaporated to dryness. Dichloromethane, 50 mL, and water, 50 mL, were added. The reaction mixture was extracted with CH₂Cl₂, 4 x 40 mL, dried over magnesium sulfate, and evaporate to provide a brown oil. The oil was dissolved in dichloromethane and purify with a RT Scientific system using hexane/ethyl acetate gradient to yield 5.5 g (46%) of yellow oil, which ultimately crystallized upon standing.

### Preparation 24: 4-Cyanomethyl-piperazine-1-carboxylic acid ethyl ester (JR3287).

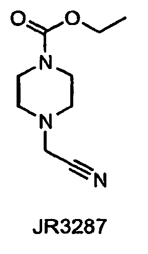

To a solution of 3g (19.0 mmol) of ethyl N-piperazinecarboxylate in 25 mL of CH₃CN was added 1.57g (1.32 mL 20.1mmol) of 2-chloroacetonitrile and 15.6g (95mmol) K₂CO₃• 1½H₂O. The suspension was stirred at room temperature for 16 hours. The reaction was analyzed using TLC (35% Ethyl acetate/Hexanes, product r.f. 0.38 vs. sm r.f. of 0.02). The analysis indicated the reaction was complete. The golden yellow solution was evaporated to dryness. The residue was extracted with CH₂Cl₂/H₂O, dried with MgSO₄, and concentrated.

### Preparation 25: 5-Prop-2-ynyl-2,5-diaza-bicyclo[2.2.1]heptane-2-carboxylic acid tert-butyl ester (JR4013).

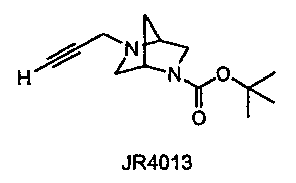

The title compound was prepared starting with 500 mg (2.52mmol) of 2,5-Diaza-bicyclo[2.2.1]heptane-2-carboxylic acid tert-butyl ester according to general method 2.

### Preparation 26: 1-Cyclohexcyl-4-prop-2-ynyl-piperazine (JR4019).

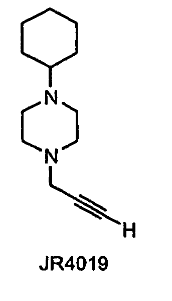

The title compound was prepared starting with 3g (17.9 mmol) of 1-cyclohexylpiperazine according to general method 2

### Preparation 27: 1-Prop-2-ynyl-piperazine (JR4029).

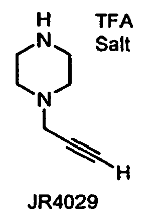

To a flame-dried 25 mL round bottom flask under nitrogen was added 2.1 g of 4-Prop-2-ynyl-piperazine-1-carboxylic acid *tert*-butyl ester. To this solid was added 5 mL of 98% TFA in 1 mL portions. The solution turned wine red, bubbled and smoked. The additional portions of TFA were added when this activity subsided. After the third portion of TFA had been added only minimal bubbling occurred. The solution was allowed to stir under nitrogen at room temperature for an additional hour and evaporated under reduced pressure to yield the product as a thick red syrup. Assumed quantitative yield of 1.16 g. The residue was suspended in 20 mL dichloromethane and used immediately without further purification for the preparation of compounds JR4031, JR4033, and JR4035.

### Preparation 28: 4-Prop-2-ynyl-piperazine-1-carboxylic acid methyl ester (JR4031).

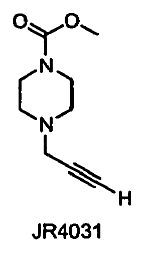

The title compound was prepared starting with 385 mg (3.1 mmol) of JR4029 and using methylchloroformate according to general method 3.

### Preparation 29: 4-Prop-2-ynyl-piperazine-1-carboxylic acid isobutyl ester (JR4035).

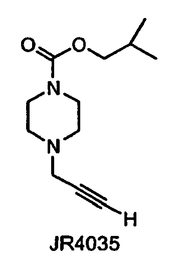

The title compound was prepared starting with 385 mg (3.1 mmol) of JR4029 and using isobutylchloroformate according to general method 3.

### Preparation 30: 3,3-Dimethyl-1-(4-prop-2-ynyl-piperidin-1-yl)-butan-1-one (JR4041).

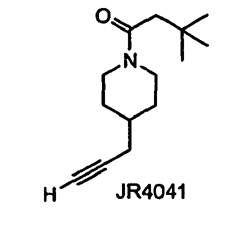

The title compound was prepared starting with *tert*-butyl ester (JR3257) and using *tert*-butylacetylchloride according to general method 3.

### Preparation 31: 1-(4-Prop-2-ynyl-piperazin-1-yl)-ethanone (JR4043).

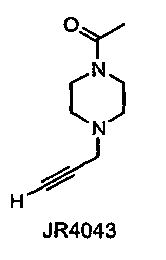

The title compound was prepared starting with 385 mg (3.1 mmol) of JR4029 and using acetyl chloride according to general method 3.

### Preparation 32: Piperidine-1,4-dicarboxylic acid mono-tert-butyl ester.

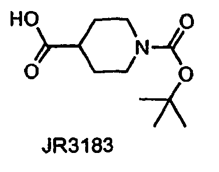

To a solution ofpiperidine-4-carboxylic acid (10 g, 77.5 mmol) and potassium carbonate (21.4 g, 155 mmol) in 150 mL of water was prepared. A solution of di-tert-butyl dicarbonate (16.9g, 77.5 mmol) in 40 mL of THF was added dropwise via addition funnel at 0 °C. The reaction was allowed to warm to room temperature gradually over 30 minutes and stirred for an additional 4 hours. The THF was removed under reduced pressure and the aqueous phase extracted with 50 mL of ether. The aqueous phase was then adjusted to pH 2 with 10 % HCl and extracted with EtOAc, 4 x 50 mL. The combined organic layers were dried over anhydrous sodium sulfate, filtered, and concentrated in vacuo to yield 17.2 g (97%) of JR3183 as a white solid. Rf= 0.2 (35% EtOAc/Hexanes stained w/ vanillin). ¹H NMR (CDCl₃) δ 11.83 (s, 1 H), 3.98 (d, J = 11.8 Hz, 2 H), 2.83 (t, J = 11.8,2 H), 2.46 (m, 1H), 1.88 (d, J = 12.9hz, 2 H), 1.2 (m, 2 H), 1.42 (s, 9 H). ¹³C NMR (CDCl₃) δ 180.0, 154.8, 79.8, 42.9, 40.8, 28.3, 27.7. APCI m/z (rel intensity) M⁻ 228.2 (100).

### Preparation 33:

The following intermediate compounds are prepared using the general method 1 described herein and the appropriate starting materials.

### (R)-1-Ethynyl-3-tert-butyl-cyclohexanol (JR3255A), (S)-1-Ethynyl-3-tert-butyl-cyclohexanol (JR3255B).

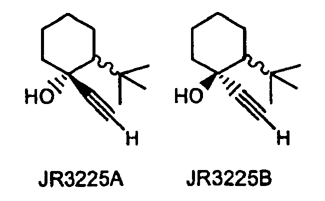

### Toluene-4-sulfonic acid 4-prop-2-ynyl-cyclobexylmethyl ester (JR3077).

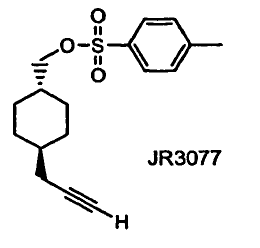

### 1-Ethyl-4-prop-2-ynyl-cyclohexane (JR3083).

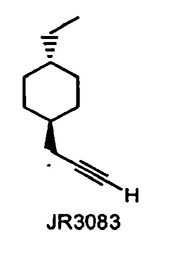

### 1-(4-Prop-2-ynyl-cyclohexyl)-ethanone (JR3115).

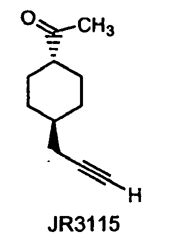

### 1,1-Dicyclohexyl-prop-2-yn-1-ol (JR3127).

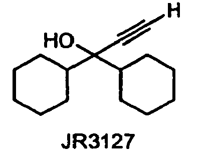

### 1-Cyclohexyl-prop-2-yn-1-ol (JR3129).

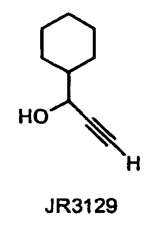

### 4-Ethyl-1-ethynyl-cyclohexanol (JR3143).

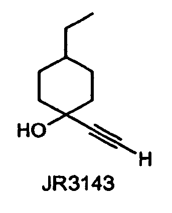

### 1-Ethynyl-3-methyl-cyclohexanol.

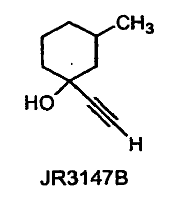

### 1-Ethynyl-3,3,5,5-tetramethyl-eyclohexanol (JR3151).

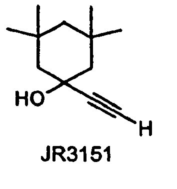

### 1-Ethynyl-4-phenyl-cyclohexanol (JR3153).

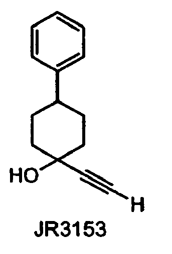

### 1-Ethynyl-2-methyl-cyclohexanol (JR3167B)

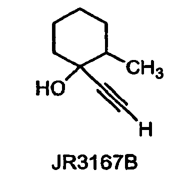

### 4-tert-Butyl-1-ethynyl-cyclohexanol (JR3191).

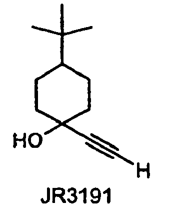

### 1-Ethynyl-3,3-dimethyl-cyclohexanol (JR3193).

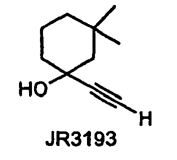

### Piperidine-1,4-dicarboxylic acid 1-tert-butyl ester 4-methyl ester (JR3195).

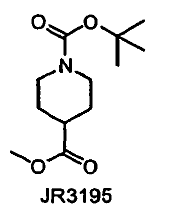

### 4-Hydroxymethyl-piperidine-1-carboxylic acid tert-butyl ester (JR3199).

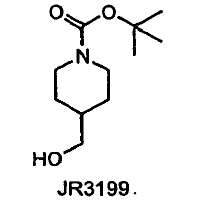

### 4-Prop-2-ynyl-piperazine-1-carboxylic acid ethyl ester (JR3211).

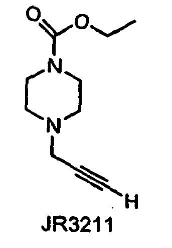

### 4-Prop-2-ynyl-piperidine-1-carboxylic acid tert-butyl ester (JR3257).

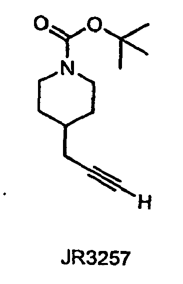

### 4-Prop-2-ynyl-piperidine-1-carboxylic acid ethyl ester (JR3267B).

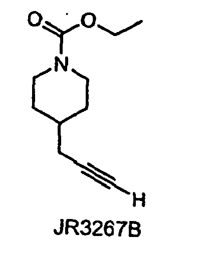

### 2-(4-Prop-2-ynyl-piperazin-1-yl)-pyrimidine (JR3277).

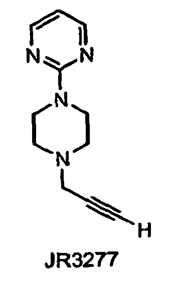

### 1-(4-Prop-2-ynyl-piperidin-1-yl)-ethanone (JR4037).

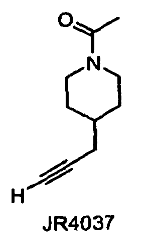

### 2,2-Dimethyl-1-(4-prop-2-ynyl-piperidin-1-yl)-propan-1-one (JR4039).

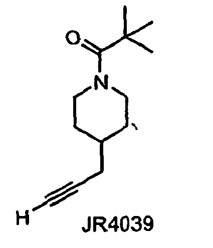

### Example 1: 4-{3-[6-Amino-9-(5-ethylcarbamoyl-3,4-dihydroxytetrahydrofuran-2-yl)-9H-purin-2-yl]-prop-2-ynyl}-cyclohexanecarboxylic acid (109).

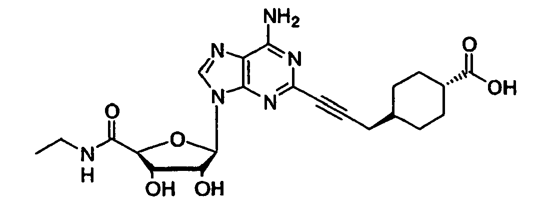

The reaction of **110** with five equivalents of LiOH in THF/water for 6 hours gave **109** (7 mg, 72%) as a white solid which was crystallized from MeOH/H₂O(0.1% TFA) after purification by reverse phase HPLC. ¹H NMR (DMSO-d6) δ 8.70 (s, 1 H), 8.41 (s, 1 H), 7.62 (s, 2 H), 5.89 (d, J = 7.25 Hz, 1 H), 4.53 (m, 1H), 4.27 (s, 1H), 4.08 (d, J = 3.6 Hz, 1 H), 2.29 (d, J = 6.4 Hz, 2 H), 2.15-1.99 (m, 1 H), 1.92- 1.76 (m, 4 H), 1.52 -1.38 (m, 1 H), 1.38 -1.19 (m, 2 H), 1.02 (t, J = 6.3 Hz 3 H); ¹³C NMR (DMSO-d6) 176.7, 169.2, 155.6, 148.9, 145.2, 141.6, 119.0, 87.7, 85.0, 84.6, 81.6, 73.1, 71.9, 43.2, 35.9, 33.3, 31.2, 28.3, 25.6, 15.0. HRMS (FAB) m/z 474.2196 [(M + H)⁺ cacld for C₂₂H₂₉N₆O₆ 474.2182].

### Example 2: 4-{3-[6-Amino-9-(5-ethylcarbamoyl-3,4-dihydroxytetrahydrofuran -2-yl)-9H-purin-2-yl]-prop-2-ynyl}-cyclohexanecarboxylic acid methyl ester (110).

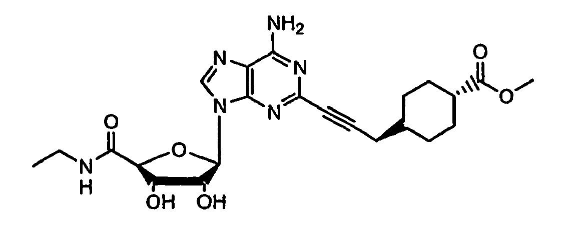

The reaction of **89** with 2-IodoNECA under the general conditions described above provided **110** (74 mg, 60%) as a white solid. ¹H NMR (CD₃OD) δ 8.23 (s, 1 H), 5.92 (d, J = 7.7 Hz, 1 H), 4.69 - 4.65 (dd, J = 7.7 Hz, 4.6 Hz, 1 H), 4.40 (s, 1 H), 4.24 (d, J = 4.6 Hz, 1 H), 3.59 (s, 3 H), 3.49 -3.31 1 (m, 2 H), 2.31 (d, J = 6.6 Hz, 2 H), 2.10 - 2.09 (m, 1H), 2.01-1.89 (m, 4 H), 1.61 - 1.32 (m, 5 H), 1.13 (t, J = 7.3 Hz, 3 H); ¹³C NMR (CD₃OD) δ 177.1, 171.1, 156.3, 149.3, 146.7, 142.4, 119.7 89.6, 86.0, 85.5, 81.6, 74.0, 72.2, 51.2, 43.2, 36.8, 34.2, 31.8, 28.9, 26.2, 14.4; HRMS (FAB) m/z 487.2325 [(M + H)⁺ cacld for C₂₃H₃₁N₆O₆ 487.2305].

### Example 3: Acetic acid 4-{3-[6-amino-9-(5-ethylcarbamoyl-3,4- dihydroxytetrahydrofuran -2-yl)-9H-purin-2-yl]-prop-2-ynyl}-cyclohexylmethyl ester (111).

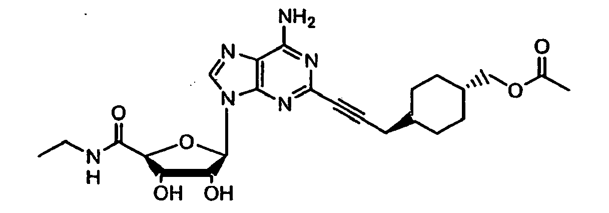

The reaction of 87 with 2-IodoNECA under the general conditions described above gave 111 (78 mg, 62%) as a white solid. ¹H NMR (CD₃OD) δ 8.22 (s, 1 H), 5.92 (d, J = 8.1 Hz, 1 H), 4.70 - 4.66 (dd, J = 8.1 Hz, 4.6 Hz, 1 H), 4.40 (d, J = 1.2 Hz, 1 H), 4.25 - 4.23 (dd, J = 4.6 Hz, 1.2 Hz, 1 H), 3.83 (d, J = 6.5, 2 H), 3.53 - 3.31 (m, 2 H), 2.29 (d, J = 6.5 Hz, 2 H), 1.97 (s, 3 H), 1.93 - 1.89 (m, 2 H), 1.79 - 1.75 (m, 2 H), 1.64 - 1.42 (m, 2 H), 1.12 (t, J = 7.3 Hz, 3 H), 1.09 - 0.91 (m, 4 H); ¹³C NMR (CD₃OD) δ 172.0, 171.2, 156.2, 149.3, 146.7, 142.5, 119.7, 89.6, 86.3, 85.5, 81.5, 74.0, 72.2, 69.6, 37.4, 37.2, 34.2, 32.1, 29.4, 26.4, 19.9, 14.5; HRMS (FAB) m/z 501.2469 [(M + H)⁺ cacld for C₂₄H₃₃N₆O₆ 501.2462].

### Example 4: 5-{6-Amino-2-[3-(4-hydroxymethyl-cyclohexyl)-prop-1-ynyl]-purin-9-yl}-3,4-dihydroxytetrahydrofuran-2-carboxylic acid ethylamide (112).

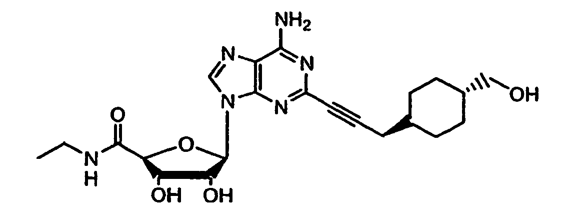

The reaction of **86** (30 mg, 0.2 mmol) with 2-IodoNECA (28 mg, 0.07 mmol) under the general conditions described above gave **112** (7 mg, 24%) as a white solid. ¹H NMR (CD₃OD) δ 8.22 (s, 1 H), 5.92 (d, J = 7.7 Hz, 1 H), 4.70 - 4.66 (dd, J = 7.7 Hz, 4.8 Hz, 1 H), 4.40 (d, J = 1.2 Hz, 1 H), 4.25 - 4.23 (dd, J = 4.8 Hz, 1.2 Hz, 1 H), 3.51 - 3.37 (m, 2 H), 3.31 (d, J = 6 Hz, 2 H), 2.30 (d, J = 6.8 Hz, 2 H), 1.94-1.89 (m, 2 H), 1.83 -1.78 (m, 2 H), 1.64-1.42 (m, 2 H), 1.12 (t, J = 7.3 Hz, 3H), 1.09- 0.91 (m, 4H); ¹³C NMR (CD₃OD) δ 170.3, 155.4, 148.5, 146.0, 141.6, 118.8, 88.7, 85.5, 84.6, 80.6, 73.1, 71.3, 66.8, 39.6, 36.9, 33.3, 31.5, 28.6, 25.6, 13.5; HRMS (FAB) m/z 459.2373 [(M + H)⁺ cacld for C₂₂H₃₁N₆O₅ 459.2356].

### Example 5: 5-{6-Amino-2-[3-(4-ethylcarbamoyl-cyclohexyl)- prop-1-ynyl]-purin -9-yl}-3,4-dihydroxytetrahydrofuran-2-carboxylic acid ethylamide (JR3037).

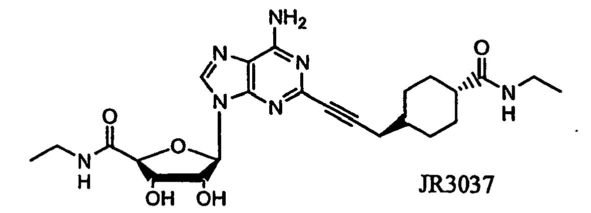

To a sealed tube containing 5 mL of freshly distilled ethylamine was added 10 mg (0.02 mmol) of ATL146e. The flask was sealed and allowed to stir at 60°C for 80hours. After this time the reaction was only about 50% complete by HPLC. The vessel was cooled to 0°C, opened, and the ethylamine was removed in vacuo to yield 4.5 mg (73%) of JR3037 as a white solid and the recovery of 4.0 mg of starting material after the residue was purified by RP-HPLC. ¹H NMR (CD₃OD-d₄) δ. ¹³C NMR (CD₃OD-d₄) δ. APCI m/z (rel intensity) 500.8 (MH⁺, 100), 327.4(3).

### Example 6: 5-{6-Amino-2-[3-(4-carbamoyl-cyclohexyl)- prop-1-ynyl]purin -9-yl}-3,4-dihydroxytetrahydrofuran-2-carboxylic acid ethylamide (JR3055).

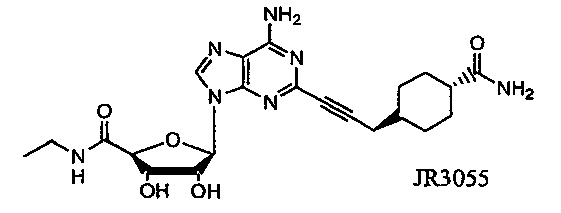

To a sealed tube containing 10 mL of saturated MeOH/NH₃ solution was added 5 mg (0.01 mmol) of ATL146e. The flask was sealed and allowed to stir at 25°C for 48 hours. The vessel was cooled to 0°C, opened, and the ammonia removed by bubbling N₂ for 1 hour. The remaining solvent was then removed in vacuo to yield 4.0 mg (83%) of JR3055 as a white solid after the residue was purified by RP-HPLC. ¹H NMR (CD₃OD-d₄) δ 8.41 (s, 1 H), 5.98 (d, J = 7.2 Hz, 1H), 4.65 (dd, J = 7.3 Hz, 4.8 Hz, 1H), 4.41 (d, J = 2.0 Hz, 1H), 4.28 (dd, J = 4.6 Hz, 2.0 Hz, 1H), 3.35 (m, 2 H), 2,37 (d, J= 6,4 Hz, 2 H) 2.10 (m, 1H), 1.90 (m,_H), 1.53 (m,_H_), 1.23 (m, _H), 1,12 (t, J = 7.3 Hz, 3 H). ¹³C NMR (CD₃OD-d₄) δ. APCI m/z (rel intensity) 472.3 (MH⁺, 100), 299.4(10).

### Example 7: 5-{6-Amino-2-[3-(4-methylcarbamoyl-cyclohexyl)- prop-1-ynyl]purin -9-yl}-3,4-dihydroxytetrahydrofuran-2-carboxylic acid ethylamide (JR3065).

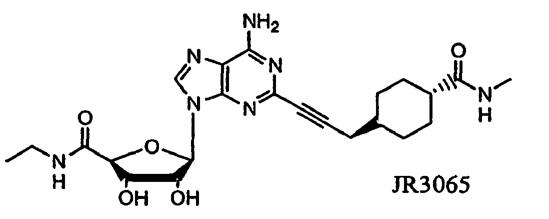

To a sealed tube containing 10 mL 2.0 M methylamine in methanol was added 16.5 mg (0.03 mmol) of ATL146e. The flask was sealed and allowed to stir at 70°C for 120hours. The vessel was cooled to 0°C, opened, and the solvent was removed in vacuo to yield 8.0 mg (48%) of JR3065 as a white solid after the residue was purified by RP-HPLC. ¹H NMR (CD₃OD-d₄) δ. ¹³C NMR (CD₃OD-d₄) δ. APCI m/z (rel intensity) 486.3 (MH⁺, 100), 313.4(35).

### Example 8: 5-[6-Amino-2-(1-hydroxy-cyclopentylethynyl)-purin-9-yl]-3,4-dihydroxytetrahydrofuran-2-carboxylic acid ethylamide (JR3135).

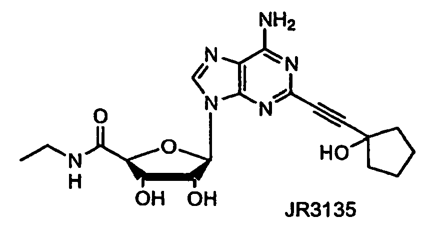

The title compound was prepared using the appropriate starting materials and procedures described herein. The results are as follows:
¹H NMR (CD₃OD-d₄) δ 8.48 (s, 1 H), 6.04 (d, J = 6.9 Hz, 1 H), 4.72 (dd, J = 6.9 Hz, J = 4.4 Hz, 1 H), 4.46 (d, J = 2.3 Hz, 1 H), 4.33 (dd, J = 4.6 Hz, J = 1.9 Hz, 1 H), 3.42 (m, 2 H), 2.04 (m, 4 H), 1.83, (m, 4 H), 1.16 (t, J = 7.3 Hz, 3 H). ¹³C NMR (CD₃OD-d₄) δ 171.9, 155.3, 150.0, 144.3, 120.6, 95.4, 90.6, 89.5, 86.2, 79.9, 74.9, 74.0, 70.5, 42.9, 35.3, 24.4, 15.3. APCI m/z (rel intensity) 417.2 (MH⁺, 100), 399.4(85), 244.3(15), 26.5(25). HRMS M⁺ actual 417.18864, observed 417.18880.

### Example 9:

### 5-[6-Amino-2-(3,3-dicyclohexyl-3-hydroxy-prop-1-ynyl)-purin-9-yl]-3,4-dihydroxytetrahydrofuran-2-carboxylic acid ethylamide (JR3139).

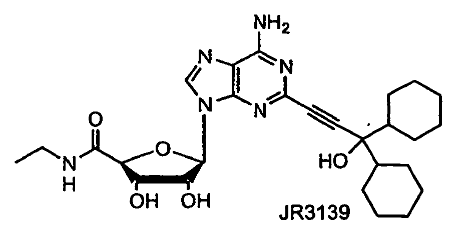

The title compound was prepared using the appropriate starting materials and procedures described herein. The results are as follows:
¹H NMR (CD₃OD-d₄) δ 8.57 (s, 1 H), 6.09 (d, J = 6.6 Hz, 1 H), 4.77 (dd, J = 6.7, Hz, J = 4.8 Hz, 1 H), 4.46 (d, J = 2.3 Hz, 1 H), 4.37 (dd, J = 4.6 Hz, J = 2.3 Hz, 1 H), 3.42 (m, 2 H) 1.80 (m, 13 H), 1.28 (m, 9 H), 1.13 (t, J = 7.3 Hz, 3 H). ¹³C NMR (CD₃OD-d₄) δ. APCI m/z (rel intensity) 527.3 (MH⁺, 60), 509.5(100), 354.4(5), 336.5(5), 279.5(8). HRMS M⁺ actual 527.29819, observed 527.29830

### Example 10:

### 5-[6-Amino-2-(4-ethyl-1-hydroxy-cyclohexylethynyl)-purin-9-yl]-3,4-dihydroxytetrahydrofuran-2-carboxylic acid ethylamide (JR3149).

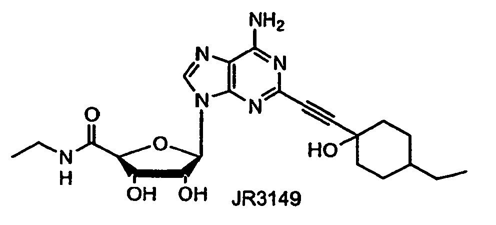

The title compound was prepared using the appropriate starting materials and procedures described herein. The results are as follows:
¹H NMR (CD₃OD-d₄) δ 8.51 (s, 1 H), 6.06 (d, J = 7.0 Hz, 1 H), 4.75 (dd, J = 6.4 Hz, J = 4.9 Hz, 1 H), 4.46 (d, J = 1.9 Hz, 1 H), 4.34 (dd, J = 4.9 Hz, J = 2.1 Hz, 1 H), 3.42 (m, 2 H), 2.12 (d, J = 11.9 Hz, 2 H), 1.80 (d, J = 11.9 Hz, 2 H), 1.58 (t, J = 12.1 Hz, 2 H), 1.28 (m, 4 H), 1.15 (t, J = 7.1 Hz, 3 H), 0.91 (t, J = 7.1 Hz, 3 H). ¹³C NMR (CD₃OD-d₄) δ 171.9, 155.4, 150.0, 144.2, 143.8, 120.6, 94.5, 90.5, 86.1, 81.8, 74.9, 74.1, 70.3, 40.5, 39.8, 35.3, 31.0, 30.2, 15.2, 12.0. APCI m/z (rel intensity) 459.4 (MH⁺, 100), 441.4(60), 268.4(10). HRMS M⁺ actual 459.23559, observed 459.23550.

### Example 11:

### 5-[6-Amino-2-(1-hydroxy-4-phenyl-cyclohexylethynyl)-purin-9-yl]-3,4-dihydroxytetrahydrofuran-2-carboxylic acid ethylamide (JR3161).

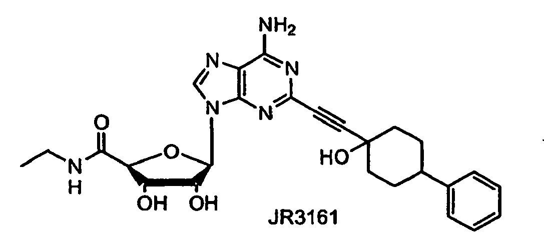

The title compound was prepared using the appropriate starting materials and procedures described herein. The results are as follows:
¹H NMR (CD₃OD-d₄) δ 8.45 (s, 1 H), 7.26 (m, 4 H), 7.14 (m, 1 H), 6.05 (d, J = 7.3 Hz, 1 H), 4.80 (dd, J = 7.3 Hz, J = 4.8 Hz, 1 H), 4.46 (d, J = 1.6 Hz, 1 H), 4.34 (dd, J = 4.7 Hz, J = 1.8 Hz, 1H), 3.44 (m, 2 H), 2.58 (m, 1 H), 2.23 (d, J = 11.7 H, 2 H), 1.92 (m, 4 H), 1.78, (m, 2 H), 1.15 (t, J = 7.2 Hz, 3 H). ¹³C NMR (CD₃OD-d₄) δ. APCI m/z (rel intensity) 507.3 (MH⁺, 100) 489.4(70), 334.3(5), 316.5(8). HRMS M⁺ actual 507.23559, observed 507.23580.

### Example 12:

### 5-[6-Amino-2-(1-hydroxy-3,3,5,5-tetramethyl-cyclohexylethynyl)purin-9-yl]-3,4-dihydroxytetrahydrofuran-2-carboxylic acid ethylamide (JR3163).

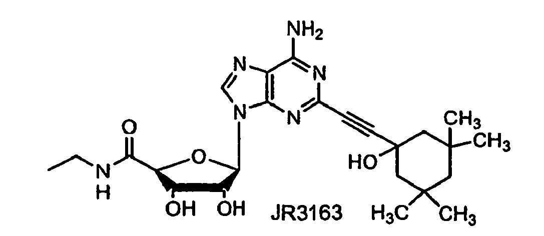

The title compound was prepared using the appropriate starting materials and procedures described herein. The results are as follows:
¹H NMR (CD₃OD-d₄) δ 8.54 (s, 1H), 6.04 (d, J = 6.9 Hz, 1 H), 4.74 (dd, J = 6.9 Hz, J = 5.0 Hz, 1 H), 4.46 (d, J = 1.9 Hz, 1H), 4.34 (dd, J = 4.7 Hz, J = 1.9 Hz, 1 H), 3.44 (m, 2 H), 1.74 (s, 4 H), 1.13 (m, 17 H). APCI m/z (rel intensity) 487.3 (MH⁺, 75), 469.4(100), 296.4 (10).

### Example 13: 5-[6-Amino-2-(1-hydroxy-2-methyl-cyclohexylethynyl)-purin-9-yl]-3,4-dihydroxytetrahydrofuran-2-carboxylic acid ethylamide (JR3177A, JR3177B).

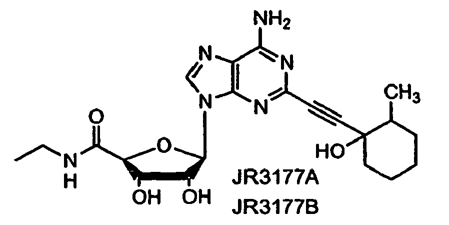

The reaction of 1-Ethynyl-2-methyl-cyclohexanol (JR3169B) (100 mg, 0.72 mmol) with 2-iodo-NECA (25 mg, 0.06 mmol) under the general coupling conditions gave JR3177A (8.0 mg) and JR3177B (8.2 mg) (overall yield 65%) as white solids after purification by a silica plug and RP-HPLC. JR3177A: ¹H NMR (CD₃OD-d₄) δ 8.47 (s, 1 H), 6.05 (d, J = 6.9 Hz, 1H), 4.77 (dd, J = 6.9 Hz, J = 4.9 Hz, 1 H), 4.45 (d, J = 1.9 Hz, 1 H), 4.34 (dd, J = 4.6 Hz, J = 2.1 Hz, 1 H), 3.41 (m, 2 H), 2.13 (d, J = 12.7 Hz, 2 H), 1.65 (m, 5 H), 1.32 (m, 2 H), 1.14 (t, J = 7.0 Hz, 3 H), 1.13 (d, J = 6.6 Hz, 3 H).. ¹³C NMR (CD₃OD-d₄) δ. APCI m/z (rel intensity) 445.3 (MH⁺, 100), 427.4(80), 254.4(14). ¹HNMR (CD₃OD-d₄) δ 8.49 (s, 1 H), 6.05 (d, J = 6.9 Hz, 1 H), 4.78 (dd, J = 6.4 Hz, J = 4.9 Hz, 1 H), 4.45 (d, J = 1.9 Hz, 1 H), 4.34 (dd, J = 4.6 Hz, J = 1.6 Hz, 1 H), 3.42 (m, 2 H), 2.12 (d, J = 12.3 Hz, 2 H), 1.65 (m, 4 H), 1.35 (m, 4 H), 1.14 (t, J = 7.3 Hz, 3 H), 1.12 (d, J = 6.6 Hz, 3 H). ¹³C NMR (CD₃OD-d₄) δ. APCI m/z (rel intensity) 445.7 (MH⁺, 100), 427.3(35), 254.4(3.5).

### Example 14: 5-[6-Amino-2-(1-hydroxy-3-methyl-cyclohexylethynyl)-purin-9-yl]-3,4-dibydroxytetrahydrofuran-2-carboxylic acid ethylamide (JR3179).

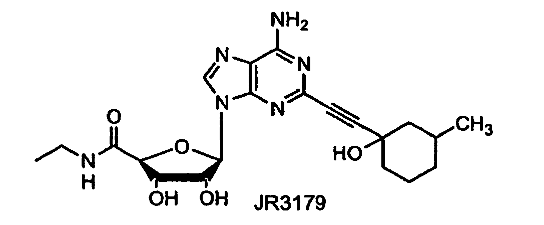

The reaction of 1-Ethynyl-3-methyl-cyclohexanol (JR3149B) (100 mg, 0.72 mmol) with 2-iodo-NECA (25 mg, 0.06 mmol) under the general coupling conditions gave JR3179 (15.0 mg, 59%) as a white solid after purification by a silica plug and RP-HPLC. ¹H NMR (CD₃OD-d₄) δ 8.49 (s, 1H), 6.06 (d, J = 6.9 Hz, 1H), 4.75 (dd, J = 6.4 Hz, J = 4.9 Hz, 1H), 4.46 (d, J = 1.9 Hz, 1H), 4.34 (dd, J = 4.9 Hz, J = 2.1 Hz, 1H), 3.42 (m, 2 H), 2.09 (d, J = 12.3 Hz, 2 H), 1.73 (m, 4 H), 1.46 (m, 1H), 1.23 (m, 1H), 1.16 9 (t, J = 7.1 Hz, 3 H), 0.95 (d, J = 6.2 Hz, 3 H), 0.89 (m, 1H). ¹³C NMR (CD₃OD-d₄) δ. APCI m/z (rel intensity) 445.3 (MH⁺, 100), 427.4(40), 254.4(4).

### Example 15: 4-{3-[6-Amino-9-(5-ethylcarbamoyl-3,4-dihydroxytetrahydrofuran -2-yl)-9H-purin-2-yl]-prop-2-ynyl}-piperazine-1-carboxylic acid ethyl ester (JR3213).

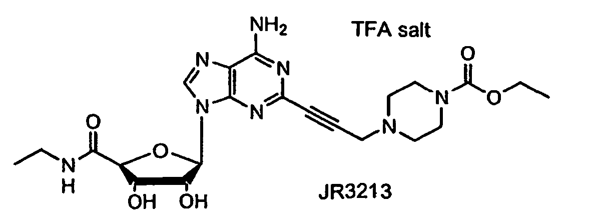

The title compound was prepared using the appropriate starting materials and procedures described herein. The results are as follows:
¹H NMR (CD₃OD-d₄) δ 8.48 (s, 1 H), 6.00 (d, J = 6.9 Hz, 1 H). 4.67 (dd, J = 6.5 Hz, J = 5.0 Hz, 1 H), 4.42 (d, J = 1.9 Hz, 1 H)), 4.39 (s, 2 H), 4.35 (dd, J = 4.7 Hz, J = 1.9 Hz, 1 H), 4,13 (q,) 3.42 (m, 2 H),. ¹³C NMR (CD₃OD-d₄) δ. APCI m/z (rel intensity) 503.4 (MH⁺, 100), 330.3 (6).

### Example 16: 5-[6-Amino-2-(3-hydrogy-2-ogo-azepan-3-ylethynyl)purin-9-yl]-3,4-dihydroxytetrahydrofuran-2-carboxylic acid ethylamide (JR3243A, JR3243B).

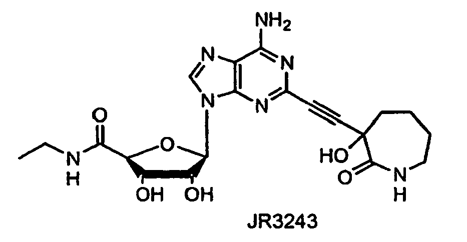

35 mg (0.081 mmol) IodoNECA (62mg alkyne, 0.41mmol), 2ml DMF, 4ml Acetonitrile, 0.2ml TEA, d(PPH3)4, CuI. Stirred overnight at room temperature (11/29/01). Rxn is tan w/ brown precipitate. TLC (20%MeOH/CH2C12) indicates rxn complete (r.f. INECA = 0.67, r.f. product = 0.45). Filtered mixture through celite, washed with 3x2mL DMF, and evaporated under vacuum to brown oil. (solid precipitates out upon the addition of MeOH, thus used DMF to load on prep plate).

The following compounds can be prepared by following the general method 4 described herein and the appropriate intermediate compounds described herein.

### Example 17: N-Ethyl 2-{3-[trans-4-(methoxycarbonyloxamethyl)-cyclohexyl]-1-propyn-1-yl}adenosine-5'-uronamide (ATL214) :

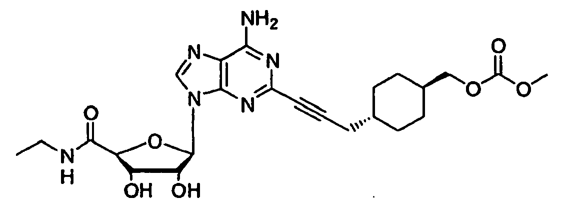

Yield 3.4 mg, 10%. ¹H NMR (CD₃OD) δ 1.18 (t, 3H, -NHCH₂C*H*₃), 1.03-1.20, 1.51-1.70, 1.79-1.85, 1.94-2.01 (4 x m, 10H, cyclohexyl), 2.35 (d, 2H, -C₆H₁₀*CH*₂CC-), 3.46 (m, 2H, -NHC*H*₂CH₃), 3.73 (s, 3H, -OC*H*₃), 3.94 (d, 2H, -C₆H₁₀C*H*₂O-), 4.29 (dd, 1H, 3'-H), 4.45 (d, 1H, 4'-H), 4.72 (dd, 1H, 2'-H), 5.97 (d, 1H, 1'-H), 8.27 (s, 1H, 8-H). APCI *mlz* 517.4 (M+H⁺).

### Example 18: N-Ethyl 2-{3-[trans-4-(isobutoxyoxycarbonyloxamethyl)-cyclohexyl]-1-propyn-1-yl}adenosine-5'-uronamide (ATL215) :

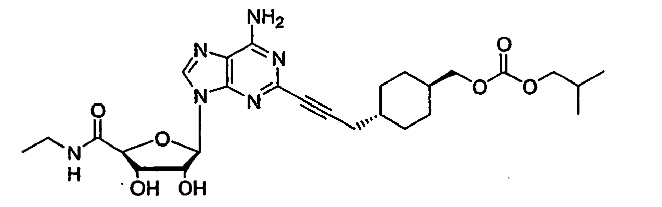

Yield 8.5 mg, 30%. ¹H NMR (CD₃OD) δ 0.94 (d, 4H, -OCH₂CH(C*H*₃)₂), 1.18 (t, 3H, -NHCH₂C*H*₃), 1.04-1.24, 1.54-1.72, 1.79-2.03 (3 x m, 11H, cyclohexyl, -OCH₂C*H*(CH₃)₂), 2.38 (d, 2H, -C₆H₁₀C*H*₂CC-), 3.43 (m, 2H, -NHC*H*₂CH₃), 3.89, 3.94 (2 x d, 4H, -C₆H₁₀C*H*₂O-, -OC*H*₂CH(CH₃)₂), 4.30 (dd, 1H, 3'-H), 4.46 (d, 1H, 4'-H), 4.71 (dd, 1H, 2'-H), 6.00 (d, 1H, 1'-H), 8.37 (br s, 1H, 8-H). APCI *m*/*z* 559.5 (M+H⁺).

### Example 19: N-Ethyl 2-{3-[trans-4-(benzoxycarbonyloxamethyl)-cyclobexyl]-1-propyn-1-yl}adenosine-5'-uronamide (ATL216):

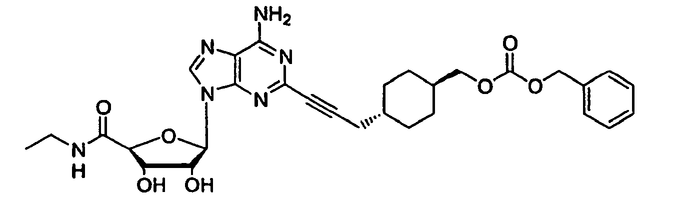

Yield 1.0 mg, 3%. ¹H NMR (CD₃OD) δ 1.17 (t, 3H, -NHCH₂CH₃), 1.03-1.23, 1.52-1.71, 1.78-1.86, 1.93-2.02 (4 x m, 10H, cyclohexyl), 2.35 (d, 2H, -C₆H₁₀C*H*₂CC-), 3.45 (m, 2H, -NHCH₂CH₃), 3.97 (d, 2H, -C₆H₁₀C*H*₂O-), 4.29 (dd, 1H, 3'-H), 4.45 (d, 1H, 4'-H), 4.72 (dd, 1H, 2'-H), 5.13 (s, 2H, -OC*H*₂Ph), 5.97 (d, 1H, 1'-H), 7.33-7.37(m, 5H, Ar), 8.30 (br s, 1H, 8-H). APCI *m*/*z* 593.3 (M+H⁺).

### Example 20: 4-{3-[6-Amino-9-(5-ethylcarbamoyl-3,4-dihydroxy-tetrahydrofuran-2-yl)-9H-purin-2-yl]-prop-2-ynyl}-cyclohexanecarboxylic acid 2-tert-butoxycarbonylamino-ethyl ester.

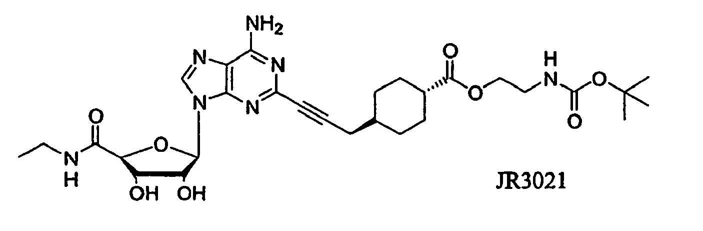

### Example 21: 5-{6-Amino-2-[3-(4-dimethylaminomethyl-cyclohexyl)-prop-1-ynyl]-purin-9-yl}-3,4-dihydroxytetrahydrofuran-2-carboxylic acid ethylamide (JR2023).

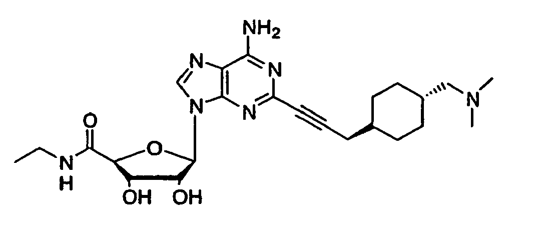

### Example 22: 4-{3-[6-Amino-9-(5-ethylcarbamoyl-3,4-dihydroxy-tetrahydrofuran-2-yl)-9H-purin-2-yl]-prop-2-ynyl}-cyclohexanecarboxylic acid 2-aminoethyl ester (JR3033).

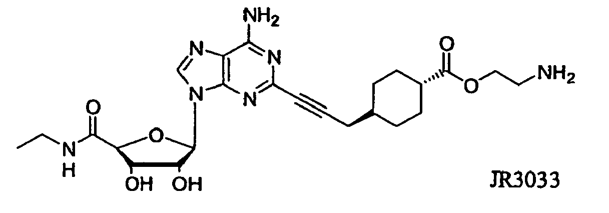

### Example 23: 4-{3-[6-Amino-9-(5-ethylcarbamoyl-3,4-dihydroxytetrahydro-furan-2-yl)-9H-purin-2-yl]-prop-2-ynyl}-1-methyl-cyclohexanecarboxylic acid methyl ester (JR3067A).

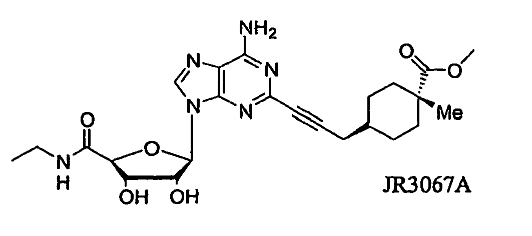

### Example 24: 4-{3-[6-Amino-9-(5-ethylcarbamoyl-3,4-dihydroxytetrahydro-furan-2-yl)-9H-purin-2-yl]-prop-2-ynyl}-1-methyl-cyclobexanecarboxylic acid methyl ester (JR3067B).

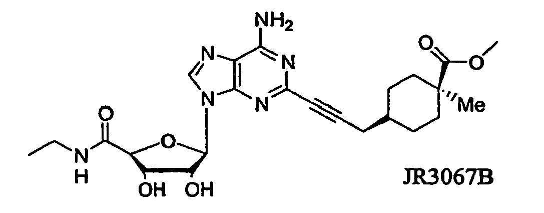

### Example 25: 5-{6-Amino-2-[3-(4-ethyl-cyclohexyl)-prop-1-ynyl]-purin-9-yl}-3,4-dihydroxytetrahydrofuran-2-carboxylic acid ethylamide (JR3087)

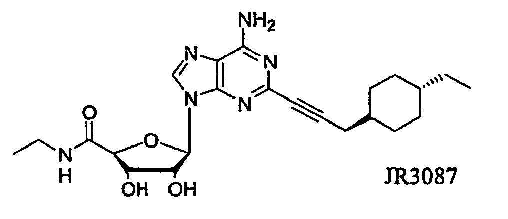

### Example 26: 5-{2-[3-(4-Acetyl-cyclohexyl)-prop-1-ynyl]-6-aminopurin-9-yl}-3,4- dihydroxytetrahydrofuran-2-carboxylic acid ethylamide (JR3119).

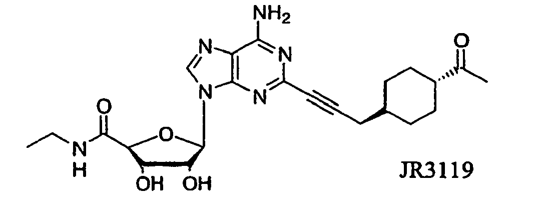

### Example 27: 5-(6-Amino-2-{3-[4-(1-hydroxy-ethyl)-cyclohexyl]-prop-1-ynyl}-purin-9-yl)-3,4-dihydroxytetrahydrofuran-2-carboxylic acid ethylamide.

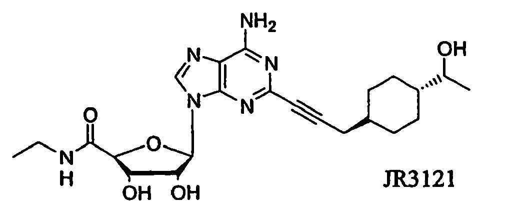

### Example 28: 5-[6-Amino-2-(1-hydroxy-2-methyl-cyclohexylethynyl)-purin-9-yl]-3,4-dihydroxytetrahydrofuran-2-carboxylic acid ethylamide (JR3181A, JR3181B).

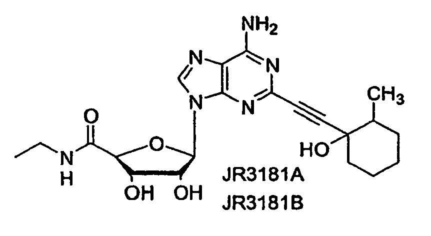

### Example 29: 5-[6-Amino-2-(1-hydroxy-3,3-dimethylcyclobexyl-ethynyl)-purin-9-yl]-3,4-dihydroxytetrahydrofuran-2-carboxylic acid ethylamide (JR3201B).

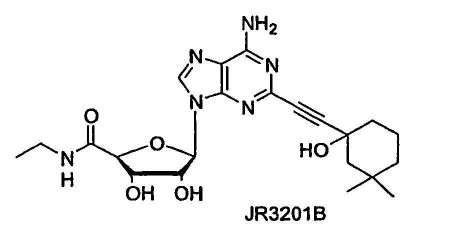

### Example 30: 5-[6-Amino-2-(4-tert-butyl-1-hydroxycyclohexylethynyl) purin-9-yl]-3,4-dihydroxytetrahydrofuran-2-carboxylic acid ethylamide (JR3203).

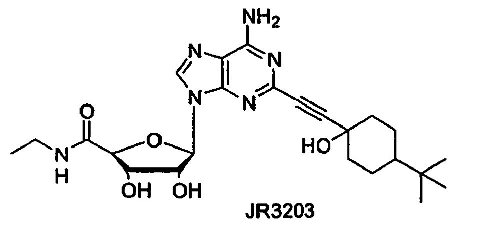

### Example 31: 5-[6-Amino-2-(1-hydroxy-3-methylcyclohexylethynyl)purin-9-yl]-3,4-dihydroxytetrahydrofuran-2-carboxylic acid ethylamide (JR3221).

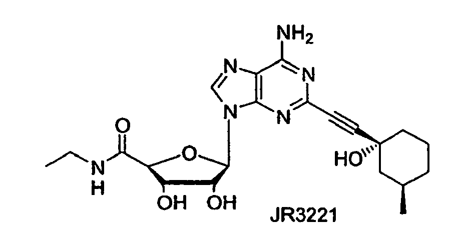

### Example 32: 5-[6-Amino-2-(1-hydroxy-3-methylcyclohexylethynyl)purin-9-yl]-3,4-dihydroxytetrahydrofuran-2-carboxylic acid ethylamide (JR3223, ATL 203).

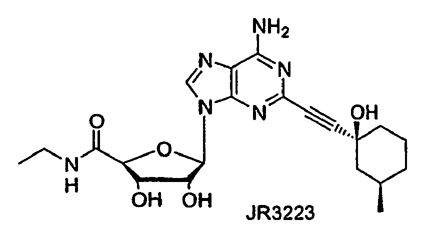

### Example 33: 5-[6-Amino-2-(2-tert-butyl-1-hydroxycyclohexylethynyl)-purin-9-yl]-3,4-dihydroxytetrahydrofuran-2-carboxylic acid ethylamide (JR3227).

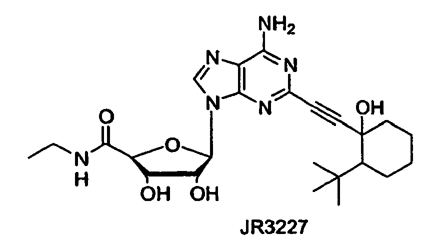

### Example 34: 1-{3-[6-Amino-9-(5-ethylcarbamoyl-3,4-dihydroxytetrahydro-furan-2-yl)-9H-purin-2-yl]-prop-2-ynyl}-piperidine-4-carboxylic acid methyl ester (JR3251).

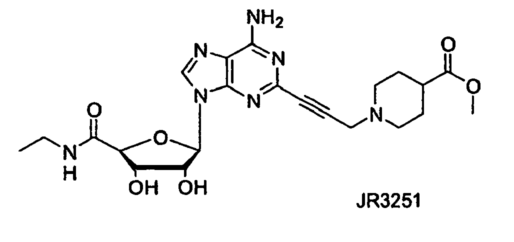

### Example 35: 1-{3-[6-Amino-9-(5-ethylcarbamoyl-3,4-dihydroxytetrahydro-furan-2-yl)-9H-purin-2-yl]-prop-2-ynyl}-piperidine-2-carboxylic acid methyl ester (JR3253).

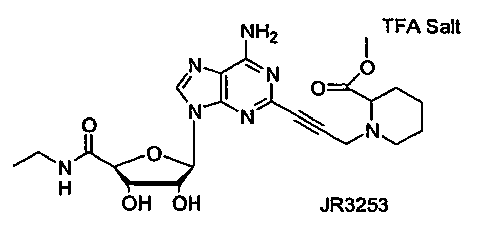

### Example 36: 4-{3-[6-Amino-9-(5-ethylcarbamoyl-3,4- dihydroxytetrahydro-furan-2-yl)-9H-purin-2-yl]-prop-2-ynyl}-piperidine-1-carboxylic acid tert-butyl ester (JR3259).

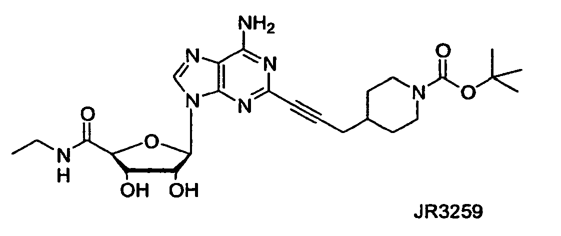

### Example 37: 4-{3-[6-Amino-9-(5-ethylcarbamoyl-3,4- dihydroxytetrahydro-furan-2-yl)-9B-purin-2-yl]-prop-2-ynyl}-piperidine-1-carboxylic acid ethyl ester (JR3269).

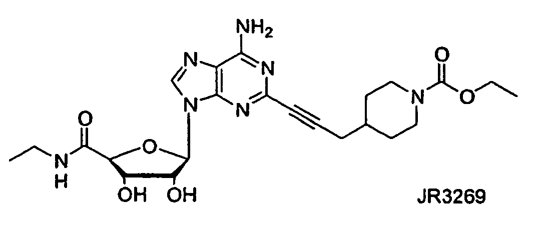

### Example 38: 1-{3-[6-Amino-9-(5-ethylcarbamoyl-3,4-dihydroxytetrahydrofuran-2-yl)-9H-purin-2-yl]-prop-2-ynyl}-piperidine-4-carboxylic acid ethyl ester (JR3279).

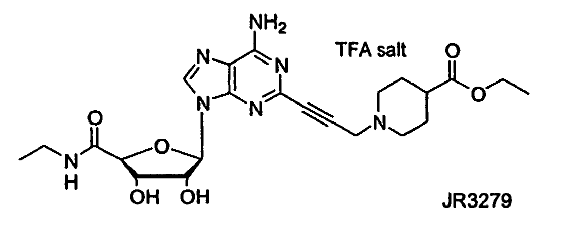

### Example 39: 4-{3-[6-Amino-9-(5-ethylcarbamoyl-3,4- dihydroxytetrahydrofuran-2-yl)-9H-purin-2-yl]-prop-2-ynyl}-piperazine-1-carboxylic acid tert-butyl ester (JR3281).

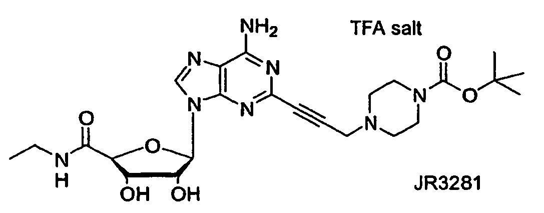

### Example 40: 5-{6-Amino-2-[3-(4-pyrimidn-2-yl-piperazin-1-yl)-prop-1-ynyl]purin-9-yl}-3,4-dihydroxytetrahydrofuran-2-carboxylic acid ethylamide (JR3283).

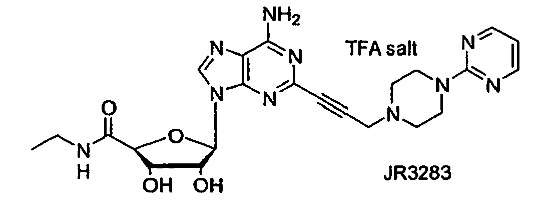

### Example 41: 5-[6-Amino-2-(3-piperazin-1-yl-prop-1-ynyl)purin-9-yl]-3,4-dihydroxytetrahydrofuran-2-carboxylic acid ethylamide (JR3289).

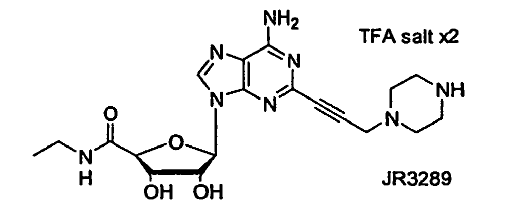

### Example 42: 1-{3-[6-Amino-9-(5-ethylcarbamoyl-3,4-dihydrozytetrahydrofuran-2-yl)-9H-purin-2-yl]-prop-2-ynyl}-piperidine-4-carboxylic acid (JR3291).

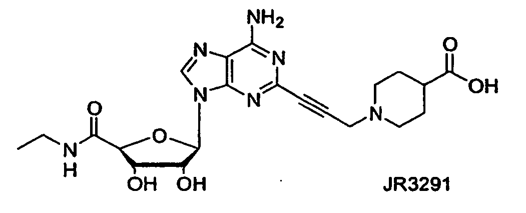

### Example 43: 4-{3-[6-Amino-9-(5-ethylcarbamoyl-3,4-dihydroxytetrahydrofuran-2-yl)-9H-purin-2-yl]-prop-2-ynyl}-piperidine-1-carboxylic acid methyl ester (JR4007).

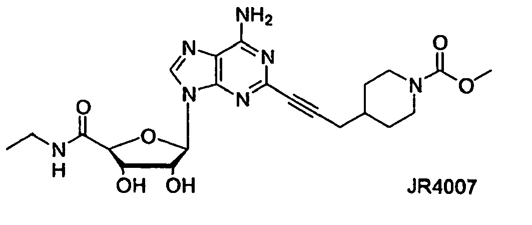

### Example 44: 4-{3-[6-Amino-9-(5-ethylcarbamoyl-3,4-dihydroxytetrahydrofuran-2-yl)-9H-purin-2-yl]-prop-2-ynyl}-piperidine-1-carboxylic acid isopropyl ester (JR4009).

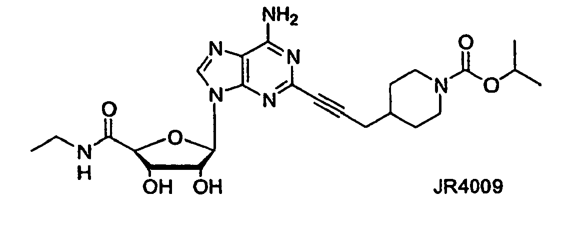

### Example 45: 4-{3-[6-Amino-9-(5-ethylcarbamoyl-3,4-dibydroxytetrahydrofuran-2-yl)-9H-purin-2-yl]-prop-2-ynyl}-piperidine-1-carboxylic acid isobutyl ester (JR4011).

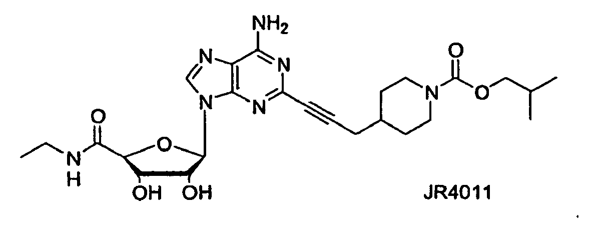

### Example 46: 5-{3-[6-Amino-9-(5-ethylcarbamoyl-3,4-dihydroxytetrahydrofuran-2-yl)-9H-purin-2-yl]prop-2-ynyl}-2,5-diazabicyclo[2.2.1]heptane-2-carboxylic acid tert-butyl ester (JR4015).

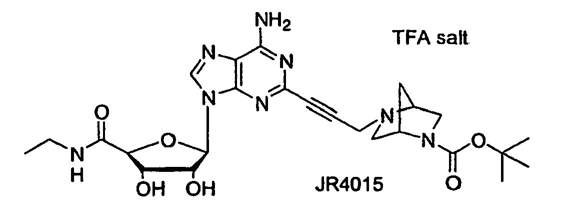

### Example 47: 5-(6-Amino-2-{3-[1-(3,3-dimethyl-butyryl)-piperidin-4-yl]-prop-1-ynyl}purin-9-yl)-3,4-dihydroxytetrahydrofuran-2-carboxylic acid ethylamide (JR4047).

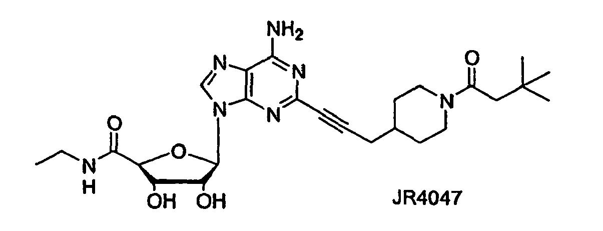

### Example 48: 5-(6-Amino-2-{3-[1-(2,2-dimethyl-propionyl)-piperidin-4-yl]-prop-1-ynyl}-purin-9-yl)-3,4-dihydroxytetrahydrofuran-2-carboxylic acid ethylamide (JR4051).

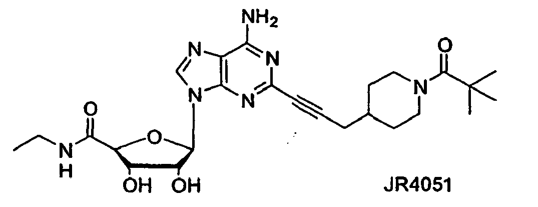

### Example 49: 4-{3-[6-Amino-9-(5-ethylcarbamoyl-3,4-dihydroxytetrahydrofuran-2-yl)-9H-purin-2-yl]-prop-2-ynyl}-piperazine-1-carboxylic acid isobutyl ester (JR4049).

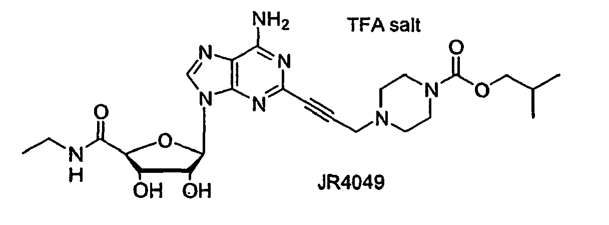

### Example 50: 5-{2-[3-(4-Acetyl-piperazin-1-yl)-prop-1-ynyl]-6-amino-purin-9-yl}-3,4-dihydroxytetrahydrofuran-2-carboxylic acid ethylamide (JR4053).

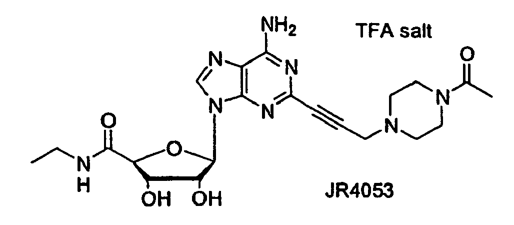

### Example 51:

The following compounds can be prepared by following the general methods described herein and the appropriate intermediate compounds:

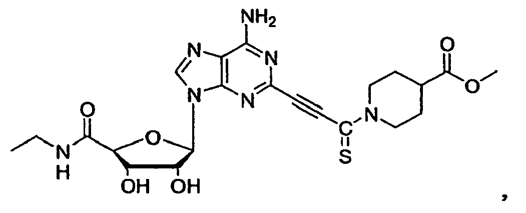

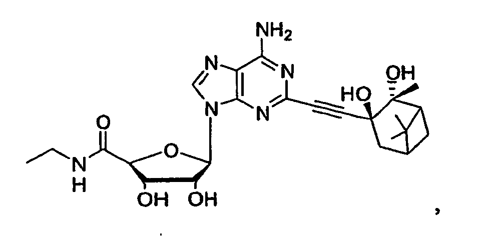

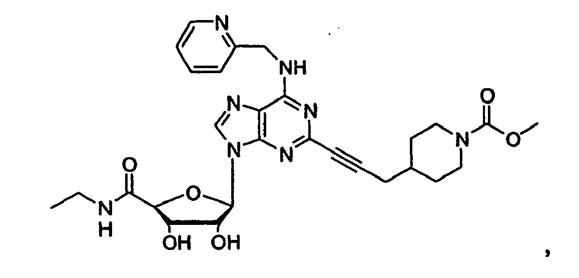

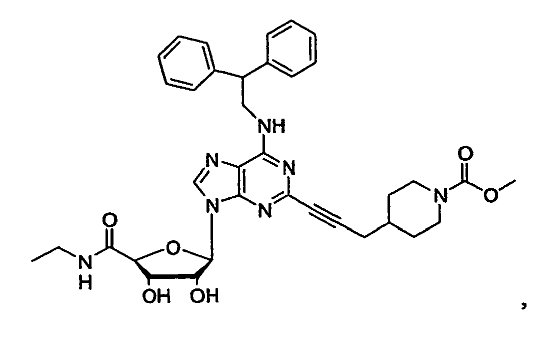

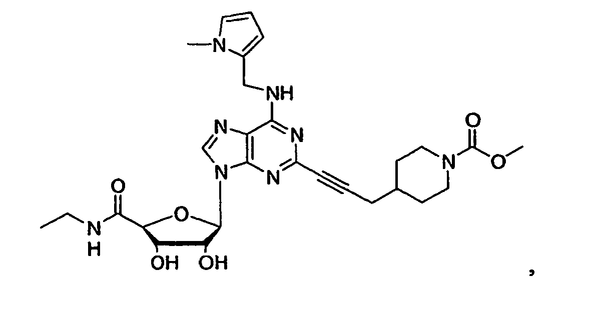

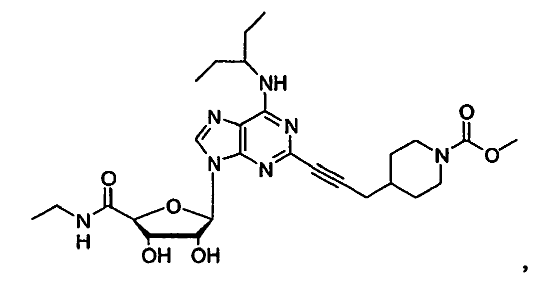

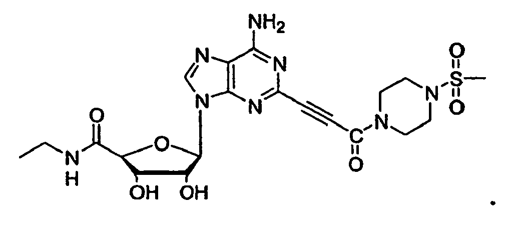

### Example 52: Preliminary in vitro studies.

The effects of A_{2A}AR agonists, initially WRC-0470 and most recently ATL146e and ATL193, were studied on phagocytic cells *in vitro* and in animal models of acute inflammation. The results indicated that these compounds are potent agonists and provide anti-inflammatory responses, both *in vitro* and *in vivo.* The effect of these compounds on human PMNL (843 receptors per cell) was characterized and quantified A_{2A}ARs. It has been documented that A_{2A}AR agonists increase PMNL intracellular cyclic AMP concentrations while decreasing TNF-enhanced adherence to a fibrinogen-coated surface. The A_{2A}AR agonists reduce TNF-stimulated superoxide release from adherent PMNLs. The super oxide release is completely blocked by the A_{2A}AR antagonist ZM 241385 (ZM). Further, A_{2A}AR agonists reduced PMNL oxidative activity in whole blood assays and decreased degranulation of activated PMNLs adhering to a biological surface. Rolipram synergistically accentuates all the effects discussed above, on activation of PMNLs. Finally, the protein kinase A inhibitor H-89 completely reversed the inhibitory effect of A_{2A}AR agonists on the PMNL oxidative burst. These findings indicate that A_{2A}AR agonists will modulate inflammation *in vivo* through direct actions on phagocytic cells.

Activation of A_{2A}ARs on human monocytes was also shown to strongly inhibit TNF release. This illustrates the anti-inflammatory action of A_{2A}AR agonists. ATL146e is more potent than CGS21680 in the inhibition of LPS-stimulated human monocyte TNF production, an effect reversed by the selective A_{2A}AR antagonist ZM241385. These data show that A_{2A}AR agonists exert A_{2A}AR-mediated anti-inflammatory effect and reduce TNF production by monocytes.

### Example 53: In vivo studies.

### Dose-response studies: murine model of septic shock

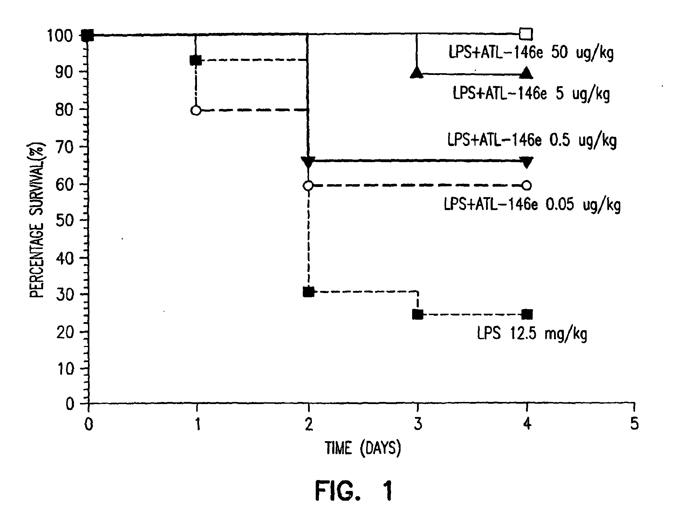

Figure 1, illustrates the mortality of C57BL/6 mice following the intraperitoneal (i.p.) inoculation with *E. coli* 026:B6 LPS (Difco). From these data, a dose of 12.5 mg/kg LPS was selected for murine mortality studies. These studies allow the generation of A_{2A} -KO mice from the same C57BL/6 background (see below). In addition, the model was validated as an excellent model of multisystem organ failure during endotoxemia and septic shock.

### A_{2A} AR agonists reduce mortality in a murine model of septic shock.

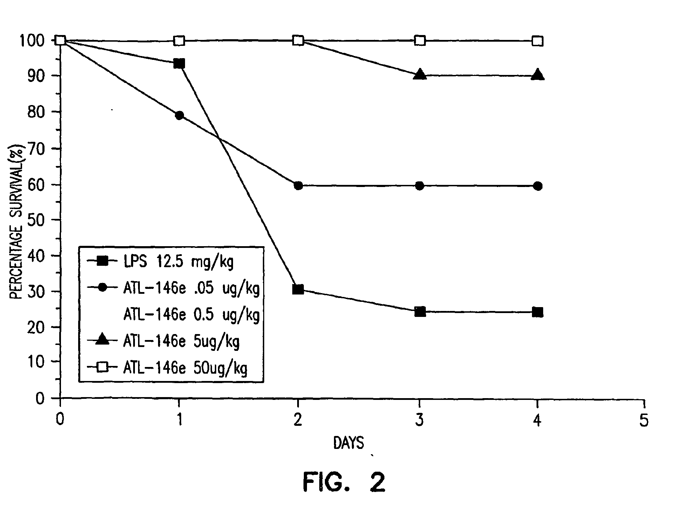

In these experiments, (n=15-16 per group; LPS 12.5mg/kg), control animals were compared to those treated with ATL146e. The agonist was dosed i.p. at 6-hour intervals for 24 hours, beginning simultaneously with the initial intraperitoneal dose of LPS. The initial dosage of ATL146e utilized was 5 µg/kg; the protection from death (p=0.0002) is illustrated in Figure 2.

At the highest dosages (of ATL146e), complete protection is achieved. All animals surviving to 4 days recover completely. At the highest doses (of ATL146e), survival at 4 days is 100% vs. mortality of 75% in mice receiving control vehicle (phosphate buffered saline i.p.). The effect of an A_{2A}AR agonist on mortality in a septic shock model is orders of magnitude superior to other "anti-inflammatory" agents used in similar models of septic shock, including corticosteroids, anti-LPS monoclonal antibodies, anti-TNF monoclonal antibodies, soluble TNF receptors, and IL-1 receptor antagonists.

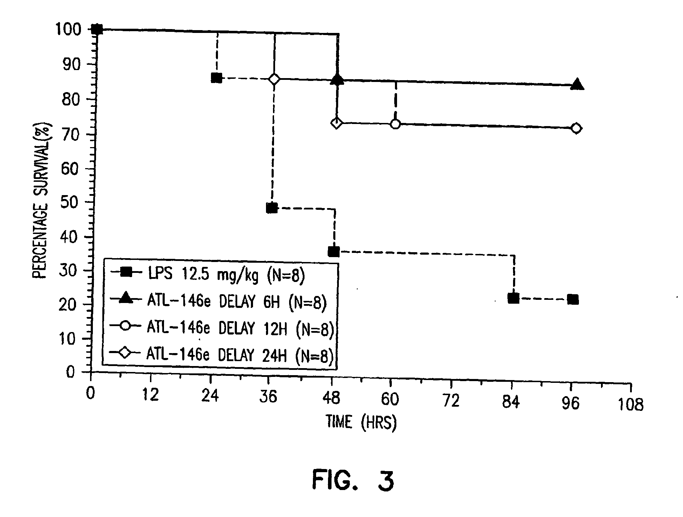

ATL146e reduced mortality in a murine model of endotoxin-induced septic shock even after a delay in the onset of therapy. In these experiments, (N=15-16 per group: LPS 12.5 mg/kg), ATL146e was administered at a dose of 5 µg/kg i.p. at six hour intervals at various times after LPS challenge for a total of four doses. The results are illustrated in Figure 3. ATL146e produced protection from death even after a delay of 24 hours following LPS challenge. These experiments are critical since patients with sepsis syndrome and septic shock are often evaluated and treated many hours after the onset of symptoms. A delay of only a few hours eliminated any protective effect in this model with monoclonal antibodies directed against LPS and/ or TNF. Thus, A_{2A} agonists may be beneficial even in far advanced, severe sepsis syndromes.

### Example 54: Protective Effects

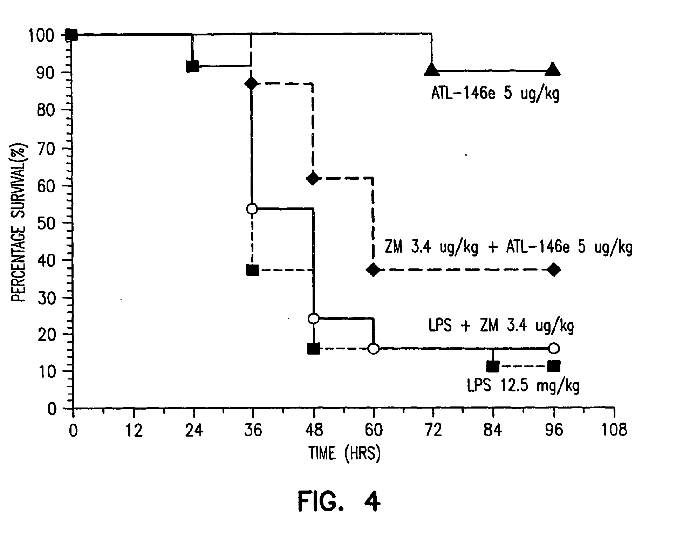

The protective effect of ATL146e on mortality in the murine model of endotoxin-induced septic shock is specific for the A_{2A} receptor. Two experimental strategies were employed to investigate the specificity of the protective effect observed with ATL146e on mortality and endotoxin-induced shock through the A_{2A} AR receptor. ZM 241385 (ZM), a specific, potent, and highly selective antagonist of the A_{2A}AR is used. As illustrated in Figure 4, ZM alone does not protect mice from death following endotoxin-induced septic shock. However, when ZM is administered in equimolor concentrations (3 µg/kg) with ATL146e, the protective efficacy of ATL146e is nearly eliminated. Thus, a specific A_{2A} AR antagonist opposes the action of ATL146e and nearly eliminates the survival benefit observed with A_{2A} agonists. In these experiments, both agents were given at six hour intervals for 24 hours beginning 12 hours after LPS challenge.

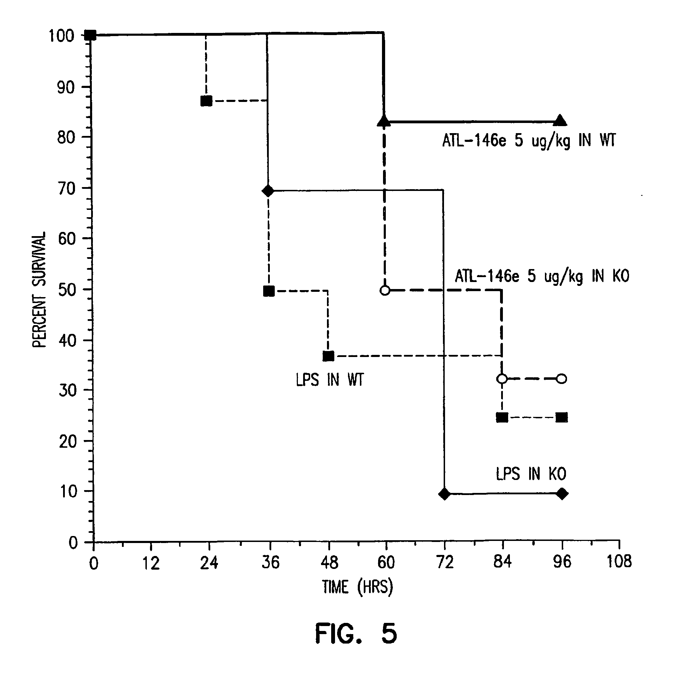

Homozygous A_{2A} -KO mice. A_{2A} -KO mice have been generated from a heterozygous breeding pair. These mice lack A_{2A}-ARs as confirmed by PCR and by localization studies of A_{2A} -ARs in wild type and A_{2A} -KO mouse brains using a selective A_{2A} AR monoclonal antibody (Figure 5). The mutant A_{2A} AR has been transferred onto a C57BL/6J background using microsattelite-assisted selection. These A_{2A} -KO mice have been used to further examine the specificity of the protective effect of ATL146e in the murine septic shock model (Figure 1). The LPS dose was again 12.5 mg/kg and approximately ten animals were included in each group. ATL was dosed at 5 µg/kg and administered for four doses at six hour intervals beginning at 12 hours following LPS inoculation. As can be seen in Figure 1, the protective effect of ATL146e is completely lost in A_{2A} -KO mice, strongly supporting the specificity of the A_{2A} agonist at the level of the A_{2A} AR

### Example 55: Treatment of mice with E. Coli.

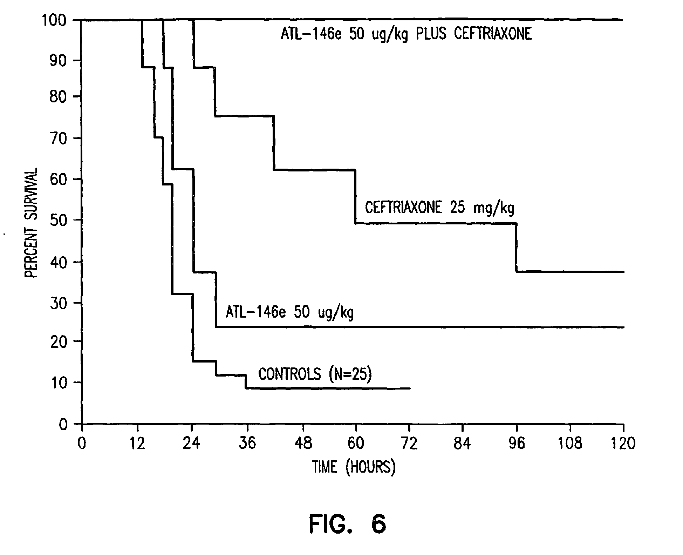

Mice were injected with live E. Coli. and treated with an antibiotic (ceftriaxone). The control group of mice were treated with antibiotic alone. All mice were injected with 20 million E. Coli IP at time 0. As indicated the mice were treated once at time 0 with ceftriaxone or with 50 µg/kg ATL146e 8 times at 6 hour intervals. The results are illustrated in Figure 6.

### Example 56: Reduction of renal production of IL-6 and RANTES.

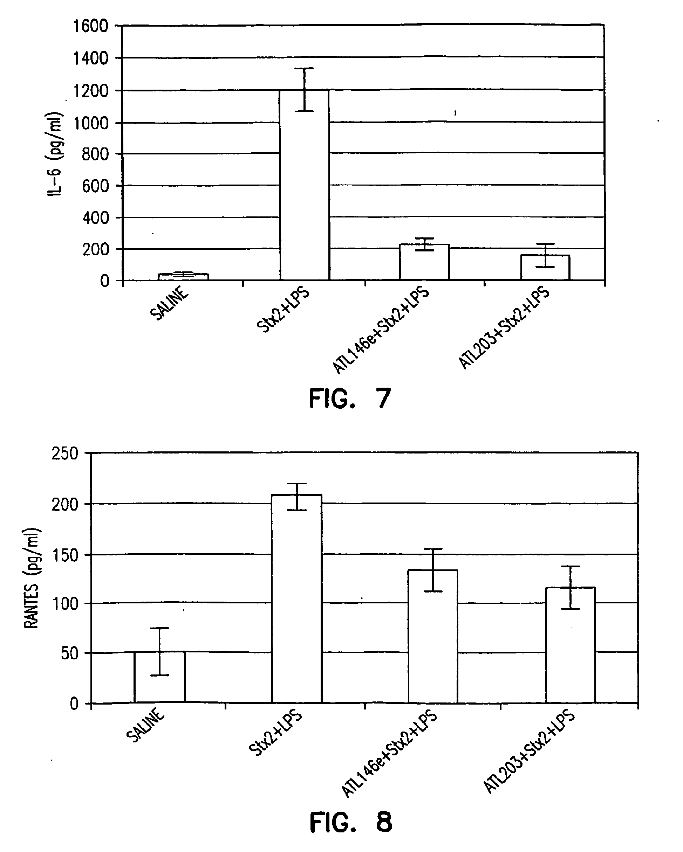

Male C57BL/6 mice were injected (i.p.) with E. coli LPS (60 ng) and purified E. coli Shiga toxin-2 (Stx2, 12 ng) at zero hour. ATL-146e or ATL-203 (both 50 µg/kg) was administered i.p. at zero hour. Animals were sacrificed at 6 hrs and the kidneys removed for processing and analysis. IL-6 protein was increased 45-fold by LPS/Stx2 at 6 hr in comparison to the saline control. Both ATL compounds sharply reduced the renal IL-6 levels to approximately 16% of those from mice exposed to LPS/Stx2 (Figure. 7).

### Reduction of renal neutrophil accumulation in mice.

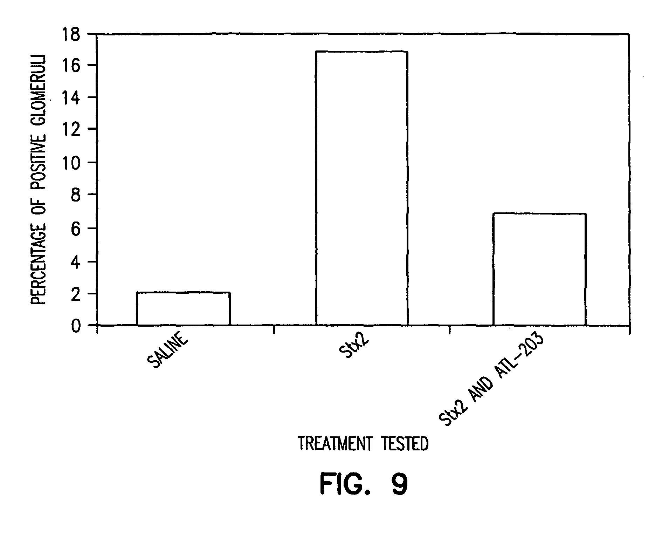

Mice received 2.4 ng purified Stx2, i.p. at zero hr. and were treated either with or without ATL-203 compound (i.p.) beginning at zero hr, and every 12 hrs thereafter. Fixed and paraffin-embedded renal samples cut to 3µ thick sections were reacted with neutrophil-specific antibody, etc. prior to analysis. The results illustrated in Figure 9 from kidneys obtained at 48 hrs post-injection of Stx2 show Stx2 caused an 8.5-fold increase in neutrophil positive glomeruli. ATL-203 effectively reduced this to 40 % of the Stx2 positive samples. A similar result was obtained when the 48 hr samples were scored for the average number of neutrophils per high-power field in the renal cortex, excluding neutrophils within the glomeruli. The accumulation of neutrophils in kidneys at 48 hrs is a natural event that leads to further renal damage and that typically contributes to the death of such animals on day 4.

These data demonstrate that the adenosine A_{2A} receptor agonist effectively reduces Stx2-dependent infiltration of neutrophils in kidneys of C57BL/6 mice (Figure 9). This result takes on added significance because the action of ATL-203 is directed at mice exposed to Stx2 alone, i.e. in the absence of LPS.

## Claims

1. Use of an A_{2A} adenosine receptor agonist in the preparation of a medicament for treating inflammation caused by pathogenic organisms in which an anti-pathogenic agent and said A_{2A} adenosine receptor agonist are to be administered to a patient simultaneously or sequentially.

2. Use according to claim 1 in the preparation of a medicament for treating inflammation caused by a viral organism.

3. Use according to claim 1, wherein the pathogen is a bacteria and the anti-pathogenic agent is an antibiotic; or
wherein the pathogen is a virus and the anti-pathogenic agent is an antiviral agent; or
wherein the pathogen is yeast or fungus and the anti-pathogenic agent is an antifungal agent.

4. Use according to claim 3, wherein the inflammation is caused by E. Coli.

5. Use according to claim 3, wherein the bacteria causes hemolytic uremic syndrome.

6. Use according to any of claims 1 to 5, wherein the A_{2A} adenosine receptor agonist is a compound having formula (I): 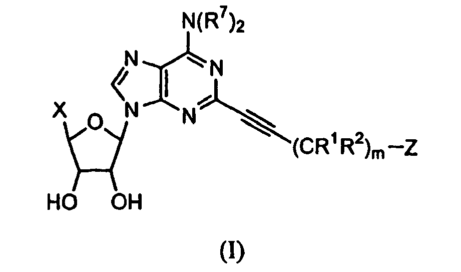 wherein
Z is CR³R⁴R⁵ or NR⁴R⁵;
each R¹ is independently hydrogen, halo, -OR^{a}, -SR^{a}, (C₁-C₈)alkyl, cyano, nitro, trifluoromethyl, trifluoromethoxy, (C₃-C₈)cycloalkyl, heterocycle, heterocycle(C₁-C₈)alkylene-, aryl, aryl(C₁-C₈)alkylene-, heteroaryl, heteroaryl(C₁-C₈)alkylene-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, R^{a}S(=O)₂-, or -N=NR^{b};
each R² is independently hydrogen, halo, (C₁-C₈)alkyl, (C₃-C₈)cycloalkyl, heterocycle, heterocycle(C₁-C₈)alkylene-, aryl, aryl(C₁-C₈)alkylene-, heteroaryl, or heteroaryl(C₁-C₈)alkylene-; or
R¹ and R² and the atom to which they are attached is C=O, C=S or C=NR^{d};
R⁴ and R⁵ together with the atoms to which they are attached form a saturated or partially unsaturated, mono-, bicyclic- or aromatic ring having 3, 4, 5, 6, 7, 8, 9 or 10 ring atoms optionally comprising 1, 2, 3, or 4 heteroatoms selected from non-peroxide oxy (-O-), thio (-S-), sulfinyl (-SO-), sulfonyl (-S(O)₂-) or amine (-NR^{b}-) in the ring;
wherein any ring comprising R⁴ and R⁵ is substituted with from 1 to 14 R⁶ groups; wherein each R⁶ is independently hydrogen, halo, -OR^{a}, -SR^{a}, (C₁-C₈)alkyl, cyano, nitro, trifluoromethyl, trifluoromethoxy, (C₁-C₈)cycloalkyl, (C₆-C₁₂)bicycloalkyl, heterocycle or heterocycle (C₁-C₈)alkylene-, aryl, aryl (C₁-C₈)alkylene-, heteroaryl, heteroaryl(C₁-C₈)alkylene-, -CO₂R^{a}, R^{a}C(=O)O-, R^{a}C(=O)-, -OCO₂R^{a}, R^{b}R^{c}BNC(=O)O-, R^{a}OC(=O)N(R^{b})-, R^{b}R^{c}N-, R^{b}R^{c}NC(=O)-, R^{a}C(-O)N (R^{b}). R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, NNR^{b}, or two R⁶ groups and the atom to which they are attached is C=O, C=S or, two R⁶ groups together with the atom or atoms to which they are attached can form a carbocyclic or heterocyclic ring;
R³ is hydrogen, halo, -OR^{a}, -SR^{a}, (C₁-C₈)alkyl, cyano, nitro, trifluoromethyl, trifluoromethoxy, (C₃-C₈)cycloalkyl, heterocycle, heterocycle(C₁-C₈)alkylene-, aryl, aryl(C₁-C₈)alkylene-, heteroaryl, heteroaryl(C₁-C₈)alkylene-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, R^{a}S(=O)₂-, -NNR^{b}: or if the ring formed from CR⁴R⁵ is aryl or heteroaryl or partially unsaturated then R³ can be absent;
each R⁷ is independently hydrogen, (C₁-C₈)alkyl, (C₃-C₈)cycloalkyl, aryl or aryl(C₁-C₈)alkylene, heteroaryl, heteroaryl(C₁-C₈)alkylene-;
X is -CH₂OR^{a}, -CO₂R^{a}, -OC(O)R^{a}, -CH₂OC(O)R^{a}, -C(O)NR^{b}R^{c}, -CH₂SR^{a}, -C(S)OR^{a}, -OC(S)R^{a}, -CH₂OC(S)R^{a} or -C(S)NR^{b}R^{c} or -CH₂N(R^{b})(R^{c});
wherein any of the alkyl, cycloalkyl, heterocycle, aryl, or heteroaryl groups of R¹, R², R³, R⁶ and R⁷ is optionally substituted on carbon with one or more (e.g. 1,2,3, or 4) substituents selected from the group consisting of halo, -OR^{a}, -SR^{a}, (C₁-C₈)alkyl, cyano, nitro, trifluoromethyl, trifluoromethoxy, (C₃-C₈)cycloalkyl, (C₆-C₁₂)bicycloalkyl, heterocycle or heterocycle(C₁-C₈)alkylene-, aryl, aryloxy, aryl (C₁-C₈)alkylene-, heteroaryl, heteroaryl(C₁-C₈)alkylene-. -CO₂R^{a}, R^{a}C(=O)O-, R^{a}C=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(-S)N(R^{b})-, -OPO₃R^{a}, R^{a}OC(=S)-, R^{a}C(=S)-, -SSR^{a}, R^{a}S(=O)ₚ--, R^{b}R^{c}NS(O)ₚ-, and -N=NR^{b};
wherein any (C₁-C₈)alkyl, (C₃-C₈)cycloalkyl, (C₆-C₁₂)bicycloalkyl, (C₁-C₈)alkoxy. (C₁-C₈)alkanoyl, (C₁-C₈)alkylene, or heterocycle, is optionally partially unsaturated;
each R^{a}, R^{b} and R^{c} is independently hydrogen, (C₁-C₈)alkyl, or (C₁-C₈)alkyl substituted with 1-3 (C₁-C₈)alkoxy, (C₃-C₈)cycloalkyl, (C₁-C₈)alkylthio, amino acid, aryl, aryl(C₁-C₈)alkylone, heteroaryl, or heteroaryl(C₁-C₈)alkylene; or R^{b} and R^{c}, together with the nitrogen to which they are attached, form a pyrrolidino, piperidino, morpholino, or thiomorpholino ring; and
R^{d} is hydrogen or (C₁-C₆)alkyl;
m is 0 to 8 and p is 0 to 2;
or a pharmaceutically acceptable salt thereof.

7. Use according to claim 6, wherein R¹ is hydrogen, -OH, -CH₂OH, -OMe, -OAc, -NH₂, -NHMe, -NMe₂ or NHAc.

8. Use according to claim 6, wherein R¹ is hydrogen, -OH, -OMe, -OAc, -NH₂, -NHMe, -NMe₂ or -NHAc.

9. Use according to claim 6, wherein R¹ is hydrogen, Oh, OMe, or NH₂.

10. Use according to claim 6, wherein R¹ is hydrogen, OH, or NH₂.

11. Use according to claim 6, wherein R¹ is hydrogen or OH.

12. Use according to any of claims 6-11, wherein R² is hydrogen, (C₁-C₈)alkyl, cyclopropyl, cyclohexyl or benzyl.

13. Use according to any of claims 6-11, wherein R² is hydrogen, methyl, ethyl or propyl.

14. Use according to any of claims 6-11, wherein R² is hydrogen or methyl; .

15. Use according to any of claims 6-11, wherein R² is hydrogen.

16. Use according to claim 6, wherein R¹, R² and the carbon atom to which they are attached is carbonyl (C=O),

17. Use according to any of claims 6 to 16, wherein R³ is hydrogen, OH, OMe, OAc, NH₂, NHMe, NMe₂ or NHAc.

18. Use according to any of claims 6 to 16, wherein R³ is hydrogen, OH, OMe, or NH₂.

19. Use according to any of claims 6 to 16, wherein R³ is hydrogen, OH, or NH₂.

20. Use according to any of claims 6 to 16, wherein R³ is hydrogen or OH.

21. Use according to any of claims 6 to 20, wherein the ring comprising R⁴, R⁵ and the atom to which they are connected is cyclopentane, cyclohexane, piperidine, dihydro-pyridine, tetrahydro-pyridine, pyridine, piperazine, decaline, tetrahydro-pyrazine, dihydro-pyrazinc, pyrazine, dihydro-pyrimidine, tetrahydro-pyrimidine, hexahydro-pyrimidine, pyrazine, imidazole, dihydro-imidazole, imidazolidine, pyrazole, dihydro-pyrazole, or pyrazolidine.

22. Use according to any of claims 6 to 20, wherein the ring comprising R⁴, R⁵ and the atom to which they are connected is cyclopentane, cyclohexane, piperidine, dihydro-pyridine, tetrahydro-pyridine, pyridine, piperazine, tetrahydro-pyrazine, dihydro-pyracine, Pyrazine, dihydro-pyrimidine, tetrahydro-pyrimidine, hexahydro-pyrimidine, pyrazine, imidazole, dihydro-imidazole, imidazolidine, pyrazole, dihydro-pyrazole, or pyrazolidine.

23. Use according to any of claims 6 to 20, wherein the ring comprising R⁴, R⁵ and the atom to which they are connected is cyclohexane, piperidine or piperazine.

24. Use according to any of claims 6 to 23, wherein R⁶ is (C₁-C₈)alkyl, or substituted (C₁-C₈)alkyl, -OR^{a}, -CO₂R^{a}, R^{a}C(=O)-, R^{a}C(=O)O-, R^{b}R^{c}N-, R^{b}R^{c}NC(=O)-, or aryl.

25. Use according to any of claims 6 to 23, wherein R⁶ is (C₁-C₈)alkyl, -OR^{a}, -CO₂R^{a}, R^{a}C(=O)-, RaC(=O)O-, R^{b}R^{c}N-, R^{b}R^{c}NC(=O)-, or aryl.

26. Use according to any of claims 6 to 23, wherein R⁶ is methyl, ethyl, butyl, OH, OR^{a}, -CO₂R^{a}, R^{a}C(=O)-, OC(=O)CH₂CH₃, -CONR^{b}R^{c}, NR^{b}R^{c} or phenyl.

27. Use according to any of claims 6 to 23, wherein R⁶ is OH, OMe, methyl, ethyl, t-butyl, -CO₂R^{a}, -CONR^{b}R^{c}, OAc, NH₂, NHMe, NMe₂, NHEt or N(Et)₂.

28. Use according to any of claims 6 to 23, wherein R⁶ is methyl, ethyl, t-butyl, phenyl, -CO₂R^{a} -CONR^{b}R^{c}, or-(=O)CR^{a}.

29. Use according to any of claims 6 to 23, wherein R⁶ is methyl, ethyl, -CO₂R^{a} -CONR^{b}R^{c}, or OAc.

30. Use according to any of claims 6 to 23, wherein R⁶ is -(CH₂)₁₋₂OR^{a}, -(CH₂)₁₋₂C(=O)OR^{a}, -(CH₂)₁₋₂OC(=O)R^{a}, -(CH₂)₁₋₂C(=O)R^{a}, -(CH₂)₁₋₂OCO₂R^{a}, -(CH₂)₁₋₂NHR^{a}, -(CH₂)₁₋₂NR^{b}R^{c}, -(CH₂)₁₋₂OC(=O)NHR^{a}, or -(CH₂)₁₋₂OC(=O)NR^{b}R^{c}.

31. Use according to any of claims 6 to 23, wherein R⁶ is -CH₂OH, -CH₂OAc, -CH₂OCH₃, -CH₂C(=O)OCH₃, -CH₂OC(=O)CH₃,-CH₂C(=O)CH₃, -CH₂OCO₂CH₃, -CH₂NH(CH₃), or -(CH₂)₁₋₂N(CH₃)₂.

32. Use according to any of claims 6 to 23, wherein R⁶ is -CH₂OH, -CH₂OAc, -C(=O)OCH₃, -C(=O)CH₃, OCO₂CH₃ -OCO₂CH₃, -CH₂NH(CH₃), or -(CH₂)₁₋₂N(CH₃)₂.

33. Use according to any claims 6 to 32, wherein number of R⁶ groups substituted on the R⁴R⁵ ring is from 1 to 4.

34. Use according to any of claims 6 to 33, wherein R^{a} and R^{b} are independently hydrogen, (C₁-C₄)alkyl, aryl or aryl(C₁-C₈)alkylene.

35. Use according to any of claims 6 to 33, wherein R^{a} and R^{b} are independently hydrogen, methyl or ethyl, phenyl or benzyl.

36. Use according to any of claims 6 to 35, wherein R^{a} is (C₁-C₈)alkyl.

37. Use according to any of claims 6 to 33, wherein R^{a} and R^{b} are independently methyl, ethyl, propyl or butyl.

38. Use according to any of claims 6 to 33, wherein R^{a} and R^{b} are independently methyl, ethyl, i-propyl, i-butyl or tert-butyl.

39. Use according to any of claims 6 to 38, wherein R^{b} and R^{c} and the atom to which they are attached form a ring.

40. Use according to any of claims 6 to 39, wherein R⁷ is hydrogen, alkyl, aryl or aryl(C₁-C₈)alkylene.

41. Use according to any of claims 6 to 39, wherein R⁷ is hydrogen, methyl or ethyl, phenyl or benzyl.

42. Use according to any of claims 6 to 39, wherein R⁷ H, or methyl.

43. Use according to any of claims 6 to 42, wherein N(R⁷)₂ is amino, methylamino, dimethylamino; ethylamino; pentylamino, diphenylethylamino, pyridylmethylamino, diethylamino or benzylamino.

44. Use according to any of claims 6 to 42, wherein N(R⁷)₂ is amino, methylamino, dimethylamino; ethylamino; diethylamino or benzylamino.

45. Use according to any of claims 6 to 42, wherein N(R⁷)₂ is amino, or methylamino.

46. Use according to any of claims 6 to 45, wherein X is -CH₂OR^{a}, -CO₂R^{a}, -OC(O)R³, -CH₂OC(O)R^{a}, -C(O)NR^{b}R^{c}.

47. Use according to any of claims 6 to 45, wherein X is -CH₂OR^{a} or -C(O)NR^{b}R^{c}.

48. Use according to any of claims 6 to 45, wherein X is -CH₂OH or -C(O)NHCH₂CH₃.

49. Use according to any of claims 6 to 19, wherein m is 0, 1, or 2.

50. Use according to any of claims 6 to 49, wherein the rings comprising R⁴, R⁵ and the atom to which they are connected are selected from the group consisting of the following, in which q is 0-14:

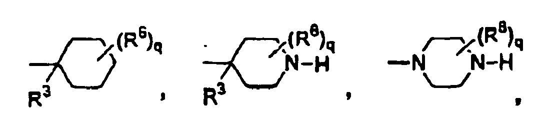

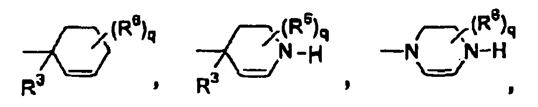

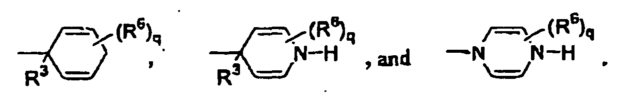

51. Use according to any of claims 6 to 49, wherein the rings comprising R⁴, R⁵ and the atom to which they are connected are selected from the group consisting of:

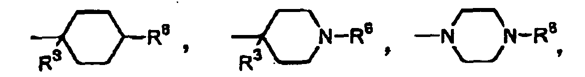

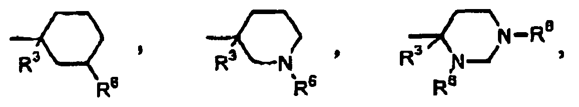

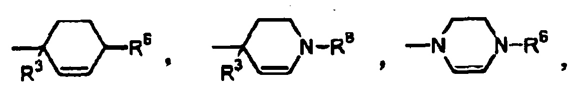

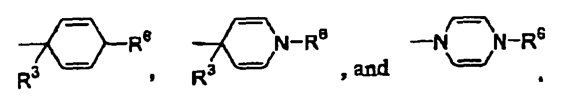

52. Use according to any of claims 6 to 51, wherein the ring comprising R⁴ and R⁵ is 2-methylcyclohexane, 2,2-dimethylcyclohexane, 2-phenyl-cyclohcxane, 2-cthylcyclohexane, 2,2-diethylcyclohexane, 2-tert-butylcyclohexane, 3-methylcyclohexane, 3,3-diroethylcyclohexane, 4-methylcyclohexane, 4-ethylcyclohexane, 4-phenyl cyclohexane, 4-tert-butylcyclohexane, 4-carboxymethyl cyclohexane, 4-carboxyethyl cyclohexane, 3,3,5,5-tetramethyl cyclohexane, 2,4-dimethyl cyclopentane- 4-cyclohexanecarboxyic acid, 4-cyclohexanecarboxyic acid esters, or 4-methyloxyalkanoyl-cyclohexane.

53. Use according to any of claims 6 to 51, wherein the ring comprising R⁴ and R⁵ is 4-piperidine, 4-piperidine-1-carboxylic acid, 4-piperidine-1-carboxylic acid methyl ester, 4-piperidine-1-carboxylic acid ethyl ester, 4-piperidine-1-carboxylic acid propyl ester, 4-piperidine-1-carboxylic acid tert-butyl ester, 1-piperidine, 1-piperidine-4-carboxylic acid methyl ester, 1-piperidine-4-carboxylic acid ethyl ester, 1-piperidine-4-carboxylic acid propyl ester, 1-piperidine-4-carboxylic acid tert-butyl ester, 1-piperidine-4-carboxylic acid methyl ester, 3-piperidine, 3-piperidine-1-carboxylic acid. 3 piperidine-1-carboxylic acid methyl ester, 3-piperidine-1-carboxylic acid tert-butyl ester, 1,4-piperazine, 4-piperazine-1-carboxylic acid. 4-piperazine-1-carboxylic acid methyl ester, 4-piperazine-1-carboxylic acid ethyl ester, 4-piperazine-1-carboxylic acid propyl ester, 4-piperazine-1-carboxylic acid tert-butylester, 1,3-piperazine, 3-piperazine-1-carboxylic acid, 3-piperazine-1-carboxylic acid methyl ester, 3-piperazine-1-carboxylic acid ethyl ester, 3-piperazine-1-carboxylic acid propyl ester, 3-piperidine-1-carboxylic acid tert-butylester, 1-piperidine-3-carboxylic acid methyl ester, 1 -piperidine-3-carboxylic acid ethyl ester, 1-piperidine-3-carboxylic acid propyl ester or 1-piperidine-3-carboxylic acid tert-butyl ester,

54. Use according to claim 6, wherein the A_{2A} adenosine receptor agonist is a compound having a formula selected from the following : 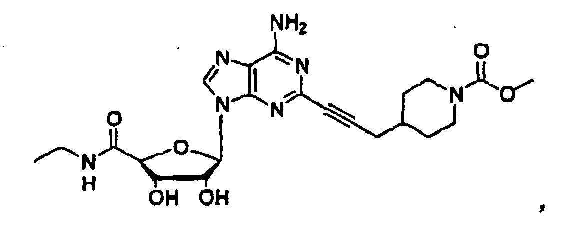 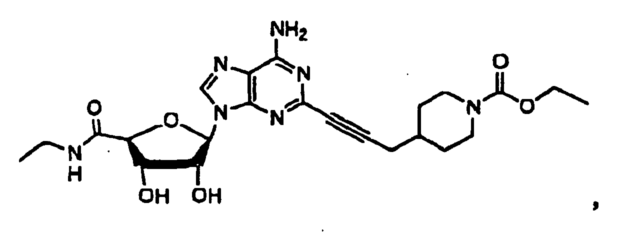 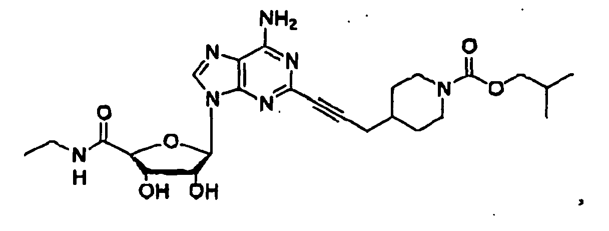 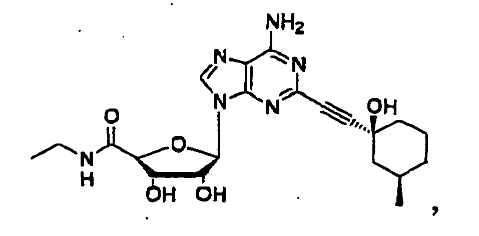 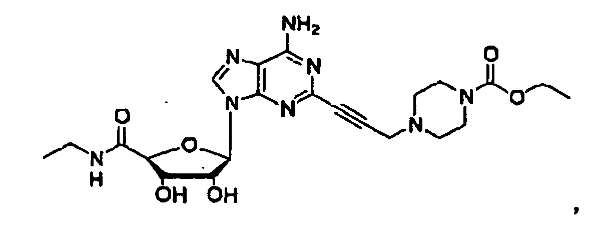 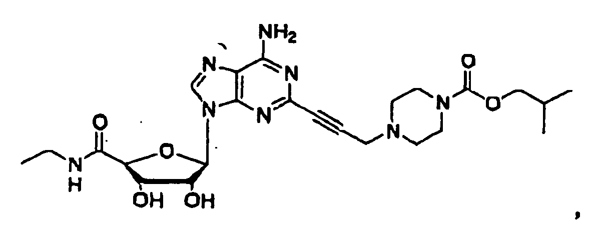 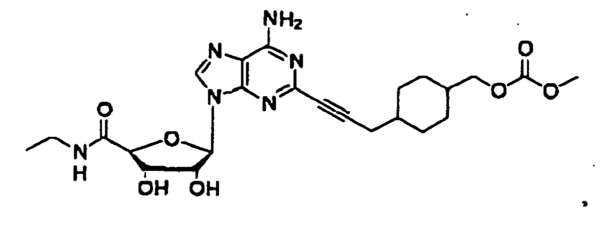 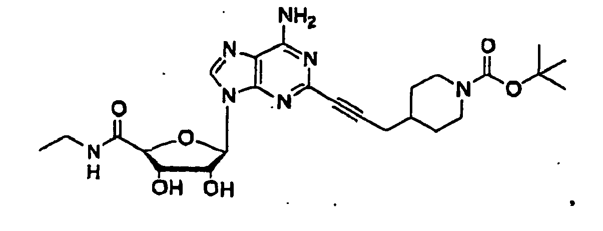 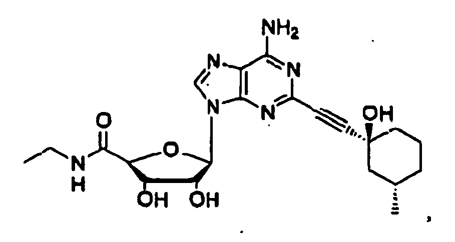 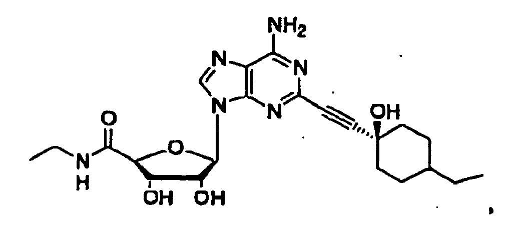 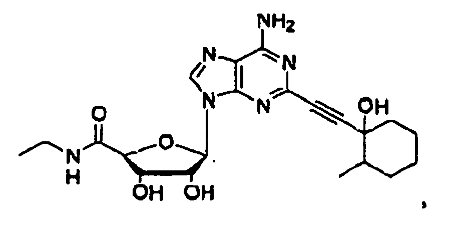 or

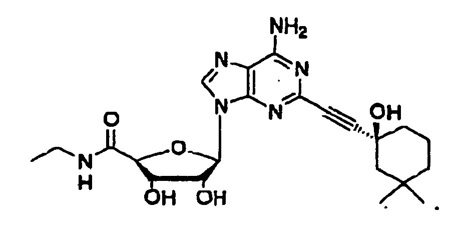

55. Use according to claim 6, wherein Z is CR³R⁴R⁵; each R¹, R² and R³ is hydrogen; R⁴ and R⁵ together with the carbon atom to which they are attached form a cycloalkyl ring having 3, 4, 5, 6, 7, 8, 9 or 10 ring atoms; and
wherein the ring comprising R⁴ and R⁵ is substituted with - (CH₂)₀₋₆-Y; where Y is -CH₂OR^{a}, -CO₂R^{a}, -OC(O)R^{a}, -CH₂OC(O)R^{a}, - C(O)NR^{b}R^{c}, -CH₂SR^{a}, -C(S)OR^{a}, -OC(S)R^{a}, -CH₂OC(S)R^{a} or C(S)NR^{b}R^{c} or -CH₂N(R^{a})(R^{b});
each R⁷ is independently hydrogen, (C₁-C₈)alkyl, (C₃-C₈)cycloalkyl, aryl or aryl(C₁-C₈)alkylene;
X is -CH₂OR^{a}, -CO₂R^{a}, -OC(O)R^{a}, -CH₂OC(O)R^{a}, -C(O)NR^{b}R^{c}, -CH₂SR^{a}, -C(S)OR^{a}, -OC(S)R^{a}, -CH₂OC(S)R^{a} or C(S)NR^{b}R^{c} or -CH₂N(R^{b})(R^{c});
each R^{a}, R^{b} and R^{c} is independently hydrogen, (C₁-C₈)alkyl, or (C₁-C₈)alkyl substituted with 1-3 (C₁-C₈)alkoxy, (C₃-C₈)cycloalkyl, (C₁-C₈)alkylthio, amino acid, aryl, aryl(C₁-C₈)alkylene, heteroaryl, or heteroaryl(C₁-C₈)alkylene; or R^{b} and R^{c}, together with the nitrogen to which they are attached, form a pyrrolidino, piperidino, morpholino, or thiomorpholino ring; and
m is 0 to 6;
or a pharmaceutically acceptable salt thereof

56. Use according to claim 6, wherein the A_{2A} adenosine receptor agonist is ATL-146e, AB-1, AB-3 or JR-3213.

57. Use according to claim 6, wherein the A_{2A} adenosine receptor agonist is ATL-146e.

58. Use according to any of claims 1 to 57, in in the preparation of a medicament in which a Type IV phosphodiesterase inhibitor is further administered to the patient.

59. Use according to claim 58, wherein the Type IV phosphodiesterase inhibitor is rolipram.

60. Use according to any of claims 1 to 59, to prepare a medicament for treating inhibition of inflammation caused by pathogenic toxins.

61. A product containing an anti-pathogenic agent and an A_{2A} adenosine receptor agonist for use as a combined preparation for simultaneous or sequential administration to a patient for treating inflammation caused by pathogenic organisms.

62. A product according to claim 61 also containing a Type IV phosphodiesterase inhibitor.

## Patentansprüche

1. Verwendung eines A_{2A}-Adenosinrezeptoragonisten bei der Herstellung eines Medikaments zur Behandlung einer Entzündung, verursacht durch pathogene Organismen, wobei ein anti-pathogenes Mittel und der besagte A_{2A}-Adenosinrezeptoragonist an einen Patienten gleichzeitig oder der Reihe nach zu verabreichen sind.

2. Verwendung nach Anspruch 1 bei der Herstellung eines Medikaments zur Behandlung einer Entzündung, verursacht durch einen viralen Organismus.

3. Verwendung nach Anspruch 1, worin das Pathogen ein Bakterium und das anti-pathogene Mittel ein Antibiotikum ist; oder
worin das Pathogen ein Virus und das anti-pathogene Mittel ein antivirales Mittel ist; oder
worin das Pathogen eine Hefe oder ein Pilz ist und das anti-pathogene Mittel ein Antipilzmittel ist.

4. Verwendung nach Anspruch 3, worin die Entzündung durch E. coli verursacht ist.

5. Verwendung nach Anspruch 3, worin die Bakterien ein hämolytischurämisches Syndrom verursachen.

6. Verwendung nach einem der Ansprüche 1 bis 5, worin der A_{2A}-Adenosinrezeptoragonist eine Verbindung ist, die die Formel (I) hat: 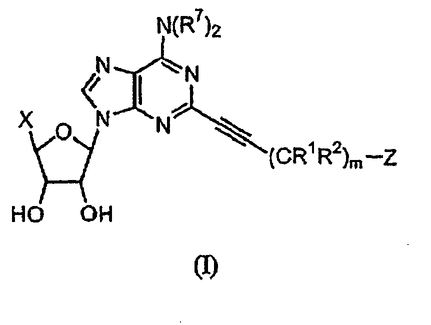 worin
Z CR³R⁴R⁵ oder NR⁴R⁵ ist;
jeder R¹ unabhängig Wasserstoff, Halo, -OR^{a}, -SR^{a}, (C₁-C₈)Alkyl, Cyano, Nitro, Trifluormethyl, Trifluormethoxy, (C₃-C₈)Zykloalkyl, Heterozyklus, Heterozyklus(C₁-C₈)alkylen-, Aryl, Aryl(C₁-C₈)alkylen-, Heteroaryl, Heteroaryl(C₁-C₈)alkylen-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, R^{a}S(=O)₂- oder -N=NR^{b} ist;
jeder R² unabhängig Wasserstoff, Halo, (C₁-C₈)Alkyl, (C₃-C₈)Zykloalkyl, Heterozyklus, Heterozyklus(C₁-C₈)alkylen-, Aryl, Aryl(C₁-C₈)alkylen-, Heteroaryl oder Heteroaryl(C₁-C₈)alkylen- ist; oder
R¹ und R² und das Atom, an welches sie gebunden sind, C=O, C=S oder C=NR^{d} sind;
R⁴ und R⁵ zusammen mit den Atomen, an welche sie gebunden sind, ein gesättigten oder teilweise ungesättigten, mono-, bizyklisch- oder aromatischen Ring bilden, der 3, 4, 5, 6, 7, 8, 9 oder 10 Ringatome hat, optional umfassend 1, 2, 3 oder 4 Heteroatome, ausgewählt aus nicht-peroxid Oxy (-O-), Thio (-S-), Sulfinyl (-SO-), Sulfonyl (-S(O)₂-) oder Amin (-NR^{b}-) in dem Ring;
worin irgendein Ring, der R⁴ und R⁵ umfasst, mit 1 bis 14 R⁶-Gruppen substituiert ist; worin jeder R⁶ unabhängig Wasserstoff, Halo, -OR^{a}, -SR^{a}, (C₁-C₈)Alkyl, Cyano, Nitro, Trifluormethyl, Trifluormethoxy, (C₁-C₈)Zykloalkylen-, (C₆-C₁₂)Bizykloalkyl, Heterozyklus oder Heterozyklus(C₁-C₈)alkylen-, Aryl, Aryl(C₁-C₈)alkylen-, Heteroaryl, Heteroaryl(C₁-C₈)alkylen-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, -NNR^{b} ist oder zwei R⁶-Gruppen und das Atom, an welches sie gebunden sind, C=O, C=S ist oder; zwei R⁶-Gruppen, zusammen mit dem Atom oder den Atomen, an welche sie gebunden sind, einen carbozyklischen oder heterozyklischen Ring bilden können;
R³ Wasserstoff, Halo, -OR^{a}, -SR^{a}, (C₁-C₈)Alkyl, Cyano, Nitro, Trifluormethyl, Trifluormethoxy, (C₃-C₈)Zylcloalkyl, Heterozyklus, Heterozyklus(C₁-C₈)alkylen-, Aryl, Aryl(C₁-C₈)alkylen-, Heteroaryl, Heteroaryl(C₁-C₈)alkylen-, -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO3R^{a}, R^{a}OC(=S)-, R^{a}C(=S)-,
-SSR^{a}, R^{a}S(=O)-, R^{a}S(=O)₂-, -NNR^{b} ist; oder, wenn der Ring, der von CR⁴R⁵ gebildet wird, ein Aryl oder ein Heteroaryl oder teilweise ungesättigt ist, dann kann R³ fehlen;
jeder R⁷ unabhängig Wasserstoff, (C₁-C₈)Alkyl, (C₃-C₈)Zylcloalkyl, Aryl oder Aryl(C₁-C₈)alkylen-, Heteroaryl, Heteroaryl(C₁-C₈)alkylen- ist;
X -CH₂OR^{a}, -CO₂R^{a}, -OC(O)R⁸, -CH₂OC(O)R^{a}, -C(O)NR^{b}R^{c}, -CH₂SR^{a}, -C(S)OR^{a}, -OC(S)R^{a}, -CH₂OC(S)R^{a} oder -C(S)NR^{b}R^{c} oder -CH₂N(R^{b})(R^{c}) ist;
worin irgendeiner aus Alkyl, Zykloalkyl, Heterozyklus, Aryl oder Heteroarylgruppen von R¹, R², R³, R⁶ und R⁷ optional an einem Kohlenstoff mit ein oder mehreren (z.B. 1, 2, 3 oder 4) Substituenten substituiert ist, ausgewählt aus der Gruppe, bestehend aus Halo, -OR^{a}, -SR^{a}, (C₁-C₈)Alkyl, Cyano, Nitro, Trifluormethyl, Trifluormethoxy, (C₃-C₈)Zykloalkyl, (C₆-C₁₂)Bizykloalkyl, Heterozyklus oder Heterozyklus(C₁-C₈)alkylen-, Aryl, Aryloxy, Aryl(C₁-C₈)alkylen-, Heteroaryl, Heteroaryl(C₁-C₈)alkylen-, -CO₂R^{a}, R^{a}C(=O)O-, R^{a}C(=O)-, -OCO₂R^{a}, R^{b}R^{c}NC(=O)O-, R^{a}OC(-O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(-S)N(R^{b})-, -OPO₃R^{a}, R^{a}OC(=S)-, R^{a}C(=S)-, -SSR^{a}, R^{a}S(=O)-, R^{a}S(=O)ₚ-, R^{b}R^{c}NS(O)ₚ- und -N=NR^{b};
worin irgendein (C₁-C₈)Alkyl, (C₃-C₈)Zykloalkyl, (C₆-C₁₂)Bizykloalkyl, (C₁-C₈)Alkoxy, (C₁-C₈)Alkanoyl, (C₁-C₈)Alkylen oder Heterozyklus optional teilweise ungesättigt ist;
jeder R^{a}, R^{b} und R^{c} unabhängig Wasserstoff, (C₁-C₈)Alkyl oder (C₁-C₈)Alkyl ist, substituiert mit 1-3 (C₁-C₈)Alkoxy, (C₃-C₈)Zykloalkyl, (C₁-C₈)Alkylthio, Aminosäure, Aryl, Aryl(C₁-C₈)alkylen, Heteroaryl oder Heteroaryl(C₁-C₈)alkylen; oder R^{b} und R^{c} zusammen mit dem Stickstoff, an welchen sie gebunden sind, einen Pyrrolidin-, Piperidin-, Morpholin- oder Thiomorpholinring bilden; und
R^{d} Wasserstoff oder (C₁-C₆)Alkyl ist;
m 0 bis 8 und p 0 bis 2 ist;
oder ein pharmazeutisch akzeptables Salz davon.

7. Verwendung nach Anspruch 6, worin R¹ Wasserstoff, -OH, -CH₂OH, -OMe, -OAc, -NH₂, -NHMe, -NMe₂ oder -NHAc ist.

8. Verwendung nach Anspruch 6, worin R¹ Wasserstoff, -OH, -OMe, -OAc, -NH₂, -NHMe, -NMe₂ oder -NHAc ist.

9. Verwendung nach Anspruch 6, worin R¹ Wasserstoff, OH, OMe, oder NH₂ ist.

10. Verwendung nach Anspruch 6, worin R¹ Wasserstoff, OH oder NH₂ ist.

11. Verwendung nach Anspruch 6, worin R¹ Wasserstoff oder OH ist.

12. Verwendung nach einem der Ansprüche 6-11, worin R² Wasserstoff, (C₁-C₈)Alkyl, Zyklopropyl, Zyklohexyl oder Benzyl ist.

13. Verwendung nach einem der Ansprüche 6-11, worin R² Wasserstoff, Methyl, Ethyl oder Propyl ist.

14. Verwendung nach einem der Ansprüche 6-11, worin R² Wasserstoff, oder Methyl ist;

15. Verwendung nach einem der Ansprüche 6-11, worin R² Wasserstoff ist.

16. Verwendung nach Anspruch 6, worin R¹, R² und das Kohlenstoffatom, an welches sie gebunden sind, Carbonyl (C=O) ist.

17. Verwendung nach einem der Ansprüche 6 bis 16, worin R³ Wasserstoff, OH, OMe, OAc, NH₂, NHMe, NMe₂ oder NHAc ist.

18. Verwendung nach einem der Ansprüche 6 bis 16, worin R³ Wasserstoff, OH, OMe oder NH₂ ist.

19. Verwendung nach einem der Ansprüche 6 bis 16, worin R³ Wasserstoff, OH oder NH₂ ist.

20. Verwendung nach einem der Ansprüche 6 bis 16, worin R³ Wasserstoff oder OH ist.

21. Verwendung nach einem der Ansprüche 6 bis 20, worin der Ring, der R⁴, R⁵ und das Atom, mit welchem sie verbunden sind, umfasst, Zyklopentan, Zyklohexan, Piperidin, Dihydropyridin, Tetrahydropyridin, Pyridin, Piperazin, Decalin, Tetrahydropyrazin, Dihydropyrazin, Pyrazin, Dihydropyrimidin, Tetrahydropyrimidin, Hexahydropyrimidin, Pyrazin, Imidazol, Dihydroimidazol, Imidazolidin, Pyrazol, Dihydropyrazol und Pyrazolidin ist.

22. Verwendung nach einem der Ansprüche 6 bis 20, worin der der Ring, der R⁴, R⁵ und das Atom, mit welchem sie verbunden sind, umfasst, Zyklopentan, Zyklohexan, Piperidin, Dihydropyridin, Tetrahydropyridin, Pyridin, Piperazin, Tetrahydropyrazin, Dihydropyrazin, Pyrazin, Dihydropyrimidin, Tetrahydropyrimidin, Hexahydropyrimidin, Pyrazin, Imidazol, Dihydroimidazol, Imidazolidin, Pyrazol, Dihydropyrazol und Pyrazolidin ist.

23. Verwendung nach einem der Ansprüche 6 bis 20, worin der der Ring, der R⁴, R⁵ und das Atom, mit welchem sie verbunden sind, umfasst, Zyklohexan, Piperidin oder Piperazin ist.

24. Verwendung nach einem der Ansprüche 6 bis 23, worin R⁶ (C₁-C₈)Alkyl ist oder mit (C₁-C₈)Alkyl, -OR^{a}, -CO₂R^{a}, R^{a}C(=O)-, R^{a}C(=O)O-, R^{b}R^{b}N-, R^{b}R^{c}NC(=O)- oder Aryl substituiert ist.

25. Verwendung nach einem der Ansprüche 6 bis 23, worin R⁶ (C₁-C₈)Alkyl, -OR^{a}_{,} -CO₂R^{a}, R^{a}C(=O)-, R^{a}C(=O)O-, R^{b}R^{c}N-, R^{b}R^{c}NC(=O)- oder Aryl ist.

26. Verwendung nach einem der Ansprüche 6 bis 23, worin R⁶ Methyl, Ethyl, Buthyl, OH, OR^{a}, -CO₂R^{a}, R^{a}C(=O)-, OC(=O)CH₂CH₃, -CONR^{b}R^{c}, NR^{b}R^{c} oder Phenyl ist.

27. Verwendung nach einem der Ansprüche 6 bis 23, worin R⁶ OH, OMe, Methyl, Ethyl, t-Buthyl, -CO₂R^{a}, -CONR^{b}R^{c}, OAc, NH₂, NHMe, NMe₂, NHEt oder N(Et)₂ ist.

28. Verwendung nach einem der Ansprüche 6 bis 23, worin R⁶ Methyl, Ethyl, t-Buthyl, Phenyl, -CO₂R^{a}, -CONR^{b}R^{c} oder -(=O)CR^{a} ist.

29. Verwendung nach einem der Ansprüche 6 bis 23, worin R⁶ Methyl, Ethyl, -CO₂R^{a}, -CONR^{b}R^{c} oder OAc ist.

30. Verwendung nach einem der Ansprüche 6 bis 23, worin R⁶ -(CH₂)₁₋₂OR^{a}, -(CH₂)₁₋₂C(=O)OR^{a}, -(CH₂)₁₋₂OC(=O)OR^{a}, -(CH₂)₁₋₂C(=O)R^{a}, -(CH₂)₁₋₂OCO₂R^{a}, -(CH₂)₁₋₂NHR^{a}, -(CH₂)₁₋₂NR^{b}R^{c}, -(CH₂)₁₋₂OC(=O)NHR^{a}, oder -(CH₂)₁₋₂OC(=O)NR^{b}R^{c} ist.

31. Verwendung nach einem der Ansprüche 6 bis 23, worin R⁶ -CH₂OH, -CH₂OAc, -CH₂CH₃, -CH₂C(=O)OCH₃, -CH₂OC(=O)CH₃, -CH₂C(=O)CH₃, -CH₂OCO₂CH₃, -CH₂NH(CH₃) oder-(CH₂)₁₋₂N(CH₃)₂ ist.

32. Verwendung nach einem der Ansprüche 6 bis 23, worin R⁶ -CH₂OH, -CH₂OAc, -C(=O)OCH₃, -C(=O)CH₃, OCO₂CH₃ -OCO₂CH₃, -CH₂NH(CH₃) oder -(CH₂)₁₋₂N(CH₃)₂ ist.

33. Verwendung nach einem der Ansprüche 6 bis 32, worin die Zahl der R⁶-Gruppen, die an den R⁴R⁵-Ring substituiert sind, 1 bis 4 ist.

34. Verwendung nach einem der Ansprüche 6 bis 33, worin R^{a} und R^{b} unabhängig Wasserstoff, (C₁-C₄)Alkyl, Aryl oder Aryl(C₁-C₈)alkylen sind.

35. Verwendung nach einem der Ansprüche 6 bis 33, worin R^{a} und R^{b} unabhängig Wasserstoff, Methyl oder Ethyl, Phenyl oder Benzyl sind.

36. Verwendung nach einem der Ansprüche 6 bis 35, worin R^{a} (C₁-C₈)Alkyl ist.

37. Verwendung nach einem der Ansprüche 6 bis 33, worin R^{a} und R^{b} unabhängig Methyl, Ethyl, Propyl oder Butyl sind.

38. Verwendung nach einem der Ansprüche 6 bis 33, worin R^{a} und R^{b} unabhängig Methyl, Ethyl, i-Propyl, i-Butyl oder tert-Butyl sind.

39. Verwendung nach einem der Ansprüche 6 bis 38, worin R^{b} und R^{c} und das Atom, an welches sie gebunden sind, einen Ring bilden.

40. Verwendung nach einem der Ansprüche 6 bis 39, worin R⁷ Wasserstoff, Alkyl, Aryl oder Aryl(C₁-C₈)alkylen ist.

41. Verwendung nach einem der Ansprüche 6 bis 39, worin R⁷ Wasserstoff, Methyl oder Ethyl, Phenyl oder Benzyl ist.

42. Verwendung nach einem der Ansprüche 6 bis 39, worin R⁷ H oder Methyl ist.

43. Verwendung nach einem der Ansprüche 6 bis 42, worin N(R⁷)₂ Amin, Methylamin, Dimethylamin; Ethylamin; Pentylamin, Diphenylethylamin, Pyridylmethylamin, Diethylamin oder Benzylamin ist.

44. Verwendung nach einem der Ansprüche 6 bis 42, worin N(R⁷)₂ Amin, Methylamin, Dimethylamin; Ethylamin; Diethylamin oder Benzylamin ist.

45. Verwendung nach einem der Ansprüche 6 bis 42, worin N(R⁷)₂ Amin oder Methylamin ist.

46. Verwendung nach einem der Ansprüche 6 bis 45, worin X -CH₂OR^{a}, -CO₂R^{a}, -OC(O)R^{a}, -CH₂OC(O)R^{a}, -C(O)NR^{b}R^{c} ist.

47. Verwendung nach einem der Ansprüche 6 bis 45, worin X -CH₂OR^{a} oder -C(O)NR^{b}R^{c} ist.

48. Verwendung nach einem der Ansprüche 6 bis 45, worin X -CH₂OH oder -C(O)NHCH₂CH₃ ist.

49. Verwendung nach einem der Ansprüche 6 bis 19, worin m 0, 1 oder 2 ist.

50. Verwendung nach einem der Ansprüche 6 bis 49, worin die Ringe, die R⁴, R⁵ und das Atom, mit welchem sie verbunden sind, umfassen, ausgewählt sind aus der Gruppe, bestehend aus dem Folgenden, in welchem q 0-14 ist:

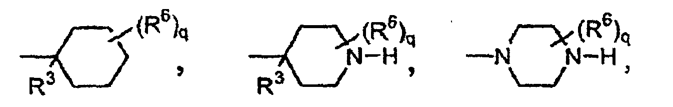

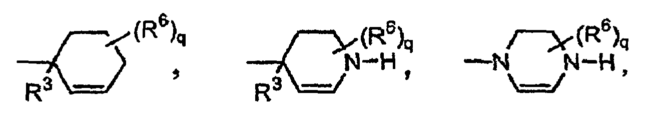

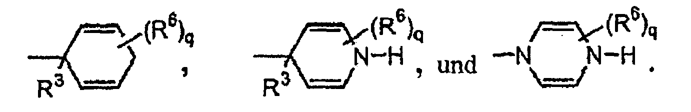

51. Verwendung nach einem der Ansprüche 6 bis 49, worin die Ringe, die R⁴, R⁵ und das Atom, mit welchem sie verbunden sind, umfassen, ausgewählt sind aus der Gruppe, bestehend aus:

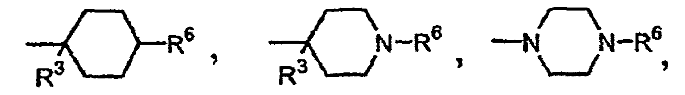

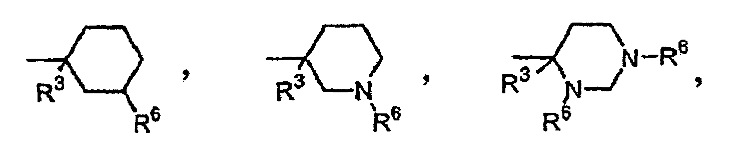

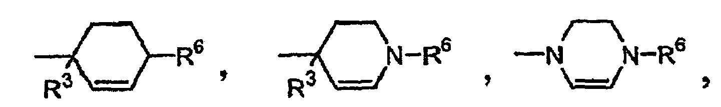

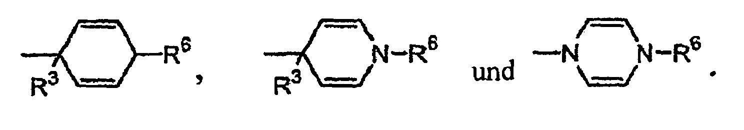

52. Verwendung nach einem der Ansprüche 6 bis 51, worin der Ring, umfassend R⁴ und R⁵, 2-Methylzyklohexan, 2,2-Dimethylzyklohexan, 2-Phenylzyklohexan, 2-Ethylzyklohexan, 2,2-Diethylzyklohexan, 2-tert-Butylzyklohexan, 3-Methylzyklohexan, 3,3-Dimethylzyklohexan, 4-Methylzyklohexan, 4-Ethylzyklohexan, 4-Phenylzyklohexan, 4-tert-Butylzyklohexan, 4-Carboxymethylzyklohexan, 4-Carboxyethylzyklohexan, 3,3,5,5-Tetramethylzyklohexan, 2,4-Dimethylzyklopentan, 4-Zyklohexancarboxylsäure, 4-Zyklohexancarboxylsäureester oder 4-Methyloxyalkanoylzyklohexan ist.

53. Verwendung nach einem der Ansprüche 6 bis 51, worin der Ring, umfassend R⁴ und R⁵, 4-Piperidin, 4-Piperidin 1-carboxylsäure, 4-Piperidin-1-carboxylsäuremethylester, 4-Piperidin-1-carboxylsäureethylester, 4-Piperidin-1-carboxylsäurepropylester, 4-Piperidin-1-carboxylsäure-tert-butylester, 1-Piperidin, 1-Piperidin-4-carboxylsäuremethylester, 1-Piperidin-4-carboxylsäureethylester, 1-Piperidin-4-carboxylsäurepropylester, 1-Piperidin-4-caboxylsäure-tert-butylester, 1-Piperidin-4-carboxylsäuremethylester, 3-Piperidin, 3-Piperidin -1-carboxylsäure, 3-Piperidin-1-carboxylsäuremethylester, 3-Piperidin-1-carboxylsäure-tert-butylester, 1,4-Piperazin, 4-Piperazin-1-carboxylsäure, 4-Piperazin-1-carboxylsäuremethylester, 4-Piperazin-1 -carboxylsäureethylester, 4-Piperazin-1-carboxylsäurepropylester, 4-Piperazine-1-carboxylsäue-tert-butylester, 1,3-Piperazin, 3-Piperazin-1-carboxylsäure, 3-Piperazin-1-carboxylsäuremethylester, 3-Piperazin-1-carboxylsäureethylester, 3-Piperazin-1-carboxylsäurepropylester, 3-Piperidin-1-carboxylsäure-tert-butylester, 1-Piperidin-3-carboxylsäuremethylester, 1-Piperidin-4-carboxylsäureethylester, 1-Piperidin-3-carboxylsäurepropylester oder 1-Piperidin-3-carboxylsäure-tert-butylester.

54. Verwendung nach Anspruch 6, worin der A_{2A}-Adenosinrezeptoragonist eine Verbindung ist, die die Formel hat, ausgewählt aus dem Folgenden:

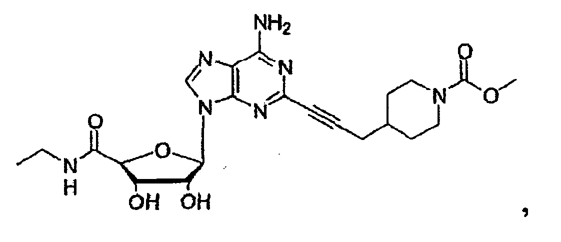

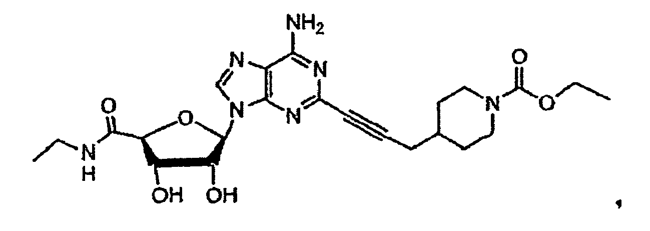

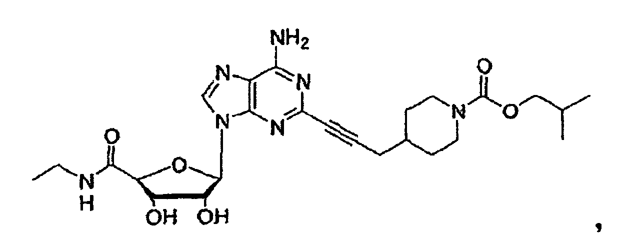

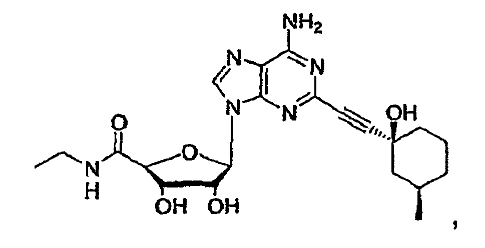

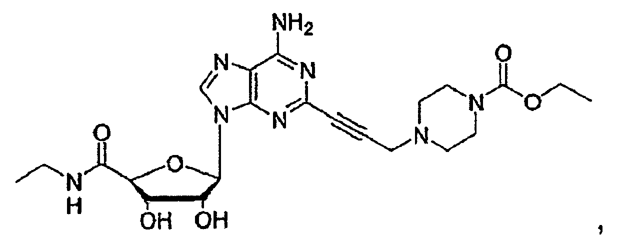

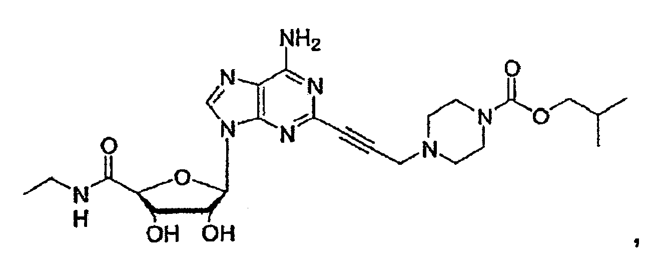

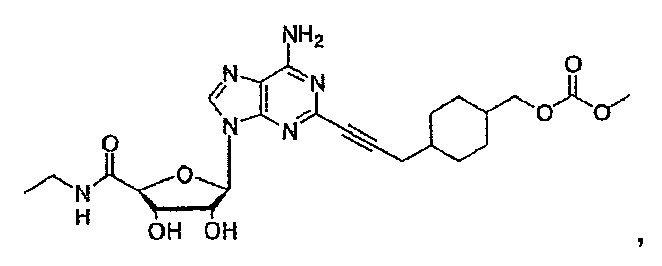

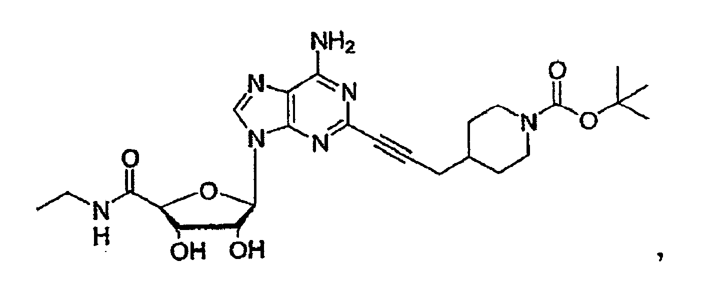

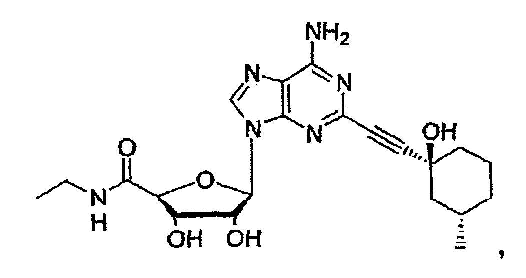

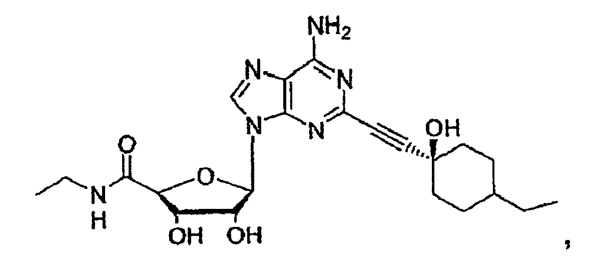

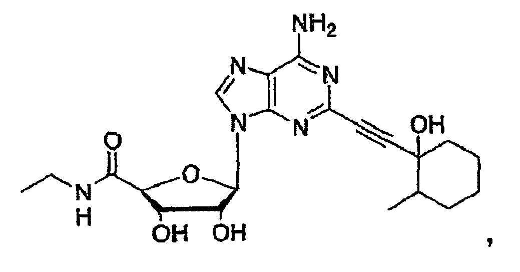

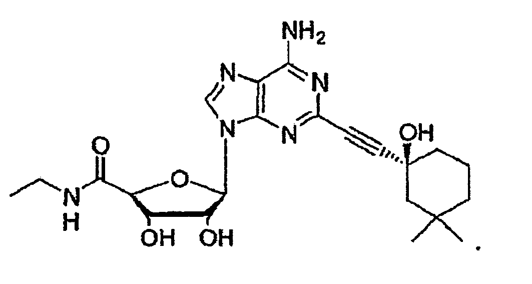

55. Verwendung nach Anspruch 6, worin Z CR³R⁴R⁵ ist; jeder R¹, R² und R³ Wasserstoff ist; R⁴ und R⁵ zusammen mit dem Kohlenstoffatom, an welches sie gebunden sind, einen Zykloalkylring bilden, der 3, 4, 5, 6, 7, 8, 9 oder 10 Ringatome hat; und
worin der Ring, der R⁴ und R⁵ umfasst, mit -(CH₂)₀₋₆- Y substituiert ist; worin Y -CH₂OR^{a}, -CO₂R^{a}, -OC(O)R^{a}, -CH₂OC(O)R^{a}, -C(O)NR^{b}R^{c}, -CH₂SR^{a}, -C(S)OR^{a}, -OC(S)R^{a}, -CH₂OC(S)R^{a} oder C(S)NR^{b}R^{c} oder -CH₂N(R^{a})(R^{b}) ist;
jeder R⁷ unabhängig Wasserstoff, (C₁-C₈)Alkyl, (C₃-C₈)Zykloalkyl, Aryl oder Aryl(C₁-C₈)alkylen ist;
X -CH₂OR^{a}, -CO₂R^{a}, -OR(O)R^{a}, -CH₂OC(O)R^{a}, -C(O)NR^{b}R^{c}, -CH₂SR^{a}, -C(S)OR^{a}, -OC(S)R^{a}, -CH₂OC(S)R^{a} oder C(S)NR^{b}R^{c} oder -CH₂N(R^{b})(R^{c}) ist;
jeder R^{a}, R^{b} und R^{c} unabhängig Wasserstoff, (C₁-C₈)Alkyl oder (C₁-C₈)Alkyl ist, substituiert mit 1-3 (C₁-C₈)Alkoxy, (C₃-C₈)Zykloalkyl, (C₁-C₈)Alkylthio, Aminosäure, Aryl, Aryl(C₁-C₈)alkylen, Heteroaryl oder Heteroaryl(C₁-C₈)alkylen; oder R^{b} und R^{c} zusammen mit dem Stickstoff, an welchen sie gebunden sind, einen Pyrrolidin-, Piperidin-, Morpholin- oder Thiomorpholinring bilden; und
m 0 bis 6 ist;
oder ein pharmazeutisch akzeptables Salz davon.

56. Verwendung nach Anspruch 6, worin der A_{2A}-Adenosinrezeptoragonist ATL-146e, AB-1, AB-3 oder JR-3213 ist.

57. Verwendung nach Anspruch 6, worin der A_{2A}-Adenosinrezeptoragonist ATL-146e ist.

58. Verwendung nach einem der Ansprüche 1 bis 57 bei der Herstellung eines Medikaments, in welchem ferner ein Typ IV-Phosphodiesteraseinhibitor an den Patienten verabreicht wird.

59. Verwendung nach Anspruch 58, worin der Typ IV-Phosphodiesteraseinhibitor Rolipram ist.

60. Verwendung nach einem der Ansprüche 1 bis 59, um ein Medikament für die Behandlung einer Entzündungshemmung herzustellen, verursacht durch pathogene Toxine.

61. Produkt, das ein anti-pathogenes Mittel und einen A_{2A}-Adenosinrezeptoragonisten für die Verwendung als ein kombiniertes Präparat für eine gleichzeitige Verabreichung oder eine Verabreichung der Reihe nach an einen Patienten zur Behandlung einer Entzündung, verursacht durch pathogene Organismen, enthält.

62. Produkt nach Anspruch 61, das auch einen Typ IV-Phosphodiesteraseinhibitor enthält.

## Revendications

1. Utilisation d'un agoniste du récepteur A_{2A} de l'adénosine pour la préparation d'un médicament destiné à traiter une inflammation provoquée par des organismes pathogènes, dans laquelle un agent anti-pathogène et ledit agoniste du récepteur A_{2A} de l'adénosine doivent être administrés à un patient simultanément ou séquentiellement.

2. Utilisation selon la revendication 1 pour la préparation d'un médicament destiné à traiter une inflammation provoquée par un organisme viral.

3. Utilisation selon la revendication 1, dans laquelle l'agent pathogène est une bactérie et l'agent anti-pathogène est un antibiotique ; ou
dans laquelle l'agent pathogène est un virus et l'agent anti-pathogène est un agent antiviral ; ou
dans laquelle l'agent pathogène est une levure ou un champignon et l'agent anti-pathogène est un agent antifongique.

4. Utilisation selon la revendication 3, dans laquelle l'inflammation est provoquée par *E.Coli.*

5. Utilisation selon la revendication 3, dans laquelle la bactérie est à l'origine du syndrome hémolytique et urémique.

6. Utilisation selon l'une quelconque des revendications 1 à 5, dans laquelle l'agoniste du récepteur A_{2A} de l'adénosine est un composé de formule (I) : 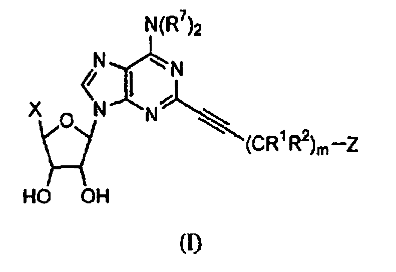 dans laquelle
Z représente CR³R⁴R⁵ ou NR⁴R⁵ ;
chaque R¹ représente, indépendamment l'un de l'autre, un atome d'hydrogène, d'halogène, un groupe -OR^{a}, -SR^{a}, alkyle en C₁ à C₈, cyano, nitro, trifluorométhyle, trifluorométhoxy, cycloalkyle en C₃ à Cg, un hétérocycle, un hétérocycle-(alkylène en C₁ à C₃), aryle, aryl-(alkylène en C₁ à C₈), hétéroaryle, hétéroaryl-(alkylène en C₁ à C₈), -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S) -, -SSR^{a}, R^{a}S(=O)-, R^{a}S(=O)₂- ou -N=NR^{b} ;
chaque R² représente, indépendamment l'un de l'autre, un atome d'hydrogène, d'halogène, un groupe alkyle en C₁ à C₈, cycloalkyle en C₃ à C₈, un hétérocycle, un hétérocycle-(alkylène en C₁ à C₈), aryle, aryl-(alkylène en C₁ à C₈), hétéroaryle ou hétéroaryl-(alkylène en C₁ à C₈) ; ou
R¹ et R², ainsi que l'atome auquel ils sont liés, représentent C=O, C=S ou C=NR^{d} ;
R⁴ et R⁵, conjointement avec les atomes auxquels ils sont liés, forment un cycle mono-, bi-cyclique saturé ou partiellement insaturé ou un noyau aromatique ayant 3, 4, 5, 6, 7, 8, 9 ou 10 atomes cycliques comprenant éventuellement 1, 2, 3 ou 4 hétéroatomes choisis parmi les groupes oxy non peroxyde (-O-), thio (-S-), sulfinyle (-SO-), sulfonyle (-S(O)₂-) ou amine (-NR^{b}-) dans le cycle ;
dans laquelle tout cycle comprenant R⁴ et R⁵ est substitué par 1 à 14 groupes R⁶ ; dans laquelle chaque R⁶ représente, indépendamment l'un de l'autre, un atome d'hydrogène, d'halogène, un groupe -OR^{a}, -SR^{a}, alkyle en C₁ à Cg, cyano, nitro, trifluorométhyle, trifluorométhoxy, cycloalkyle en C₁ à C₈, bicycloalkyle en C₆ à C₁₂, hétérocycle ou hétérocycle-(alkylène en C₁ à C₈), aryle, aryl-(alkylène en C₁ à C₈), hétéroaryle, hétéroaryl-(alkylène en C₁ à C₈), -CO₂R^{a}, R^{a}C(=O)O-, R^{a}C(=0)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-, -SSR^{a}, R^{a}S(=O)-, -NNR^{b}, ou bien deux groupes R⁶, ainsi que l'atome auquel ils sont liés, représentent C=O, C=S ; ou deux groupes R⁶, conjointement avec le ou les atomes auxquels ils sont liés, peuvent former un carbocycle ou un hétérocycle ;
R³ représente un atome d'hydrogène, un groupe halogéno, -OR^{a}, -SR^{a}, alkyle en C₁ à C₈, cyano, nitro, trifluorométhyle, trifluorométhoxy, cycloalkyle en C₃ à C₈, un hétérocycle, un hétérocycle-(alkylène en C₁ à Cg), aryle, aryl-(alkylène en C₁ à C₈), hétéroaryle, hétéroaryle-(alkylène en C₁ à C₈), -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O) N (R^{b})-, R^{b}R^{c}NC(=O)N(R^{b})-, R^{b}R^{c}NC(=S)N(R^{b})-, -OPO₃R^{a}, R^{a}OC(=S)-, R^{a}C(=S)-. -SSR^{a}, R^{a}S(=O)-, R^{a}S(=O)₂-, -NNR^{b}; ou si le cycle formé à partir de CR⁴R⁵ représente un cycle aryle ou hétéroaryle ou est partiellement insaturé, alors R³ peut être absent ;
chaque R⁷ représente, indépendamment l'un de l'autre, un atome d'hydrogène, un groupe alkyle en C₁ à C₈, cycloalkyle en C₃ à C₈, aryle ou aryl-(alkylène en C₁ à C₈), hétéroaryle, hétéroaryl-(alkylène en C₁ à C₈) ;
X représente -CH₂OR^{a}, -CO₂R^{a}, -OC (O) R^{a}, -CH₂OC(O)R^{a}, -C(O)NR^{b}R^{c}, -CH₂SR^{a}, -C(S)OR^{a}, -OC(S)R^{a}, -CH₂OC(S)R^{a} ou -C (S) NR^{b}R^{c} ou -CH₂N(R^{b})(R^{c}) ;
dans laquelle l'un quelconque des groupes alkyle, cycloalkyle, hétérocycle, aryle ou hétéroaryle de R¹, R², R³, R⁶ et ⁷ est éventuellement substitué sur le carbone avec un ou plusieurs (par exemple 1, 2, 3 ou 4) substituants choisis dans le groupe constitué par un groupe halogéno, -OR^{a}, -SR^{a}, alkyle en C₁ à C₈, cyano, nitro, trifluorométhyle, trifluorométhoxy, cycloalkyle en C₃ à C₈, bicycloalkyle en C₆ à C₁₂, hétérocycle ou hétérocycle-(alkylène en C₁ à C₈), aryle, aryloxy, aryl-(alkylène en C₁ à C₈), hétéroaryle, hétéroaryl-(alkylène cn C₁ à C₈), -CO₂R^{a}, R^{a}C(=O)O-, R^{a}C(=O)-, -OCO₂R^{a}, R^{b}R^{c}NC(=O)O-, R^{a}OC(=O)N(R^{b})-, R^{b}R^{c}N-, R^{b}R^{c}NC(=O)-, R^{a}C(=O)N(R^{b})-, R^{b}R^{c}NC(=O)N(R^{b})-, R^{b}R^{c}NC(-S)N(R^{b})-, -OPO₃R^{a}, R^{a}OC(=S)-, R^{a}C(=S)-, -SSR^{a}, R^{a}S(=O)ₚ-, R^{b}R^{c}NS(O)ₚ-, et -N=NR^{b};
dans laquelle tout groupe alkyle en C₁ à C₈, cycloalkyle en C₃ à C₈, bicycloalkyle en C₆ à C₁₂, alcoxy en C₁ à C₈, alcanoyle en C₁ à C₈, alkylène en C₁ à C₈ ou hétérocycle, est éventuellement partiellement insaturé ;
chaque R^{a}, R^{b} et R^{c} représentent, indépendamment l'un de l'autre, un atome d'hydrogène, un groupe alkyle en C₁ à C₈ ou alkyle en C₁ à C₈ substitué par 1 à 3 groupes alcoxy en C₁ à C₈, cycloalkyle en C₃ à C₈, (alkyle en C₁ à C₈)-thio, acide aminé, aryle, aryl-(alkylène en C₁ à C₈), hétéroaryle ou hétéroaryl-(alkylène en C₁ à C₈) ; ou R^{b} et R^{c}, conjointement avec l'azote auquel ils sont liés, forment un groupe pyrrolidine, pipéridine, morpholine ou thiomorpholino ; et
R^{d} représente un atome d'hydrogène ou un groupe alkyle en C₁ à C₆ ;
m vaut de 0 à 8 et p vaut de 0 à 2 ;
ou un de leurs sels acceptables sur le plan pharmaceutique.

7. Utilisation selon la revendication 6, dans laquelle R¹ représente un atome d'hydrogène, un groupe -OH, -CH₂OH, -OMe, -OAc, -NH₂, -NHMe, -NMe₂ ou -NHAc.

8. Utilisation selon la revendication 6, dans laquelle R¹ représente un atome d'hydrogène, un groupe -OH, -OMe, -OAc, -NH₂, -NHMe, -NMe₂ ou -NHAc.

9. Utilisation selon la revendication 6, dans laquelle R¹ représente un atome d'hydrogène, un groupe OH, OMe ou NH₂.

10. Utilisation selon la revendication 6, dans laquelle R¹ représente un atome d'hydrogène, un groupe OH ou NH₂.

11. Utilisation selon la revendication 6, dans laquelle R¹ représente un atome d'hydrogène ou un groupe OH.

12. Utilisation selon l'une quelconque des revendications 6 à 11, dans laquelle R² représente un atome d'hydrogène, un groupe alkyle en C₁ à C₈, cyclopropyle, cyclohexyle ou benzyle.

13. Utilisation selon l'une quelconque des revendications 6 à 11, dans laquelle R² représente un atome d'hydrogène, un groupe méthyle, éthyle ou propyle.

14. Utilisation selon l'une quelconque des revendications 6 à 11, dans laquelle R² représente un atome d'hydrogène ou un groupe méthyle.

15. Utilisation selon l'une quelconque des revendications 6 à 11, dans laquelle R² représente un atome d'hydrogène.

16. Utilisation selon la revendication 6, dans laquelle R¹, R² et l'atome de carbone auquel ils sont liés représentent un groupe carbonyle (C=O).

17. Utilisation selon l'une quelconque des revendications 6 à 16, dans laquelle R³ représente un atome d'hydrogène, un groupe OH, OMe, OAc, NH₂, NHMe, NMe₂ ou NHAc.

18. Utilisation selon l'une quelconque des revendications 6 à 16, dans laquelle R³ représente un atome d'hydrogène, un groupe OH, OMe ou NH₂.

19. Utilisation selon l'une quelconque des revendications 6 à 16, dans laquelle R³ représente un atome d'hydrogène, un groupe OH ou NH₂.

20. Utilisation selon l'une quelconque des revendications 6 à 16, dans laquelle R³ représente un atome d'hydrogène ou un groupe OH.

21. Utilisation selon l'une quelconque des revendications 6 à 20, dans laquelle le cycle comprenant R⁴, R⁵ et l'atome auquel ils sont liés représentent le cyclopentane, le cyclohexane, la pipéridine, la dihydro-pyridine, la tétrahydro-pyridine, la pyridine, la pipérazine, la décaline, la tétrahydro-pyrazine, la dihydro-pyrazine, la pyrazine, la dihydro-pyrimidine, la tétrahydro-pyrimidine, l'hexahydro-pyrimidine, la pyrazine, l'imidazole, le dihydro-imidazole, l'imidazolidine, le pyrazole, le dihydro-pyrazole ou la pyrazolidine.

22. Utilisation selon l'une quelconque des revendications 6 à 20, dans laquelle le cycle comprenant R⁴, R⁵ et l'atome auquel ils sont liés représentent le cyclopentane, le cyclohexane, la pipéridine, la dihydro-pyridine, la tétrahydro-pyridine, la pyridine, la pipérazine, la tétrahydro-pyrasine, la dihydro-pyrazine, la pyrazine, la dihydro-pyrimidine, la tétrahydro-pyrimidine, l'hexahydro-pyrimidine, la pyrazine, l'imidazole, le dihydro-imidazole, l'imidazolidine, le pyrazole, le dihydro-pyrazole ou la pyrazolidine.

23. Utilisation selon l'une quelconque des revendications 6 à 20, dans laquelle le cycle comprenant R⁴, R⁵, ainsi que l'atome auquel ils sont liés, représentent le cyclohexane, la pipéridine ou la pipérazine.

24. Utilisation selon l'une quelconque des revendications 6 à 23, dans laquelle R⁶ représente un groupe alkyle en C₁ à C₈ ou (alkyle en C₁ à C₈)substitué, -OR^{a}, -CO₂R^{a}, R^{a}C(=O)-, R^{a}C(=O)O-, R^{b}R^{c}N-, R^{b}R^{c}NC(=O)- ou aryle.

25. Utilisation selon l'une quelconque des revendications 6 à 23, dans laquelle R⁶ représente un groupe alkyle en C₁ à C₈, -OR^{a}, -CO₂R^{a}, R^{a}C(=O)-, R^{a}C(=O)O-, R^{b}R^{c}N-, R^{b}R^{c}NC(=O)- ou aryle.

26. Utilisation selon l'une quelconque des revendications 6 à 23, dans laquelle R⁶ représente un groupe méthyle, éthyle, butyle, OH, CR^{a}, -CO₂R^{a}, R^{a}C(=O)-, OC(=O)CH₂CH₃, -CONR^{b}R^{c}, NR^{b}R^{c} ou phényle.

27. Utilisation selon l'une quelconque des revendications 6 à 23, dans laquelle R⁶ représente un groupe OH, OMe, méthyle, éthyle, t-butyle, -CO₂R^{a}, -CONR^{b}R^{c}, OAc, NH₂, NHMe, NMe₂, NHEt ou N(Et)₂.

28. Utilisation selon l'une quelconque des revendications 6 à 23, dans laquelle R⁶ représente un groupe méthyle, éthyle, t-butyle, phényle, -CO₂R^{a}-, CONR^{b}R^{c} ou -(=O)CR^{a}.

29. Utilisation selon l'une quelconque des revendications 6 à 23, dans laquelle R⁶ représente un groupe méthyle, éthyle, -CO₂R^{a}-, CONR^{b}R^{c} ou OAc.

30. Utilisation selon l'une quelconque des revendications 6 à 23, dans laquelle R⁶ représente -(CH₂)₁₋₂OR^{a}, -(CH₂)₁₋₂C(=O)OR^{a}, -(CH₂)₁₋₂OC(=O)R^{a}, -(CH₂)₁₋₂C(=O)R^{a}, -(CH₂)₁₋₂OCO₂R^{a}, -(CH₂)₁₋₂NHR^{a}, -(CH₂)₁₋₂NR^{b}R^{c}, -(CH₂)₁₋₂OC(=O)NHR^{a} ou -(CH₂)₁₋₂OC(=O)NR^{b}R^{c}.

31. Utilisation selon l'une quelconque des revendications 6 à 23, dans laquelle R⁶ représente -CH₂OH, -CH₂OAc, -CH₂OCH₃, -CH₂C(=O)OCH₃, -CH₂OC(=O)CH₃, -CH₂OC(=O)CH₃, -CH₂OCO₂CH₃, -CH₂NH(CH₃) ou -(CH₂)₁₋₂N(CH₃)₂.

32. Utilisation selon l'une quelconque des revendications 6 à 23, dans laquelle R⁶ représente -CH₂OH, -CH₂OAc, -C(=O)OCH₃, -C(=O)CH₃, OCO₂CH₃-OCO₂CH₃, -CH₂NH(CH₃) ou -(CH₂)₁₋₂N(CH₃)₂.

33. Utilisation selon l'une quelconque des revendications 6 à 33, dans laquelle le nombre de groupes R⁶ substitués sur le cycle R⁴R⁵ va de 1 à 4.

34. Utilisation selon l'une quelconque des revendications 6 à 33, dans laquelle R^{a} et R^{b} représentent chacun, indépendamment l'un de l'autre, un atome d'hydrogène, un groupe alkyle en C₁ à C₄, aryle ou aryl-(alkylène en C₁ à C₈).

35. Utilisation selon l'une quelconque des revendications 6 à 33, dans laquelle R^{a} et R^{b} représentent chacun, indépendamment l'un de l'autre, un atome d'hydrogène, un groupe méthyle ou éthyle, phényle
ou benzyle.

36. Utilisation selon l'une quelconque des revendications 6 à 35, dans laquelle R^{a} est un groupe alkyle en C₁ à C₈,

37. Utilisation selon l'une quelconque des revendications 6 à 33, dans laquelle R^{a} et R^{b} représentent chacun, indépendamment l'un de l'autre, un groupe méthyle, éthyle, propyle ou butyle.

38. Utilisation selon l'une quelconque des revendications 6 à 33, dans laquelle R^{a} et R^{b} représentent chacun, indépendamment l'un de l'autre, un groupe méthyle, éthyle, i-propyle, i-butyle ou butyle.

39. Utilisation selon l'une quelconque des revendications 6 à 38, dans laquelle R^{b} et R^{c}, ainsi que l'atome auquel ils sont liés, forment un cycle.

40. Utilisation selon l'une quelconque des revendications 6 à 39, dans laquelle R⁷ représente un atome d'hydrogène, un groupe alkyle, aryle ou aryl-(alkylène en C₁ à C₈).

41. Utilisation selon l'une quelconque des revendications 6 à 39, dans laquelle R⁷ représente un atome d'hydrogène, un groupe méthyle ou éthyle, phényle ou benzyle.

42. Utilisation selon l'une quelconque des revendications 6 à 39, dans laquelle R⁷ représente un atome d'hydrogène ou un groupe méthyle.

43. Utilisation selon l'une quelconque des revendications 6 à 42, dans laquelle N(R⁷)₂ représente un groupe amino, éthylamino, diméthylamino, éthylamino, pentylamino, diphényléthylamino, pyridylméthylamino, diéthylamino ou benzylamino.

44. Utilisation selon l'une quelconque des revendications 6 à 42, dans laquelle N(R⁷)₂ représente un groupe amino, éthylamino, diméthylamino, éthylamino, diéthylamino ou benzylamino.

45. Utilisation selon l'une quelconque des revendications 6 à 42, dans laquelle N(R⁷)₂ représente un groupe amino ou méthylamino.

46. Utilisation selon l'une quelconque des revendications 6 à 45, dans laquelle X représente -CH₂OR^{a}, -CO₂R^{a}, -OC(O)R^{a}, -CH₂OC (O) R^{a}, -C (O) NR^{b}R^{c}.

47. Utilisation selon l'une quelconque des revendications 6 à 45, dans laquelle X représente -CH₂OR^{a} ou -C(O)NR^{b}R^{c}.

48. Utilisation selon l'une quelconque des revendications 6 à 45, dans laquelle X représente -CH₂OH ou -C(O)NHCH₂CH₃.

49. Utilisation selon l'une quelconque des revendications 6 à 19, dans laquelle m vaut 0, 1 ou 2.

50. Utilisation selon l'une quelconque des revendications 6 à 49, dans laquelle les cycles comprenant R⁴, R⁵ et l'atome auquel ils sont liés sont choisis dans le groupe constitué par ce qui suit, où q vaut de 0 à 14 :

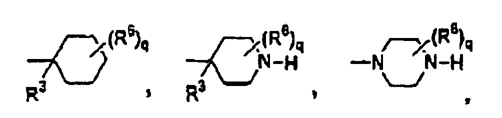

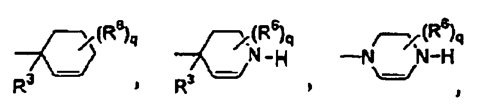

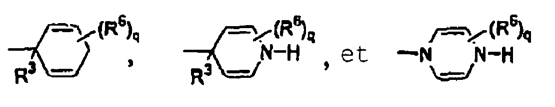

51. Utilisation selon l'une quelconque des revendications 6 à 49, dans laquelle les cycles comprenant R⁴, R⁵ et l'atome auquel ils sont liés sont choisis dans le groupe constitué par :

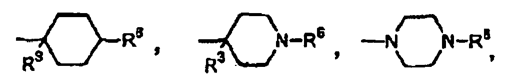

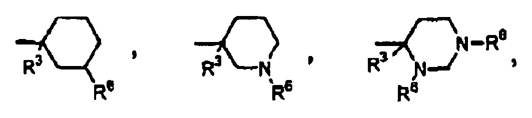

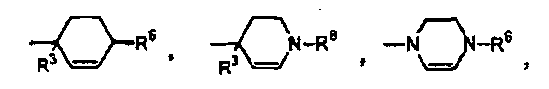

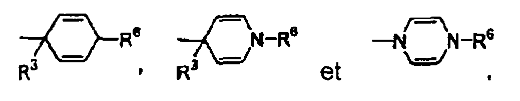

52. Utilisation selon l'une quelconque des revendications 6 à 51, dans laquelle le cycle comprenant R⁴ et R⁵ représente le 2-méthylcyclohexane, le 2,2-diméthylcyclohexane, le 2-phényl-cyclohexane, le 2-éthylcyclohexane, le 2,2-diéthylcyclohexane, le 2-tert-butyl-cyclohexane, le 3-méthylcyclohexane, le 3,3-diméthylcyclohexane, le 4-méthylcyclohexane, le 4-éthylcyclohexane, le 4-phénylcyclohexane, le 4-tert-butylcyclohexane, le 4-carboxyméthylcyclohexane, le 4-carboxyéthylcyclohexane, le 3,3,5,5-tétraméthylcyclohexane, le 2,4-diméthylcyclopentane, l'acide 4-cyclohexanecarboxylique, les esters de l'acide 4-cyclohexanecarboxylique ou le 4-méthyloxyalcanoyl-cyclohexane.

53. Utilisation selon l'une quelconque des revendications 6 à 51, dans laquelle le cycle comprenant R⁴ et R⁵ représente la 4-pipéridine, l'acide 4-pipéridine-1-carboxylique, l'ester méthylique de l'acide 4-pipéridine-1-carboxylique, l'ester éthylique de l'acide 4-pipéridine-1-carboxylique, l'ester propylique de l'acide 4-pipéridine-1-carboxylique, l'ester tert-butylique de l'acide 4-pipéridine-1-carboxylique, la 1-pipéridine, l'ester méthylique de l'acide 1-pipéridine-4-carboxylique, l'ester éthylique de l'acide 1-pipéridine-4-carboxylique, l'ester propylique de l'acide 1-pipéridine-4-carboxylique, l'ester tert-butylique de l'acide 1-pipéridine-4-carboxylique, l'ester méthylique de l'acide 1-pipéridine-4-carboxylique, la 3-pipéridine, l'acide 3-pipéridine-1-carboxylique, l'ester méthylique de l'acide 3-pipéridine-1-carboxylique, l'ester tert-butylique de l'acide 3-pipéridine-1-carboxylique, la 1,4-pipérazine, l'acide 4-pipérazine-1-carboxylique, l'ester méthylique de l'acide 4-pipérazine-1-carboxylique, l'ester éthylique de l'acide 4-pipérazine-1-carboxylique, l'ester propylique de l'acide 4-pipérazine-1-carboxylique, l'ester tert-butylique de l'acide 4-pipérazine-1-carboxylique, la 1,3-pipérazine, l'acide 3-pipérazine-1-carboxylique, l'ester méthylique de l'acide 3-pipérazine-1-carboxylique, l'ester éthylique de l'acide 3-pipérazine-1-carboxylique, l'ester propylique de l'acide 3-pipérazine-1-carboxylique, l'ester tert-butylique de l'acide 3-pipéridine-1-carboxylique, l'ester méthylique de l'acide 1-pipéridine-3-carboxylique, l'ester éthylique de l'acide 1-pipéridine-3-carboxylique, l'ester propylique de l'acide 1-pipéridine-3-carboxylique ou l'ester tert-butylique de l'acide 1-pipéridine-3-carboxylique.

54. Utilisation selon la revendication 6, dans laquelle l'agoniste du récepteur A_{2A} de l'adénosine est un composé ayant une formule choisie parmi ce qui suit : 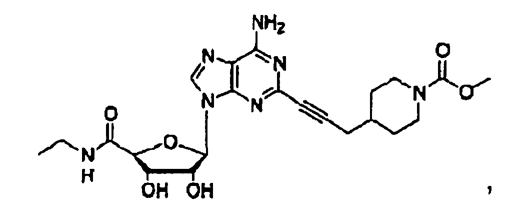 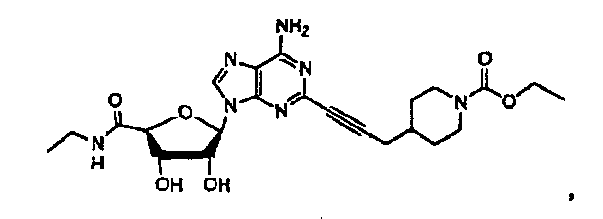 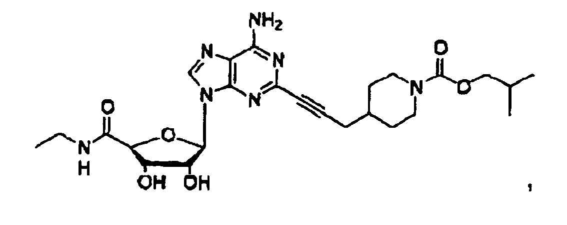 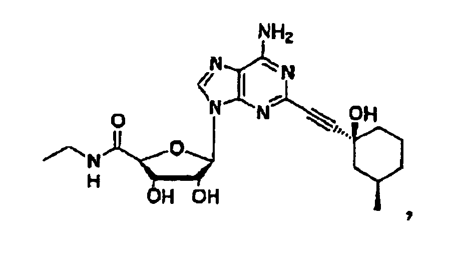 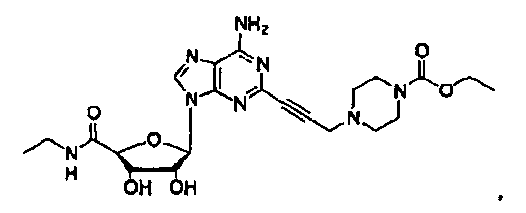 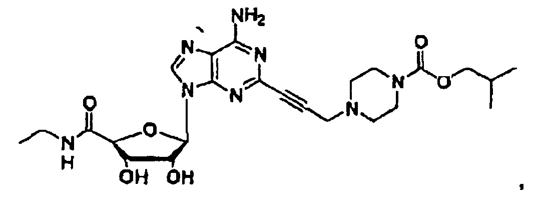 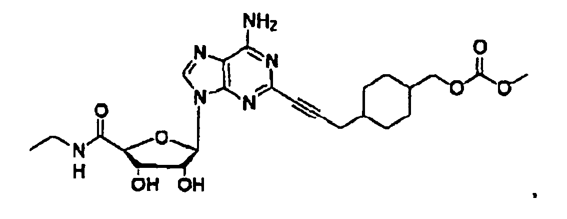 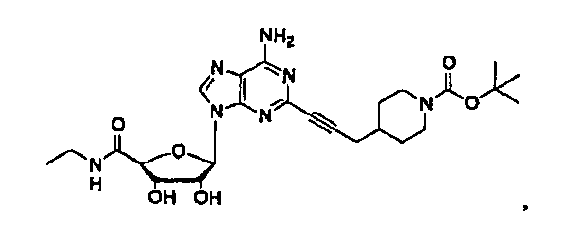 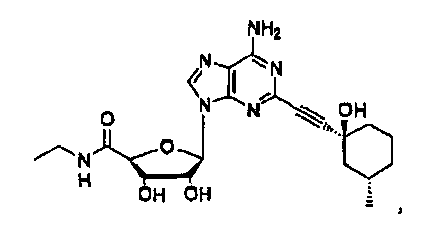 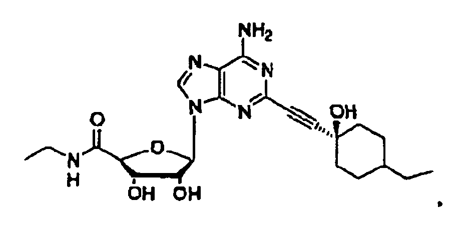 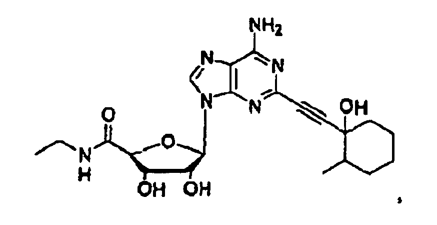 ou

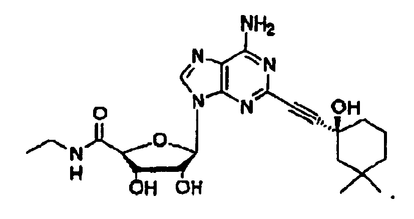

55. Utilisation selon la revendication 6, dans laquelle Z représente CR³R⁴R⁵ ; chaque R¹, R² et R³ représentent, indépendamment l'un de l'autre, un atome d'hydrogène ; R⁴ eL R⁵, conjointement avec l'atome de carbone auquel ils sont liés, forment un groupe cycloalkyle ayant 4, 4, 5, 6, 7, 8, 9 ou 10 atomes cycliques ; et
dans laquelle le cycle comprenant R⁴ et R⁵ est substitué par -(CH₂)₀₋₆-Y; où Y représente -CH₂OR^{a}, -CO₂R^{a}, -OC(O)R^{a}, -CH₂OC(O)R^{a}, -C(O)NR^{b}R^{c}, -CH₂SR^{a}, -C(S)OR^{a}, -OC (S) R^{a}, -CH₂OC(O) R^{a} ou C(S)NR^{b}R^{c} ou -CH₂N(R^{a})(R^{b});
chaque R⁷ représente, indépendamment l'un de l'autre, un atome d'hydrogène, un groupe alkyle en C₁ à C₈, cycloalkyle en C₃ à C₈, aryle ou aryl-(alkylène en C₁ à C₈) ;
X représente -CH₂OR^{a}, -CO₂R^{a}, -OC(O)R^{a}, -CH₂OC(O)R^{a}, -C(O)NR^{b}R^{c}, -CH₂SR^{a}, -C(S)OR^{a}, -OC(S)R^{a}, -CH₂OC(S)R^{a} ou C(S)NR^{b}R^{c} ou -CH₂N(R^{b})(R^{c}) ;
chaque R^{a}, R^{b} et R^{c} représentent, indépendamment l'un de l'autre, un atome d'hydrogène, un groupe alkyle en C₁ à C₈ ou alkyle en C₁ à C₈ substitué par 1 à 3 groupes alcoxy en C₁ à C₈, cycloalkyle en C₃ à C₈, (alkyle en C₁ à C₈)-thio, acide aminé, aryle, aryl-(alkylène en C₁ à C₈), hétéroaryle ou hétéroaryl-(alkylène en C₁ à C₈) ; ou R^{b} et R^{c}, conjointement avec l'atome d'azote auquel ils sont liés, forment un cycle pyrrolidino, pipéridino, morpholino ou thiomorpholino ; et
m vaut de 0 à 6 ;
ou un de leurs sels acceptables sur le plan pharmaceutique.

56. Utilisation selon la revendication 6, dans laquelle l'agoniste du récepteur A_{2A} de l'adénosine est ATL-146e, AB-1, AB-3 ou JR-3213.

57. Utilisation selon la revendication 6, dans laquelle l'agoniste du récepteur A_{2A} de l'adénosine est ATL-146e.

58. Utilisation selon l'une quelconque des revendications 1 à 57 pour la préparation d'un médicament dans lequel un inhibiteur de la phosphodiestérase de type IV est en outre administré au patient.

59. Utilisation selon la revendication 58, dans laquelle l'inhibiteur de la phosphodiestérase de type IV est le rolipram.

60. Utilisation selon l'une quelconque des revendications 1 à 59, en vue de préparer un médicament destiné à traiter l'inhibition d'une inflammation provoquée par des toxines pathogènes.

61. Produit contenant un agent anti-pathogène et un agoniste du récepteur A_{2A} de l'adénosine pour une utilisation sous la forme d'une préparation combinée en vue d'une administration simultanée ou séquentielle à un patient dans le but de traiter une inflammation provoquée par des organismes pathogènes.

62. Produit selon la revendication 61, contenant également un inhibiteur de la phosphodiestérase de type IV.
